(19) Europäisches Patentamt
European Patent Office
Office européen des brevets

(11) EP 3 616 204 B1

(12) EUROPEAN PATENT SPECIFICATION

(45) Date of publication and mention of the grant of the patent:
**17.07.2024 Bulletin 2024/29**

(21) Application number: **19705421.6**

(22) Date of filing: **07.01.2019**

(51) International Patent Classification (IPC):
***G16B 25/20*** *(2019.01)* ***G16B 30/10*** *(2019.01)*

(52) Cooperative Patent Classification (CPC):
**G16B 30/10; G16B 25/20**

(86) International application number:
**PCT/US2019/012511**

(87) International publication number:
**WO 2019/136364 (11.07.2019 Gazette 2019/28)**

# (54) PROCESS FOR ALIGNING TARGETED NUCLEIC ACID SEQUENCING DATA

VERFAHREN ZUR AUSRICHTUNG VON ZIELGERICHTETEN NUKLEINSÄURESEQUENZIERUNGSDATEN

PROCÉDÉ D'ALIGNEMENT DE DONNÉES DE SÉQUENÇAGE CIBLÉ D'ACIDE NUCLÉIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.01.2018 US 201862614088 P**

(43) Date of publication of application:
**04.03.2020 Bulletin 2020/10**

(73) Proprietor: **Illumina, Inc.**
**San Diego, CA 92122 (US)**

(72) Inventors:
- **BEAN, Gordon Jeff**
  **San Diego, California 92122 (US)**
- **BRUAND, Jocelyn**
  **San Diego, California 92122 (US)**
- **KELLEY, Ryan Matthew**
  **San Diego, California 92122 (US)**
- **LEE, Chih**
  **San Diego, California 92122 (US)**
- **EMIG-AGIUS, Dorothea Margherita**
  **San Diego, California 92122 (US)**
- **ALLEN, Eric**
  **San Diego, California 92122 (US)**
- **SUN, Youting**
  **San Diego, California 92122 (US)**

(74) Representative: **Robinson, David Edward Ashdown**
**Marks & Clerk LLP**
**1 New York Street**
**Manchester M1 4HD (GB)**

(56) References cited:
**US-A1- 2016 194 694** **US-A1- 2018 075 186**

- **ANA CONESA ET AL: "A survey of best practices for RNA-seq data analysis", GENOME BIOLOGY, vol. 17, no. 1, 26 January 2016 (2016-01-26), XP055577369, DOI: 10.1186/s13059-016-0881-8**
- **MANUEL GARBER ET AL: "Computational methods for transcriptome annotation and quantification using RNA-seq", NATURE METHODS, vol. 8, no. 6, 27 May 2011 (2011-05-27), New York, pages 469 - 477, XP055577368, ISSN: 1548-7091, DOI: 10.1038/nmeth.1613**
- **ALEXANDER DOBIN ET AL: "STAR: ultrafast universal RNA-seq aligner", BIOINFORMATICS., vol. 29, no. 1, 25 October 2012 (2012-10-25), GB, pages 15 - 21, XP055500895, ISSN: 1367-4803, DOI: 10.1093/bioinformatics/bts635**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

FIELD OF THE INVENTION

The subject matter disclosed herein relates to methods and computer systems for aligning RNA. More particularly, this disclosure relates to aligning reads from expressed RNA to a modified reference genome including transcripts transcribable from a reference genome with primers according to a gene model.

BACKGROUND OF THE INVENTION

RNA alignment includes identifying RNA transcripts present in a test sample, such as RNA produced by a cell or population of cells. Standard whole-genome alignment analyses are not well-adapted for processing amplicon sequencing data because the amplicon data contain unique primer artifacts, are affected by false-positive (off-target) amplifications, and exhibit qualitative differences that violate some assumptions made by standard tools, such as a lack of coverage uniformity, a number of duplicates, etc. Furthermore, conventional RNA alignment methods are highly inefficient computationally, limiting the variety of computer systems that can be used for performing such methods and the utility of such methods and systems. For example, conventional RNA alignment methods require large amounts of RAM, up to 32 gigabytes, rendering many computer systems and sequencing equipment having processors incapable of performing RNA alignment or unsuitable for performing RNA alignment in a necessary time frame.

In addition, conventional RNA alignment methods by which RNA is aligned to a complete reference genome include identifying targets to which reads correspond after they have been aligned. Only thereafter may reads-per-target be quantified. That is, in applications where quantifying the amount of RNA per a given target is present in a test sample, according to conventional methodology first RNA is aligned to a whole reference genome. Because RNA transcripts may include assembled sequences that are not fully contiguous in a genome from which they were transcribed, however, doing so does not directly allow for identification of transcript targets to which reads correspond. Rather, an additional analysis is required to identify transcript targets to which aligned reads correspond, according to conventional RNA alignment methodology. Such a requirement complicates workflows by imposing additional time and attention demands on users of such methods and computer systems implementing them, as well as delays in analysis and imposition of additional demands on computational capacity, hampering output volume.

The present disclosure is directed to overcoming these and other deficiencies in conventional RNA alignment methods and computer systems therefor.

US 2016/0194694 A1 describes multiplex transcriptome analysis. US 2018/075186 A1 describes systems and methods for off-target sequence detection. Conesa A et al, "A survey of best practices for RNA-seq data analysis", Genome Biol 17, 13 (2016), and Garber M et al, "Computational methods for transcriptome annotation and quantification using RNA-seq", Nat Methods, 2011 Jun;8(6):469-77, review computational RNA-seq data analysis. Dobin A et al, "STAR: ultrafast universal RNA-seq aligner", Bioinformatics, 2013 Jan 1;29(1): 15-21, describes an algorithm for aligning RNA-seq data to a reference genome.

SUMMARY OF THE INVENTION

In one aspect, provided is a computer-implemented method of aligning RNA comprising: receiving onto a data storage unit a plurality of primer sequences and a plurality of transcript sequences transcribable from a reference genome; analyzing, using a microprocessor, the plurality of primer sequences to determine a plurality of target sequences amplifiable from the plurality of transcript sequences by the plurality of primer sequences; generating, using a microprocessor, a modified reference genome based on the plurality of target sequences, wherein the modified reference genome comprises the plurality of target sequences and excludes sequences not amplifiable from the plurality of transcript sequences by the plurality of primer sequences; aligning, using a microprocessor, sequence reads generated from a test sample including RNA amplicon molecules to the modified reference genome, wherein the RNA amplicon molecules are generated by amplifying RNA sequences in the test sample using primers having sequences of the plurality of primer sequences; and generating an alignment profile for the test sample based on the aligning.

In an embodiment, the method may also include assigning primer sequences individual loci corresponding to loci of respective transcript sequences. For example, the method may include removing one or more of the generated target sequences based on the one or more of the generated target sequences spanning more than one on-target sequence. In another example, the plurality of primer sequences may include a plurality of primer pairs, and a first primer pair may include a first primer and a second primer for a first locus, and a second primer pair may include the first primer and a second primer for a second locus.

In another embodiment, the transcript sequences may be transcribable from the reference genome according to a gene model, and the gene model may include identification of splice junctions, fusion junctions, or both, in the

transcript sequences transcribable from the reference genome. For example, the method may further include translating sequence reads aligned to targets derived from splice and fusion junctions.

In yet another embodiment, the plurality of target sequences may include on-target sequences and off-target sequences. For example, the method may include reducing a number of off-target sequences by excluding one or more primer sequence from the plurality of primer sequences.

In still a further embodiment, the method may include computationally comparing gene expression of two or more samples, wherein aligned reads generated from a first sample of RNA are compared to aligned reads generated from a second sample of RNA, wherein the alignment is performed using the plurality of target sequences. In another embodiment, the alignment profile may include at least one of placement, a quality score, and sequence integrity for the sequence reads of the test sample. In yet another embodiment, the method may include translating the sequence reads from the test sample to a whole reference genome using the mapped target sequences and the modified reference genome.

In another embodiment, generating an alignment profile may further include aligning a sequence read comprising an unaligned fusion junction to non-contiguous sequences of the reference genome. In yet another embodiment, the alignment profile may include a fusion junction and the fusion junction was identified in the gene model.

In a still further embodiment, provided is a computer-implemented method of aligning RNA comprising: receiving onto a data storage unit a plurality of primer sequences and a plurality of transcript sequences from a reference genome, the transcript sequences being transcribable from the reference genome according to a gene model comprising identification of splice junctions, fusion junctions, or both, in the reference genome; assigning primer sequences individual loci corresponding to loci of respective transcript sequences; analyzing, using a microprocessor, the plurality of primer sequences to determine a plurality of target sequences amplifiable from the plurality of transcript sequences by the plurality of primer sequences; generating, using a microprocessor, a modified reference genome based on the plurality of target sequences, wherein the modified reference genome comprises the plurality of target sequences and excludes sequences not amplifiable from the plurality of transcript sequences by the plurality of primer sequences; aligning, using a microprocessor, sequence reads generated from a test sample comprising RNA amplicon molecules to the modified reference genome, wherein the RNA amplicon molecules are generated by amplifying RNA sequences in the test sample using primers having sequences of the plurality of primer sequences; generating an alignment profile wherein the alignment profile includes at least one of placement, a quality score, and sequence integrity for the sequence reads of the test sample; and translating the sequence reads from the test sample to a whole reference genome using the mapped target sequences and the modified reference genome.

In another aspect, provided is a computer system for aligning RNA comprising: one or more microprocessors; and one or more memories storing a plurality of primer sequences and a plurality of transcript sequences transcribable from a reference genome, the one or more memories storing instructions that, when executed by the one or more microprocessors, cause the computer system to: analyze the plurality of primer sequences to determine a plurality of target sequences amplifiable from the plurality of transcript sequences by the plurality of primer sequences; generate a modified reference genome based on the plurality of target sequences, wherein the modified reference genome comprises the plurality of target sequences and excludes sequences not amplifiable from the plurality of transcript sequences by the plurality of primer sequences; align sequence reads generated from a test sample comprising RNA amplicon molecules to the modified reference genome, wherein the RNA amplicon molecules are generated by amplifying RNA sequences in the test sample using primers having sequences of the plurality of primer sequences; and generate an alignment profile for the test sample based on the aligning.

In an embodiment, the instructions may cause the computer system to assign primer sequences individual loci corresponding to loci of respective transcript sequences. In an example, the instructions may cause the computer system to remove one or more of the generated target sequences based on the one or more of the generated target sequences spanning more than one on-target sequence. In another example, the plurality of primer sequences may include a plurality of primer pairs, and a first primer pair may include a first primer and a second primer for a first locus, and a second primer pair may include the first primer and a second primer for a second locus

In another embodiment, the transcript sequences are transcribable from the reference genome according to a gene model, and the gene model may include identification of splice junctions, fusion junctions, or both, in the transcript sequences transcribable from the reference genome. In yet another embodiment, the plurality of target sequences may include on-target sequences and off-target sequences. In an example, the instructions may cause the computer system to reduce a number of off-target sequences by excluding one or more primer sequence from the plurality of primer sequences.

In still another embodiment, the instructions may cause the computer system to compare gene expression of two or more samples, whereby aligned reads generated from a first sample of RNA are compared to aligned reads generated from a second sample of RNA.

In another embodiment, generating an alignment profile may further include aligning a sequence read comprising an unaligned fusion junction to non-contiguous sequences of the reference genome. In yet another embodiment, the

alignment profile may include a fusion junction and the fusion junction was identified in the gene model.

## BRIEF DESCRIPTION OF THE DRAWINGS

These and other features, aspects, and advantages of the present disclosure will become better understood when the following detailed description is read with reference to the accompanying drawings, wherein:

FIG. 1 is a block diagram of an example system implementing off-target matching detection for a reference sequence.
FIG. 2 is a flowchart of an example method of off-target matching detection.
FIG. 3 is a block diagram of an example system verifying candidate matches.
FIG. 4 is a flowchart of an example method of verifying candidate matches.
FIG. 5 is a block diagram of an example system having a cache for common regions within candidate strings.
FIG. 6 is a flowchart of an example method of identifying matches for a candidate string via a cache.
FIG. 7 is a flowchart of an example method of building a cache for candidate primer sequences.
FIG. 8 is a block diagram of an example system implementing a multi-level cache.
FIG. 9 is a block diagram of an example system using a k-mer index.
FIG. 10 is a block diagram of an example system implementing an off-target predictor.
FIG. 11 is a flowchart of an example method of generating an off-target prediction for a candidate primer sequence.
FIG. 12 is a block diagram of an example system implementing sequence proximity groupings.
FIG. 13 is a flowchart of an example method of identifying off-target matches via sequence proximity groupings.
FIG. 14 is a block diagram of example off-target match conditions.
FIG. 15 is a block diagram of an example system employing sequence proximity groupings for off-target determination.
FIG. 16 is a block diagram showing a multi-level cache for common regions.
FIG. 17 is a block diagram showing skipped candidates via a cache.
FIG. 18 is a block diagram showing extending a common region.
FIG. 19 is a block diagram showing results with a rule satisfaction cache.
FIG. 20 is a block diagram showing correlation between hits on positive and negative strands of a reference genome.
FIG. 21 is a block diagram showing correlation between number of candidates and number of hits for different sequence lengths.
FIG. 22 is a block diagram showing historical data of number of hits versus a prediction using Calculation A.
FIGS. 23 and 24 show results for applying match prediction before searching for matches.
FIG. 25 is a diagram of an example computing system in which described embodiments can be implemented.
FIG. 26 shows a flowchart for RNA alignment in accordance with the present disclosure.
FIG. 27 shows an example of a process for determining matches of primers from a primer set to transcript sequences for the generation of targets in creating a modified reference genome.
FIG. 28 shows an example of how a locus or loci are assigned to a primer or primers.
FIG. 29 shows a schematic of an example for filtering out expected cross-loci targets.
FIG. 30 is a schematic of different amplifiable targets that could be generated from different RNA transcripts that share some sequences but not others.
FIG. 31 is a schematic of an example of translating sequence reads aligned to targets derived from splice junctions.
FIG. 32 is a plot of fusion junction false positives relative to exclusion criteria.

## DETAILED DESCRIPTION OF THE INVENTION

Some embodiments of subject matter disclosed herein are discussed in detail below. In describing embodiments, specific terminology is employed for the sake of clarity. However, the disclosed methods and computer systems are not intended to be limited to the specific terminology so selected. A person skilled in the relevant art will recognize that other equivalent components can be employed, and other methods developed.

Identifying RNA transcripts present in a test sample (whether a human sample or sample from another organism), such as from a cell or population of cells, may include amplifying copies of the RNA, sequencing the amplified copies, and aligning the sequenced copies, or reads, to a reference genome, such as a reference genome of the cell type from which the RNA was samples. For example, the entirety of RNA molecules produced by a cell or population of cells, which may be referred to as the cell's or cells' "transcriptome" for such test sample, may be amplified, sequenced, and aligned, to identify all of the genomic sequences that are transcribed in a given cell, such as cells of a given tissue type, or potentially diseased tissue such as a tumor, or for comparison of different individuals' transcriptomes, or for comparing effects different environmental factors or treatments may have on transcription in given cells. Such methods involve reverse transcribing a cell or cell populations' RNA to DNA, then amplifying the reverse-transcribed DNA to permit

sequencing and aligning for determination of the transcriptome.

DNA amplification is a technique that increases the number of copies of a target nucleic acid molecule (such as RNA or DNA, including DNA that was reverse-transcribed from a cell's RNA, including all or substantially all of the cell's RNA). An example of DNA amplification is multiplex polymerase chain reaction (multiplex PCR). Multiplex PCR assays involve amplification of multiple target nucleic acid molecules in a single reaction. Typically, a pair of oligonucleotide primers is selected for amplification of each target nucleic acid molecule. For aligning RNA, amplification involves reverse-transcribing RNA to DNA, pairs of nucleotides are used to create and amplify DNA sequences corresponding to the RNA sequences present, a process referred to as reverse transcription PCR. The term PCR as used herein includes reverse transcription PCR. A sample containing template nucleic acid comprising the target nucleic acid molecules is contacted with the selected pairs of oligonucleotide primers under conditions that allow for the hybridization of the pairs of primers to the targets on the template in the sample. The primers are extended under suitable conditions, dissociated from the template, re-annealed, extended, and dissociated to amplify the number of copies of the target nucleic acid molecules. The product of amplification can be characterized as needed, for example by nucleic acid sequencing.

The target nucleic acid molecules can be any nucleic acid molecule contained within the template nucleic acid in the sample, including DNA reverse transcribed from a cells RNA. Target nucleic acid molecules for multiplex PCR assays can be 70-1000 base pairs in length, such as 100-150, 200-300, 400-500, and even 70-120 base pairs in length. The members of the primer pairs selected for the multiplex PCR assay hybridize to the up- and down-stream ends of the target nucleic acid molecule to initiate amplification.

Primers are nucleic acid molecules, usually DNA oligonucleotides of about 10-50 or 20-25 nucleotides in length (longer lengths are also possible). Primers can also be of a maximum length, for example no more than 25, 40, 50, 75 or 100 nucleotides in length. Hybridization specificity of a particular primer typically increases with its length. Thus, for example, a primer including 20 consecutive nucleotides typically will anneal to a target with a higher specificity than a corresponding primer of only 10 nucleotides. The 5' end of oligonucleotide primers for multiplex PCR assays can be linked to additional moieties (including additional oligonucleotides) for use in analysis of amplified target. For example, the 5' end of the primers in the primer pairs can be linked to additional oligonucleotide sequences that facilitate sequencing of the amplified target and analysis of resulting sequence reads (for example, adapter sequences, bar code sequences, and the like).

As discussed herein, design and selection of primers for multiplex PCR assays can include screening of a candidate primer having a candidate sequence to determine if there is a likelihood of an off-target hybridization event (off-target match) of the candidate primer to a template nucleic acid molecule having a reference sequence (reference string) that would interfere with the multiplex PCR assay. This involves identifying candidate hybridization locations (candidate matching locations) on the template nucleic acid molecule where the primer may hybridize, and determining if the candidate hybridization locations are verified hybridization locations (verified matching locations) based on a comparison of the candidate primer sequence with the sequence of the candidate matching locations according to one or more verification criteria (matching verification rules). In terms of the technologies described herein, candidate sequences can take the form of primer sequences, which are represented as paired primers (e.g., strings). For purposes of convenience, such internal representations are sometimes simply called a "sequence." An actual physical sequence is represented internally by a string of characters. The reference genome sequence can take the form of a representation of the reference genome or partial reference genome that is targeted by the primers. Thus, a reference genome sequence can represent a sequence of nucleotides and can indicate a designated 3' end and 5' end. Both positive and negative strands can be represented by a single reference genome sequence in a technique that generates reverse complements of the primers and includes them as candidate strings. A primer reverse complement that matches to the reference genome sequence indicates a match on the negative strand of the reference genome at the location indicated by the match. Such matches of primer reverse complements are of interest because if they are within a threshold distance (e.g., off-target condition window length), they can interfere with proper PCR reaction and result in an off-target condition.

If a candidate hybridization location is identified as a verified hybridization location because the verification criteria are satisfied, then additional analysis can be performed to determine if hybridization of the candidate primer to the verified hybridization location, in combination with the hybridization of additional candidate primers for the multiplex PCR assay to corresponding verified hybridization locations on the template nucleic acid molecule, could interfere with the amplification of a target nucleic acid molecule and/or amplify of a non-target nucleic acid molecule (form an off-target condition). If the verification criteria for a first candidate primer would also apply to a second candidate primer (for example, because of similarity of the sequences of the two candidate primers), then for efficiency the analysis to determine if the verification criteria are satisfied for the first candidate primer can be reused for the second candidate primer.

Matching at the character level between a candidate primer sequence and a reference genome sequence can be calculated based on whether the two characters are complementary nucleotides (e.g., they would bind). Thus 'A' is considered complementary to 'T' and 'C' is considered complementary to 'G.' As would be understood, whereas DNA sequences include 'T' nucleotides, RNA instead includes 'U' nucleotides in place of 'T,' with 'A' nucleotides being complementary the 'U' nucleotides. Upon reverse transcription of RNA into DNA and multiplex amplification of the reverse transcribed DNA, DNA sequences corresponding to RNA sequences of a test sample would have 'T' nucleotides instead of 'U" nucleotides, such than presence of a 'T' nucleotide in a sequence reverse transcribed and amplified from test sample RNA would indicate the presence of a 'U' nucleotide in the RNA sequence from which it was reverse transcribed and amplified.

For aligning a test sample's transcriptome by amplifying sequences corresponding to RNA of the test sample--that is, reverse transcribing DNA from a test sample's transcribed RNA and amplifying said reverse transcripts--alignment to a reference genome is computationally demanding. Nucleotide sequences of messenger RNA (mRNA) may lack exons, meaning they are composed of sections of sequences that are not directly contiguous in the genome but are conjoined by splicing mechanisms after genomic DNA has been transcribed. Moreover, different cell types or cells of different organs or tissues may splice a given transcript differently from how such transcripts are spliced in other cell types, organs, or tissues, and a given cell type or tissue or organ may produce differently spliced transcripts under different conditions or at different times, yielding the existence of splice variants in such different cell types or organs or tissues. So too may the transcriptomes of different individuals' cells or tissues differ or of diseased tissues differ by exhibiting splice variants that differ from RNA transcripts in cells, organs, or tissues from other individuals or non-diseased tissue. In addition, RNA fusion, where RNA transcribed from different regions of genomic DNA, not initially as portions of a primary RNA transcript, become attached to one another to form a continuous RNA transcript, adds an additional scale of variability and complexity in aligning RNA. In other instances, translocation of genomic DNA from one locus to another may result in production of an RNA transcript which appears as a fusion, with one portion of the transcript with a sequence corresponding to a locus to which genomic DNA was translocated contiguous with another portion of the transcript with a sequence corresponding to a locus from which genomic DNA was translocated.

The existence of, for example, splice variants and RNA fusion within a transcriptome adds a layer of computational complexity on top of the complexity of conventional nucleotide alignment methods. Conventional RNA alignment methods are highly taxing of computational power, traditionally requiring up to 32 gigabytes of RAM in order for alignment processing to be performed. In many cases such computational demands render it impossible to perform RNA alignment with an available computer system, or requires use of an unavailable or otherwise unnecessarily powerful or costly computer system or one which cannot easily be provided as a component of other hardware used for sequencing. As disclosed herein, by combining primer design with generation of a modified reference genome representing sequences that could be amplified from a test sample, computational demands on RNA alignment may be substantially reduced, such that alignment of RNA such as a test sample's transcriptome may be performed using only 16 gigabytes of RAM or less. The streamlined method disclosed herein, and computer systems for the performance thereof, improve computer functionality by reducing demands of processing power and further improve workflows by eliminating steps rendered unnecessary thereby.

For aligning RNA, as explained more fully below, identifying an entire set of RNA that may be transcribable from a given genome, and using such set of identified transcribable sequences, simplifies RNA alignment as disclosed herein. A complete reference genome includes significant portions of DNA that are not transcribed, and other portions that are transcribed but intronic and therefore removed from RNA. A reference genome also may not directly identify splice variants or fusion RNA transcripts, although it contains sequences that determine where transcribed RNA sequences may be spliced or fused together. A set of all RNA theoretically transcribable from a reference genome therefore occupies far less memory storage in a computer system than the reference genome, reducing memory demands required for accessing its sequence information, excluding as it does non-transcribable DNA, and also includes splice variants and fusion RNA not directly present in a reference genome. Because RNA present in a test sample is more likely to resemble portions of hypothetically transcribable sequences from a reference genome that a reference genome itself, as disclosed herein, transcripts of sequences transcribable from a reference genome may be used as source of reference sequences in aligning a test sample's RNA.

A reference genome's transcript sequences may be constructed by a computer with reference to a reference genome and a gene model. A gene model may include a set of instructions executable by a computer processor identifying rules specifying sequences that may be transcribable from a reference genome, on the basis of identifying regions of the reference genome that direct transcription of particular sequences, transcription stop points, exon-intron boundaries within transcribed sequences, variable splicing permutations, RNA fusion products that may be produced, and other factors that determine what sequences of the reference genome would be included and excluded when all possible transcription events occur and what variations of transcription products are possible. A gene model may include instructions to include transcribable sequences in transcript sequences on the basis of sequences of a reference genome known to indicate occurrence and sequence of transcribed sequences, and their potential different sequence arrangements on the basis of splicing, RNA fusion or both. A gene model may also include instructions to include transcribable sequences in a modified transcript sequences on the basis of transcripts known to be produced by cells having a given reference genome.

As described above, aligning RNA of a test sample may involve amplification of the test sample's RNA, through

the use of primers. Selection of primers for multiplex synthesis and amplifying of DNA corresponding to a test sample's RNA determines on-target and off-target sequences that may be amplified from the sample for creating reads for alignment. Systems and methods for identifying on-target and off-target sequences that may be amplified from nucleotide sequences in a test sample are described in U.S. Patent Application Serial No. 15/705,079. For a given set of primer sequences, sequences that would be amplified from a reference genome, or from transcript sequences transcribable from a reference genome according to a gene model, may be determined. Of those, sequences that represent on-target sequences, resulting from amplification of sequences corresponding to target sequences in the reference genome's target sequences, and sequences that represent off-target sequences, resulting from hybridization of probes other than at target sequences and subsequent amplification other than of target sequences, may be identified. Identification of on-target sequences and off-target sequences amplifiable from a given set of reference transcripts of a reference genome by a given set of primers is modifiable based on rules defining whether an amplified target satisfy on-target or off-target definitions. For example, some allowable of upper limit of a number of mismatches between a primer's sequence and a region of a reference genome's transcript sequences to which the primer may align and promote amplification during multiplex amplification may be set. Or a maximum allowable number of mismatches between nucleotides at an end of a primer, such as its 3' end, and a region of a reference genome's transcript sequences to which the primer may align and promote amplification during multiplex amplification may be set.

Primers that result from hybridization of such primers to such regions may be deemed to lead to generation of off-target sequences during multiplex amplification. Increasing or decreasing the maximum number of mismatches or primer-end mismatches may decrease or increase, respectively, the number of targets classified as off-target when the given primer is used in multiplex amplification. Where there is a preference for fewer or no off-target sequences, more stringent parameters for identifying off-target sequences may be used and primers resulting in amplification of off-target sequences may be excluded from use in amplification.

For identification and alignment of reads generated from a test sample's RNA, a modified reference genome can be generated from the transcript sequences produced from a reference genome according to a gene model. By predetermining the amplification products likely to be produced from a test sample's RNA, aligning the test sample's RNA is rendered far more computationally efficient, as opposed to aligning reads from test sample RNA to the reference genome, as explained above, as well as in comparison to aligning to a hypothetical transcriptome comprised of all possible transcribable sequences from a reference genome. Only sequences whose amplification would be stimulated by use of suitable primers in a multiplex amplification process would be expected to correspond to reads in an RNA alignment method. As disclosed herein, sets of primers may be analyzed to determine the amplification products and therefore reads they would result in the development of in an RNA alignment method.

Transcript sequences, transcribable from a reference genome pursuant to a gene model, and primer sequences, may be received by a data storage unit. One or more microprocessors may then identify transcripts that such primers, in a multiplex amplification process, would lead to the generation of. The targets thereby identified would serve as a modified reference genome against which reads corresponding to a test sample's RNA would be aligned. The size of a reference modified genome would depend on the numbers of primers used to generate it, and could also depend on stringency of parameters for definition of off-target sequences and rules for inclusion or exclusion of off-target sequences in the modified reference genome. It is not necessary that primers be selected for amplification of sequences corresponding to all RNA transcripts present in a test sample, although amplification of all RNA sequences in a test sample is also included in the methods and systems discloses herein. In either case, primers or proposed candidate primers intended for use in a multiplex amplification process for RNA alignment may first be analyzed to determine sequences they would be predicted to amplify by reference to transcript sequences of the reference genome according to the gene model.

In any of the examples herein, candidate primer sequences can be decomposed into substrings or subsequences of length k (the k mers) to facilitate finding a match. The k mers can be generated for a candidate primer sequence. In practice, all such substrings or subsequences are generated, but other arrangements are possible.

In any of the examples herein, identifying matching locations on a reference genome sequence for a candidate primer sequence can comprise decomposing the candidate primer sequence into k mers and searching a k mer index with the k mers.

Primer sequences, or k-mers, may be matched against transcript sequences from the reference genome to determine whether the primer would give rise to an amplification target. Parameters may be set for whether a k-mer does so, including a minimum number of consecutive base pairs matching between a primer and a transcript sequence, a maximum number of mismatches across a primer permitted, and a maximum number of mismatches between the primer's 5' end and the transcript sequence allowed. Also included in rules for generating a modified reference genome from a reference genome's transcript sequences for a given et of primers may be a maximum and minimum length or predicted targets included in the modified reference genome. Primers that do not meet parameters set for defining primers that generate targets, and targets that do not meet the definitions set for targets to be included in the reference genome, may be excluded.

In an example of identifying targets to include in a modified referenced genome, primers are matched to the reference genome's transcript sequences, beginning at the 5' end and continuing to the 3' end. The transcript sequences include sequence information from the plus strand and the complementary minus strand, and primers may be analyzed for whether they match to each strand, according to the parameters established for classifying a primer as a match as described above. If the primer matches to a sequence on the plus strand, it and its match location may be stored in a memory cache. If the primer matches to the minus strand, it may be stored to the memory cache. Pairs of primers, a forward and reverse primer, one matching to each of the pair of complementary strands of a reference genome transcript sequence, together generate amplified products during multiplex amplification. Thus, when a primer that matches to the negative strand is identified and cached, it may be compared to primers previously cached as matching a sequence of the reference genome's transcript sequences. When cached primers, one forward and reverse, are determined to lead to amplification of a target, the target may be added to the modified reference genome.

As the matching of primers to transcript sequences proceed along the transcript sequences, from 5' to 3', and matches of additional primers are identified for comparison with upstream primer matches for identification of targets amplifiable by the primers, checking whether a new primer match can form an amplifiable target with a prior upstream match may be performed for every prior match. As one proceeds down the template sequences, location of match sequences of a prior match and a new match will be farther apart and such potentially amplifiable target therebetween longer. If an amplifiable target between a new primer match and a prior upstream primer match would be of a length that exceeds parameters for a target to include in the modified reference genome, the upstream target can be disregarded in subsequent evaluations of amplifiable targets.

A pair of primers leads to generation and inclusion of a target in the modified reference genome provided any parameters for primer matching and target size have been satisfied. However a primer may match to more than one sequence in transcript sequences of the reference genome. Unless such duplicates were identified and removed, duplicates of targets in the modified reference genome may result. In an example, for avoidance of such duplications, a single locus is determined for a matching primer. For each primer that is unique to a region in the transcript sequences, the primer may be assigned to the locus. If at least one of the primers of a pair of primers determined to lead to amplification of a template matches to sequences in more than one transcript, it may be assigned the locus of a transcript to which both primers have a matching sequence if such a transcript exists. In the event there are multiple transcripts to which both primers have matching sequences, an arbitrary rule for assigning which of such multiple transcripts as a locus for each or both primers may be used. In an example, the first transcript, alphabetically according to its locus ID, may be assigned to each primer. If there is no single transcript with sequences to which both primers of a pair match when one of the primers of the pair matches to sequences of multiple transcripts, an arbitrary rule for assigning which of such multiple transcripts as the locus of the one of the primers may be used. For example, the first transcript, alphabetically according to its locus ID, having a sequence to which each primer matches may be assigned to each primer, respectively.

When two targets that are in relative proximity to each other, a longer target that includes the two targets within it may also be detected. Such cross-loci do not pose significant problems in alignment, as the proximal targets may be formable from the cross-loci target during amplification but the larger targets cannot be formed from either of the smaller targets, meaning they would be of lower copy number and therefore less represented. Nevertheless, such cross-loci targets may be filtered out from or not added to the modified reference genome by characterizing them as off-target sequences. In order to be filtered out from the modified reference genome, a larger target's upstream target must match to a sequence within one intended target, and its downstream primer must match to a different target, and the larger target must be larger than either of the targets to which its primers match.

Sequences within the modified reference genome may then be mapped back to the reference genome, for inclusion of corresponding genome location information in the modified reference genome. Due to splicing and RNA fusion, contiguous sequences of the modified reference genome require segmentation in order to be mapped back to locations in the reference genome. It is possible that RNA transcripts in a sample that differ from each other may, when amplified by primers in the primer set during multiplex amplification, give rise to amplification products, or amplicons, that are the same as each other. For example, two splice variants of each other may give rise to the same amplicon as each other when a pair of primers leads to amplification of a sequence spanning to adjoining exons contained in each splice variant, notwithstanding differences between other portions of the splice variant. Other primer pairs may give rise, from splice variants, to amplicons that differ from each other. For example, the presence of an exon between primers in one splice variant which exon is absent from the other splice variant would result in different amplicons generated from the splice variants by the pair of primers. RNA templates are considered to be identical to each other if the list of targets that can be amplified therefrom by a primer set used in multiplex amplification corresponds to the same locations in the genome as each other.

Once a modified reference genome has been constructed, primers having sequences of the primers of the primer set used in construction of the modified reference genome can be used for multiplex PCR amplification of RNA sequences in a test sample. Reads may be generated corresponding to the detected amplicons from the test sample then mapped back to the modified reference genome. Mapping involves aligning sequences contiguously, on the basis

of any overlapping ends, and identifying where in the modified reference genome the reads correspond. Generally, sequencing data gathered as part of a sequence analysis is stored in a sequence alignment dataset. Common file types for storing sequence alignment data are the SAM (.sam) and BAM (.bam) file formats. Sequence alignment software ("aligners") outputs a sequence alignment dataset file, e.g. a BAM file, that indicates alignments of read sequence(s) to a reference genome or, in accordance with the present disclosure, a modified genome reference consisting of amplifiable targets from transcript sequences of the reference genome.

An alignment file may include an alignment profile of for a test sample based on the aligning. The alignment profile may contain further information pertaining to the aligned sequence as contained in the alignment file. For example, if, as disclosed in an example herein, the sequence information included in the modified reference genome may contain identification of locations in the reference genome corresponding to sequences in the modified reference genome, then aligning reads from a test sample to a modified reference genome enables mapping the reads to the reference genome as well by reference to the reference genome location information contained in the modified reference genome sequences to which the aligned reads pertain. In some instances, this may include translating sequence reads aligned to targets derived from splice and fusion junctions. For example, a target from a modified reference genome may contain exon-exon boundaries, and a read or sequence within a read may align across such a boundary. Or a target from a modified reference genome may include an RNA fusion, including a junction between sequences of RNA that did not originate from a single transcript but from individual transcript molecules transcribed from independent loci within a reference genome. In another example, a fusion may result from translocation of genomic DNA leading to an RNA transcript with sequence information from two previously non-contiguous loci. If a modified reference genome includes chromosomal locus-identifying information, alignment of a read may include generating a profile of the aligned read including identification of chromosomal loci within the reference genome from which the aligned read or sequence within the aligned read was transcribed. Similarly, aligned reads that do not span exon-exon boundaries or boundaries between fused sequences within RNA fusion sequences may also be translated back to chromosomal loci within the reference genome and such information included in an alignment profile.

In some examples, a sample may contain RNA fusion products that are accounted for in the gene model. In such a case, such a fusion junction may be present in the modified reference genome, because the gene model may have identified it as transcribable from the reference genome. When a sequence read corresponding to such a fusion junction is present, it may be aligned to the modified reference genome and classified as an aligned fusion junction. Such classification may be reflected in an alignment profile.

In other examples, a sample may contain RNA fusion products that are not present in a gene model. In such cases, a corresponding fusion junction may be absent from a modified reference genome. Sequence reads corresponding to the fusion junction-containing transcripts from the sample may thus be unable to be aligned to the modified reference genome, or may align incompletely or poorly. For example, they may align only partially to each of two, non-contiguous or dispersed loci in the modified reference genome, one corresponding to sequence present on the 5' side of the fusion junction and the other corresponding to sequence present on the 3' side of the fusion junction. Alignment of sequence reads corresponding to such fusion junctions may not be alignable to a modified reference genome. In such an example, unsuccessful attempts to align such a sequence read to a modified reference genome may result in its being classified as an unaligned fusion junction.

As disclosed herein, an unaligned fusion junction may still be aligned and its alignment included in a generated alignment profile. An unaligned fusion junction may be aligned to a reference genome, rather than to the modified reference genome which was assembled from a plurality of target sequences transcribable from the reference genome. In such an example, aligning the unaligned fusion junction to the reference genome may result in identification of genomic loci corresponding to each of the sides of the fusion junction, i.e. representing the sequences that were either joined by splicing in creating the fusion junction or joined by translocation of genomic DNA. Aligning sequence reads corresponding to RNA transcripts often involved mapping reads to regions of genomic DNA that may be separated, for example where removal of introns from an RNA transcript and splicing together of the exons on either side of the intron ultimately results in generation of a sequence read with a 5' portion with a sequence corresponding to one portion of genomic DNA in the reference genome and another 3' portion with a sequence corresponding to a different region of genomic DNA in the reference genome. In similar fashion, it is possible to align an unaligned fusion junction to a reference genome and identify loci that are disparate in the reference genome that came together in formation of the transcript the yielded the sequence read.

As discussed above, aligning sequence reads to a reference genome can be a computationally demanding and time consuming computer-implemented method. A reason for such a high computational demand includes the high number of sequence reads that may be generated from a sample and require aligning. A benefit of an example of the method and system disclosed herein may be that the number of reads requiring aligning to a reference genome may be reduced. For example, after aligning sequence reads to a modified reference genome and translating them back to a reference genome, it may be unnecessary to subsequently directly re-align such sequence reads to a reference genome, their corresponding location in the reference genome already having been identified as described. When

unaligned fusion junctions have been classified, they may be aligned directly to a reference genome. However, in such a case, computational and time demands may be substantially reduced compared to demands that would have been required if they were aligned without first having been classified as unaligned fusion junctions (that is, unaligned with reference to a modified reference genome). By aligning sequence reads to a modified reference genome and classifying unaligned fusion junctions, aligning the unaligned fusion junctions to a reference genome may be done without also having to align sequence reads that were aligned to the modified reference genome. By first aligning sequence reads to a modified reference genome, a total number of sequence reads for aligning to a reference genome may be substantially reduced, i.e. to only the unaligned fusion junctions. Such reduction in the number of sequence reads for aligning directly to a reference genome significantly reduces the computational and time demands required for aligning unaligned fusion junctions to the reference genome, which otherwise would have to have been aligned to the reference genome together with the full set of sequence reads from a sample.

In some such examples, a sequence read that was not unaligned to the modified reference genome may be aligned to a reference genome and such aligning may indicate that the sequence read represents a fusion junction but such indication may be incorrect, in that the sequence read does not in fact represent a fusion junction. Such an example may be referred to as a fusion junction false positive. When identifying sequence reads classified as unaligned fusion junctions, in some examples sequence reads not including actual fusion junctions may be included among the unaligned fusion junctions to be aligned to a reference genome and some of them may be aligned to the reference genome and erroneously identified as a fusion junction while others, having bene unaligned to the modified reference genome, may be correctly identified as fusion junctions once aligned to the reference genome. It may be advantageous to differentiate between unaligned fusion junctions that were aligned to the modified reference genome and accurately identified as fusion junctions and fusion junction false positives.

Several examples of screens to differentiate between accurately identified fusion junctions and fusion junction false positives may be used, individually or together, in accordance with the present disclosure. For example, a minimum sequence read alignment length may be established such that a sequence read identified as a fusion junction after being classified as an unaligned fusion junction then aligned to the reference genome is not classified as a false positive unless its alignment length is below such minimum sequence read alignment length. For example, a sequence read may be classified as a fusion junction false positive when its alignment length is not greater than 70. Other minimum alignment lengths may be used instead, such as 50, 60, 80, 90, 100, 150, of 200 nucleotides as a minimum sequence read alignment length.

In another example, a sequence read may need to have had at least a minimum number of copies reflected in the sample of sequence reads in order not to be characterized as a fusion junction false positive. For example, if an unaligned fusion junction is aligned to a reference genome and identified as a fusion junction, a requirement may be applied by which it is characterized as a fusion junction false positive unless it has at least 100 reads. In some examples, the minimum number of reads may be 200, or 300, or 500, or 750, or 1000. Other minimums may also be used.

In still other examples, ratio of an alignment length of a sequence read to a local alignment length may need to exceed a minimum in order for a sequence read not to be classified as a fusion junction false positive. For example, a sequence read may appear to represent a fusion junction, in that one end of the read may align to one portion of a reference genome, and the other end of the sequence read may align to another region of the reference genome that is not contiguous with the first (e.g., on a different chromosome, or disparate from the first on the same chromosome). However, the sequence read may additionally appear alignable, at least partly, with another region of the reference genome, in a contiguous manner (i.e., in a way that does not span non-contiguous regions or signify a fusion junction). This latter alignment, alternative to an alignment signifying a fusion junction, may be referred to as a local alignment. An alignment signifying presence of a fusion junction may have an alignment length, which is the length of its sequence that is aligned to a reference genome (partially to one locus and partially to another locus). The alternative, local alignment, may also have a local alignment length, which is the length of its sequence that is alternatively alignable to a contiguous sequence of reference genome. In order not to be characterized as a fusion junction (i.e., as a qualification for characterization as a false positive), the alignment length of the sequence read aligned as a fusion junction may be required to exceed the alternative, local alignment legth for the seqeunce read. In the event a sequence read may have more than one possible local alignment length, the longest such local alignment length may be selected and used for comparing to the fusion junction alignment length.

In some examples, a sequence read may need to satisfy any one or two or all three of these criteria in order not to be classified as a fusion junction false positive. Upon alignment to a reference genome of fusion junctions that were unaligned to a modified reference genome, and upon confirming that they are not to be classified as fusion junction false positives, the fusion junctions and corresponding locations in the reference genome may be included an alignment profile.

Additional information may also be included in an alignment profile. For example, the profile may include a score indicating whether particular reads have been misaligned, known as a quality score, or sequence read integrity, or other indicia of accuracy or completeness of read, putative presence of insertions or deletions or other mismatches, etc.

Methods disclosed herein may also be used to compare RNA alignments of different test samples to each other, in addition to testing any given sample against a reference genome (through alignment to a modified reference genome as disclosed herein). A modified reference genome may be constructed from a reference genome, then sequence alignments created from RNA contained in different test samples. The different samples may be from different individuals, different tissues from an individual, or diseased tissue such as a tumor cell population and non-diseased tissue. An alignment file may be created for each sample, and each file may also include an alignment profile. Comparisons are then possible between the alignment files of the two or more samples to identify differences in RNA present in each sample type. Differential expression software may be used to compare alignment files of different test samples generated against a common modified reference genome and analyze whether and when apparent differences between alignment files represent actual differences between samples' RNA.

EXAMPLES

The following examples are intended to illustrate particular embodiments of the present disclosure, but are by no means intended to limit the scope thereof.

In any of the examples herein, the technologies can be applied to specificity calculations for primers in a multiplex polymerase chain reaction scenario. Thus, fast specificity checking for multiplex polymerase chain reaction primer design can be accomplished. Multiplex polymerase chain reaction is widely used in diagnostic testing and forensic testing to simultaneously amplify multiple DNA regions of interest (targets). The successful running of a multiplex PCR involves the design of a suitable set of primer pairs. Each pair of primers comprises a forward primer and a reverse primer extracted from the upper and lower regions of the targets. Ideally, each designed pair should only amplify the intended targets, but not any unintended targets (off targets). The process of checking potential off-targets is called specificity checking, which is a key step in primer design.

Primer sequences can be grouped into clusters based on the target region of the reference genome sequence. For example, if a primer generation tool is used to generate primer candidates for multiple target regions in a multiplex PCR scenario, the primers can be stored as associated based on the target region (e.g., primers for different target regions are stored in different clusters). Common region determination can be performed as described herein based on such clusters.

Thus, the candidate primer sequences herein can be known to match a target, and it can be desirable that there be few or no off-target matches for such candidate primers. Candidate primer sequence pairs can be associated with known locations on the reference genome to represent their target and allow confirmation of an off-target condition. Matches at the target are considered to be on-target.

The task of specificity checking is nontrivial because there are several factors considered when deciding whether a DNA or RNA region could be amplified by a primer: notably, the overall similarity of the target and the stability of the 3' end. Typical existing approaches only report results with hundreds of primers at most. The techniques described herein can easily scale to hundreds of thousands of primers. Thus, the techniques can dramatically reduce the runtime of specificity checking by adopting rule calculation caching, off target prediction, and sequence proximity groupings.

Off-target detection can be implemented for a plurality of candidate primer sequences as described herein. Caching can re-use rule satisfaction calculations for candidate primer sequences sharing a common region. Match prediction can be used to filter candidates, and sequence proximity groupings can be used to facilitate identifying off-target match conditions. Other features relating to common region extension can be employed to achieve the technologies as described herein.

Benefits of the technologies include more scalability, especially for large numbers of candidate primer sequences targeting multiple regions on a large reference genome sequence.

Off-target detection can be useful in specificity calculations as described herein.

Therefore, overall performance of off-target detection can be enhanced as described herein.

Example 1 - Example System Implementing Off-Target Matching Detection

FIG. 1 is a block diagram of an example system 100 implementing off-target matching detection for generating a modified reference genome sequence from a transcript sequence 180. In any of the examples herein, a string can take the form of a sequence of characters representing a string of values. Although called a "string" herein, internal representation can take the form of a string, array, or other data structure. Characters can take the form of characters or codes representing such characters.

In the example, a plurality of candidate primer sequences 110 are received as input by the off-target detection tool 150. As described herein, such candidate primer sequences 110 can take the form of primer pairs targeting a particular location on a transcript sequence 180 representing positive and negative strands of transcript sequences transcribable from a reference genome as described herein. Therefore, the candidate primer sequences 110 are aimed

at targets on the transcript sequence 180. In some cases, off-target matches may also occur, whether in conjunction with a primer in the same pair or another pair (e.g., an inter-locus off-target match). In a multiplex scenario, the candidate primer sequences 110 can be targeted to multiple locations of the transcript sequence 180, resulting in higher computational complexity when finding off-target matches. This higher computational complexity results in expending more resources and processing for a greater amount of time.

The off-target detection tool generates acceptable sequences 160 (e.g., input candidate primer sequences (e.g., pairs of primers) that are considered acceptable in light of detected off-target matches).

Internally, the off-target detection tool 150 can apply a plurality of rules 120 when determining whether a primer sequence matches a location of the transcript sequence 180. The tool 150 can also make use of a k-mer index 170 of the transcript sequence 180 to assist in matching determination. In practice, a match may initially be considered a candidate match and then verified to be a verified match.

A rule satisfaction calculation cache 125 can be used to alleviate the computational complexity associated with multiplex scenarios. As described herein, the cache 125 can leverage common regions in clusters of candidate primer sequences 110.

The off-target correlator 127 can accept verified matches and determine whether such verified matches result in an off-target match condition. As described herein, sequence proximity groupings can be applied to reduce computations involved in identifying an off-target match condition.

The off-target detection tool 150 can also accept settings as input that configure operation, such as parameters for the rules 120, or the like.

In any of the examples herein, although some of the subsystems are shown in a single box, in practice, they can be implemented as computing systems having more than one device. Boundaries between the components can be varied. For example, although the off-target detection tool 150 is shown as a single entity, it can be implemented by a plurality of devices across a plurality of locations. The rules 120 can be shared among multiple tools 150, and so forth.

In practice, the systems shown herein, such as system 100, can vary in complexity, with additional or less functionality, more or less complex components, and the like. For example, additional indexes, tables, and the like can be implemented as part of the system 100. Additional components can be included to implement security, redundancy, load balancing, auditing, and the like.

In practice, a large number of candidate primer sequences 110 and a large reference genome sequence 180 can be checked for off-target matches in a multiplex scenario.

The described computing systems can be networked via wired or wireless network connections. Alternatively, systems can be connected through an intranet connection (e.g., in a corporate environment, government environment, educational environment, research environment, or the like).

The system 100 and any of the other systems described herein can be implemented in conjunction with any of the hardware components described herein, such as the computing systems described below (e.g., processing units, memory, and the like). In any of the examples herein, the inputs, outputs, caches, indexes, strings, rules, and the like can be stored in one or more computer-readable storage media or computer-readable storage devices. The technologies described herein can be generic to the specifics of operating systems or hardware and can be applied in any variety of environments to take advantage of the described features.

Example 2 - Example Method of Off-target Matching Detection

FIG. 2 is a flowchart of an example method 200 of implementing off-target matching detection and can be implemented, for example, in a system such as that shown in FIG. 1. A plurality of candidate primer sequences targeting multiple targets on a transcript sequence can be supported.

In practice, actions can be taken before the method begins, such as generating the candidate primer sequence pairs using a primer generation tool or the like.

At 220, a candidate primer sequence is received. The candidate primer sequence can take any of the forms described herein.

At 230, for a candidate primer sequence, matches on a transcript sequence are identified. Match determination can involve applying a plurality of rules as described herein. For example, a plurality of candidate matching conditions can be identified on the transcript sequence (e.g., via a matching rule as described herein). Out of the candidate matching locations, verified matching locations on the transcript sequence can be identified. Such verification can comprise determining which of the candidate locations on the transcript sequence satisfy matching rules as described herein.

Identifying candidate matching locations or verifying matching locations can comprise reusing a rule satisfaction calculation already calculated for another candidate primer sequence sharing a common region with the candidate primer sequence as described herein.

At 240, it is determined whether the verified matching locations form an off-target match condition on the transcript sequence. As described herein, a match can be considered in conjunction with matches for another candidate

primer sequence (e.g., on another, opposite direction transcript sequence represented as described herein) to find a pair of candidate primer sequences that result in an off-target match.

Based on whether the verified matching locations form an off-target match condition, it is determined whether the candidate primer sequence is acceptable. For example, a threshold number of off-target matches can be applied, or no off-target matches may be allowed. Candidate primer sequence pairs, or their associated candidate targets, are included in the acceptable primer sequences if they meet the off-target threshold. More off-target matches result in lower specificity, making the candidate primer sequence less desirable.

As described herein, the method 200 can be performed for a plurality of candidate primer sequences (e.g., it is repeated for other candidate primer sequences). In practice, parallel and/or concurrent computation scenarios can be applied.

The method 200 and any of the other methods described herein can be performed by computer-executable instructions (e.g., causing a computing system to perform the method) stored in one or more computer-readable media (e.g., storage or other tangible media) or stored in one or more computer-readable storage devices. Such methods can be performed in software, firmware, hardware, or combinations thereof. Such methods can be performed at least in part by a computing system (e.g., one or more computing devices).

In any of the technologies described herein, the illustrated actions can be described from alternative perspectives while still implementing the technologies. For example, at 220, the method describes receiving a candidate primer sequence. However, such an act can also be described as "sending the candidate primer sequence" for a different perspective.

Example 3 - Example Off-target Matching Detection

In any of the examples herein, an off-target match can take the form of a pair of candidate primer sequences (e.g., whether from an original pair or two different pairs) that match at proximate locations as described herein. In practice, the proximate locations can be on two different (e.g., one original and one reversed and complementary to the original) transcript sequences as described herein; computations can be accomplished with a single transcript sequence by taking a reverse complement of a candidate primer sequence and including it in the candidate primer sequences. As described herein, detecting such an off-target match can be used to determine whether a candidate primer sequence is acceptable or not. A candidate primer sequence that exceeds an off-target match condition threshold (and its pair) can be considered unacceptable.

Example 4 - Example *k*-mers

In any of the examples herein, candidate primer sequences can be decomposed into substrings or subsequences of length *k* (the *k*-mers) to facilitate finding a match. The *k*-mers can be generated for a candidate primer sequence. In practice, all such substrings or subsequences are generated, but other arrangements are possible.

In any of the examples herein, identifying matching locations on a transcript sequence for a candidate primer sequence can include decomposing the candidate primer sequence into *k*-mers and searching a *k*-mer index with the *k*-mers.

Example 5 - Example Matching

In any of the examples herein, a sequence is considered to match a transcript sequence at a particular location when rules are satisfied. Example matching rules can comprise the following:

Rule 1. There are at least *k* consecutive matching characters (e.g., matches at the character level).
Rule 2. There are not more than e * *l* character mismatches in total, where *l* is the length of the candidate primer sequence, and *e* is a parameter (e.g., a percentage, fraction, or the like).
Rule 3. There are not more than *m* character mismatches on an end of the candidate primer sequence.

Matching and mismatching characters can be determined based on complementary matches between characters as described herein. During match processing, a match can be treated as a candidate match until the three rules are verified as satisfied, at which point the match can become a verified match.

In any of the examples herein, the three matching rules above can be incorporated for determining matches. One or more rules can be designated as initial rules, while one or more others are designated as matching verification rules. For example, Rule #1 regarding consecutive matches can be designated as an initial rule, and candidate matches satisfying the initial rule can be verified via the other rules. Other arrangements for rules can be implemented.

In any of the examples herein, a match can take the form of the location on the transcript sequence where the

match occurs (e.g., an integer indicating *i* characters from the beginning of the transcript sequence, a pointer to the location, or the like). The match can also take the form of an indication of the candidate primer sequence involved (and an identifier of a pair or an identifier of another candidate primer sequence in the pair). In scenarios with multiple transcript sequences or representations thereof, the match can also indicate on which transcript sequence the match occurs.

Verified matches can take the form of a match and also include an indication that the match has been verified. Verification can be implied (e.g., because the match appears in a list of verified matches).

Example 6 - Example Candidate Match Verification

In any of the examples herein, identifying matches on a transcript sequence can take the form of verifying candidate matches. FIG. 3 is a block diagram of an example system 300 verifying candidate matches of candidate primer sequences 310 and can be used in any of the examples herein. By separating calculations for determining a match, some calculations can be re-used for candidate primer sequences sharing a common region. For example, certain candidate matches 325 can be safely skipped. Such an arrangement can be used to implement the system shown in FIG. 1.

In the example, an off-target detection tool 350 employs a match finder 340 that applies the matching rules 320 to determine verified matches 360.

In practice, a *k*-mer index 370 for the transcript sequence 380 can be used to identify candidate matches 325 (e.g., the *k*-mer index of the transcript sequence can be searched for decomposed *k*-mers of the candidate primer sequences, and a hit indicates a candidate match). Some of the matches 328A, 328B are verified as verified matches 360, while others are discarded from consideration.

Example 7 - Example Method of Verifying Candidate Matches

FIG. 4 is a flowchart of an example method 400 of verifying candidate matches and can be implemented, for example, in a system such as that shown in FIG. 3.

At 430, a candidate match (e.g., location on the transcript sequence) can be identified (e.g., using the *k*-mer index to search for an occurrence of a k-mer of a candidate primer sequence to find if an initial matching rule such as Rule #1 described herein is satisfied or partially satisfied). The candidate match is then verified via the matching verification rules at 440. For example, the additional portions of the candidate primer sequence or further rules can be considered.

The method 400 can be performed for a plurality of candidate matches (e.g., the method is repeated for other candidate matches).

Example 8 - Example Rule Calculation Cache for Common Regions

FIG. 5 is a block diagram of an example system 500 having a rule satisfaction calculation cache for common regions within candidate primer sequences that can be used in any of the examples described herein. In the example, clusters 5 10A, 510B or candidate primer sequences 520A-F are associated with common regions 530A-B, which are then associated with locations on the transcript sequence 580.

The common regions 530A-B are regions (e.g., substrings, subsequences, or the like) of the candidate primer sequences that are shared among the candidates (e.g., the candidates contain identical substrings, subsequences, or the like).

The rule satisfaction calculation cache 540 is organized by the different common regions and stores rule satisfaction calculations 532A-B for respective of the common regions 530A-B that are associated with different respective clusters 510A-B of the input candidate primer sequences 520A-F. As described herein, certain candidate matches 538A, 538B can be safely skipped for the candidate primer sequences because a prior calculation has already determined that a matching rule was not satisfied (e.g., Rule #2 was not satisfied because there are too many mismatches).

Example 9 - Example Rule Satisfaction Calculation Cache

In any of the examples herein, calculations for determining whether the rules are satisfied can be cached for use by a plurality of candidate primer sequences in a rule satisfaction calculation cache (e.g., a matching rule satisfaction calculation cache). As described herein, common regions among candidate primer sequences can be determined.

Based on the logic of the rules, certain calculations concerning rule satisfaction can be reused. For example, if it is known that a common region has at least *k* consecutive matches, any candidate primer sequence containing such a region satisfies rule #1 (e.g., in can only have *k* or more consecutive matches). Therefore, the determination that the region satisfies rule #1 can be reused for candidate primer sequences having the common region. Similarly, if it is known that a common region has more than e * *l* mismatches, then any candidate primer sequence of length *l* will not satisfy rule #2 (e.g., it can have no more than e * *l* mismatches). Therefore, the determination that the region does not satisfy

rule #2 can be reused for candidate primer sequences having the common region.

Cached rule satisfaction calculations can include a stored location at which the calculation applies (e.g., a location on the reference genome sequence involved in the cached calculation, such as where a match occurs, where a mismatch occurs, or the like).

Multiple levels of the cache can store rule satisfaction calculations for different conditions or different lengths of sequences (e.g., *l*, *l*+1, *l*+3, or the like).

In practice, non-common regions can then be incorporated into the determination. For example, if the cache indicates that there are m mismatches in the common region, further mismatches can be added to *m* to determine the overall candidate primer sequence mismatches and calculate if the overall mismatches meet rule #2.

Thus, total rule satisfaction calculations (e.g., whether the condition of a rule is satisfied) or partial rule satisfaction calculations (e.g., partial calculations of whether the condition of a rule is satisfied) can be cached.

Example 10 - Example Method of Identifying Matches via Cache

FIG. 6 is a flowchart of an example method 600 of identifying matches for a candidate primer sequence via a cache and can be implemented, for example, in a system such as that shown in FIG. 5. In practice, such a method is typically performed by a match finder or other part of an off-target verification tool and can be performed as part of the method shown in FIG. 4.

A candidate primer sequence can be received when match processing begins.

At 630, a common region is identified for the candidate primer sequence. Associations between candidate primer sequences and common regions can be stored when the cache is built.

At 640, a rule satisfaction calculation of the common region is reused for the candidate match. In other words, the cache can be consulted instead of re-doing a calculation for rule satisfaction. For example, the calculation can be used to safely skip the candidate match (e.g., the candidate primer sequence cannot possibly match the location on the transcript sequence.) Or, the calculation can be used to confirm that the candidate primer sequence meets a rule condition.

The method 600 can be done for a plurality of candidate primer sequences. So, it can be repeated for other candidate primer sequences.

Example 11 - Example Method of Identifying Matches via Rule Satisfaction Calculation Cache

FIG. 7 is a flowchart of an example method 700 of building a cache for candidate primer sequences and can be implemented in any system employing a cache, such as that shown in FIG. 5. Cache building can be performed prior to or in conjunction with match processing (e.g., as shown in FIG. 4).

At 730, candidate primer sequences grouped into a cluster are received. In practice, it may be known that a set of candidate primer sequences are associated with a common origin, and they can be grouped into a cluster accordingly. Or, clustering can be performed by finding likely common regions among the sequences.

At 740, a common region is identified for the cluster. An incoming cluster may already have some initial indication of a common region or likely common region, or the candidate primer sequences can be aligned to determine a common region. The initial common region can be called a "seed" before it is extended.

In any of the examples herein, the common region can be extended as shown at 750. Computing resource increases can be balanced against computing resource decreases as a result of extending the common region. The advantages and disadvantages of extending the common region can be considered when determining whether to extend the region. For example, a computing resource increase for extending the region (e.g., the resources expended for building the cache) can be calculated, the computing resource decrease for extending the common region (e.g., the resources saved by searching with the cache) can be calculated, and the computing resource increase for not extending the region (e.g., the resources expended for searching without the cache) can be calculated. Deciding whether to extend the common region can be determined by balancing the computing resource increases against the computing resource decrease. For example, extending the common region may only reach a subset of candidate primer sequences in the cluster.

At 760, rule satisfaction calculations for the common region are stored as described herein. Such calculations can be associated with the common region in the cache for later use when processing candidate primer sequences having the common region. Similarly, associations between the common region and candidate primer sequences containing the common region can be stored.

The method 700 can be performed for a plurality of clusters. For example, it can be repeated for other clusters.

In any of the examples herein, the common region between a candidate primer sequence and another candidate primer sequence can be identified. A rule satisfaction calculation can be performed for the common region, and the rule satisfaction calculation can be stored in a cache. Based on the cache, the calculation can be skipped (e.g., for the candidate primer sequence). The cache can support multiple levels (e.g., for respective different lengths of candidate

primer sequences) as described herein.

Example 12 - Example System Implementing Multi-Level Cache

FIG. 8 is a block diagram of an example system 800 implementing a multi-level cache 810 and can be implemented in any of the examples herein using a cache.

In the example, the rule satisfaction calculation cache 810 is organized by common region 830A and includes separate rule satisfaction calculations 832AA and 832AB that are stored for different levels of the cache 810.

For example, calculations for different rules, or calculations for different parameters of the rules (e.g., different candidate primer sequence lengths) can be stored.

Various candidate matches for the common region and the transcript sequence 880 can be associated with the cache. Certain candidate matches 838A, 838B can be indicated as not meeting a rule and therefore can be safely skipped when processing other candidate primer sequences containing the common region. Those candidate primer sequences of different lengths can limit re-use of calculations to those appropriate for the rule (e.g., Rule #2 above incorporates a length component).

Example 13 - Example System Implementing *k*-mer Index

FIG. 9 is a block diagram of an example system 900 implementing a *k*-mer index 950. The example shows a basic implementation. In practice, any number of variations are possible. Any variety of *k*-mer index schemes can be employed for the technologies.

In the example, the index 950 comprises k-mer keys 952A-N and respective locations 954A-N at which the *k*-mer occurs in the transcript sequence 980. The locations can take the form of a list (e.g., of integers, pointers, or the like that specify a location in the transcript sequence 980).

Example 14 - Example Off-Target Predictor

In an implementation checking specificity of primers, off-target determination can be done with reference to whether the primers would amplify unintended regions of the genome. FIG. 14 is a block diagram of example off-target match conditions.

When unintended regions are amplified, an off-target match condition exists for the primers. A primer pair can comprise a forward primer and a reverse primer. When a primer pair binds at an unintended location, unintended amplification can result. Thus, detection of a match of one primer at a location on one strand of an amplicon derived from RNA or sequences transcribable from a reference genome in conjunction with detection of a match of another primer at a neighboring location on the other strand of the amplicon or corresponding transcript sequence indicates an off-target match condition. When the primer is from another pair, an off-target match condition still results and is called an "inter locus off target" condition. With multiplex PCR primer design, primer sets for several targets are designed simultaneously, making primer selection more complex and challenging.

A method of detecting off-targets can receive collected matches (e.g., matching locations for primers meeting the rule conditions) on the transcript sequence and check if there are matches within a threshold distance (e.g., off-target condition window length) of each other on the transcript sequence. Such a method can perform determining whether verified matching locations form an off-target match condition on a transcript sequence when considered in conjunction with at least one other match for at least one other candidate primer sequence. Reverse complements of primers can be included as described to account for the negative strand. Such collected matches that are not at a desired target location on the transcript sequence are considered an off-target match. One method of detecting off-target conditions can simply compare each match location to the other match locations (e.g., each other match location) to see if they are within the threshold distance, resulting in a computation of order $n^2$. Upon detection of two match locations within a threshold distance, further processing can be done (e.g., to confirm that the matches are on different strands of the transcript sequence) to confirm the off-target condition. The strand of a match can be stored as part of its representation (e.g., if the associated candidate primer is a reverse complement, then it is indicated to be a match on the negative strand; otherwise, it is a match on the positive strand). A set of matches at an intended target is not indicated as an off-target condition.

In any of the examples herein, the off-target condition window length can be equal to or substantially similar to that of the maximum expected length of the target nucleic acid molecules (e.g., typically 25-1000 base pairs in length, 200-1000, 500-1000, 200-800, or 300-700 base pairs in length) in a PCR reaction as described herein. A value of 1000 was used for the off-target condition window length in examples described herein. off-targets being score based on their length.

FIG. 10 is a block diagram of an example system 1000 implementing an off-target predictor and can be used

in any of the examples herein for a candidate primer sequence. Such a predictor can be used with implementations having or not having a cache. Before searching for matches, a number of matches can be predicted. A large number of matches is correlated with an off-target match. So, if the predicted number of matches meets a threshold, the candidate primer sequence can be discarded (e.g., skipped), thereby reducing the number of calculations and increasing performance.

One predictor takes the form of the following Calculation A using trained parameters a, b, c, and d:

$$y = e^{(a * \log x + b*l + c*\text{floor}[l*e] + d)}$$

where

- y: number of hits (+ or - strand, which are highly correlated)
- x: number of candidate hits (matches) returned by k-mer index for candidate primer sequence
- *l*: length of the candidate primer sequence
- e: fraction of mismatches allowed (from rule #2) or the mismatch rate allowed or the error rate allowed.

The parameters a, b, c, and d can be calculated from historical data. Linear regression can be used to fit the predictive model Calculation A to the observed data set of y and x hits. The parameters a, b, c, and d can be applied if an additional value of x is then given without its accompanying value of y, and the fitted model can be used to make a prediction of the value of y.

In the example, the off-target predictor 1050 accepts a candidate primer sequence 1010 as input and applies the parameters a, b, c, and d to a prediction engine 1060 (the calculation shown above) to generate a predicted number of matches on the transcript sequence. *l* and *x* can be derived from the candidate primer sequence 1010. If the matches meet (or exceed) a threshold, the candidate primer sequence can be discarded from consideration (e.g., matching processing need not be performed for the candidate primer sequence or its paired sequence). Thus, the off-target detection tool can store the threshold and apply it as described.

In any of the examples herein, the off-target prediction technologies can be used as a pre-filter to discard those candidate primers having more than a threshold number of hits. In one implementation involving the human genome, a threshold (e.g., off-target condition window length) of 1,000 was used, but other values in the range of 800-1200 (e.g., 900, 1100, or the like can be used). Other implementations involved transcripts transcribable from the human genome according to a gene model, including a corresponding threshold of 1000, or 800-1,200, or 900 or 1,100, or other threshold, higher or lower or intermediate, may be used. A prediction is generated for candidate primers as described herein, and if the number of predicted hits meets the threshold, the candidate primer is discarded from consideration (e.g., the cache need not be considered for the candidate primer sequence).

FIG. 23 depicts a block diagram showing results for applying match prediction via Calculation A described above using the human genome, with the parameters, before searching for matches. In the example, a threshold of 1000 matches was set. If the prediction for a particular candidate primer sequence met the threshold, it was discarded from consideration. Runtime improvement and dramatic reduction of memory usage resulted. The off targets checking time was reduced from 1 hour to 10 minutes. The straightforward method resulted in 5.5 seconds per primer; the cached method resulted in 0.38 seconds per primer; the prediction/filtering method resulted in 0.29 seconds per primer. By filtering 14% of the sequences, 56.4% of the matches (hits) were filtered. Filtering sequences with too many hits can reduce memory usage.

As shown in FIG. 24, more than 93% of the filtered sequences have more than 800 actual observed hits. Therefore, filtering based on the prediction generated by Calculation A can be considered valid.

Other thresholds of about 250, about 500, about 1000, about 1500, or about 2000 could also be used.

Thus, filtering of some candidate primer sequences can be accomplished by removing primer sequences that are predicted to have many hits (e.g., and thus are likely to result in an off-target match condition). The embodiments of FIGS. 10 and 11 can implement such an approach. Thus, in any of the examples herein, primers can be pre-filtered by removing those primers that are predicted to have a threshold number of hits (matches). Such a prediction can be generated by training a calculated result based on observations of actual matches (e.g., as it varies based on length of the primer). Any number of calculations generating a prediction can be used. The following Calculation A can be used as an example with parameters as described herein:

$$y = e^{(a * \log x + b*l + c*\text{floor}[l*e] + d)}$$

Any of the following embodiments can be implemented. For example, prefiltering of candidate primers can be achieved using the match prediction technologies of FIG. 10 and 11 in any multiplex PCR scenario, independent of the cache and sequence proximity groupings technologies. So, for a candidate primer sequence considered for inclusion as a primer in a multiplex PCR reaction, the sequence can be received, a prediction of a number of matches on the transcript sequence for the candidate primer sequence can be generated, and responsive to determining that the predicted number of matches exceeds a threshold, the candidate primer sequence can be discarded from consideration (e.g., filtered out). The calculation and thresholds can take the forms described herein.

Off-target detection via sequence proximity groupings can be applied in any multiplex PCR primer specificity evaluation scenario, independent of the cache and match prediction technologies. So, for a plurality of verified matches for a plurality of candidate primers, the verified matches can be placed into sequence proximity groupings as described herein. Such matches can be verified via techniques other than the cache techniques described herein (e.g., by applying matching rules without the cache described herein). The proximity groupings can then be checked to identify an off-target match condition.

Example 15 - Example Method of Off-Target Prediction

FIG. 11 is a flowchart of an example method 1100 of generating an off-target prediction for a candidate primer sequence and can be implemented, for example, in a system such as that shown in FIG.10. Such a method can be used with implementations using or not using a cache.

At 1130 a candidate primer sequence is received.

At 1140, a prediction of the number of matches on the transcript sequence is generated via applying the parameters to a prediction engine.

At 1150, the candidate primer sequence is discarded from consideration (e.g., the actual matches are not determined) responsive to determining that the predicted number of matches exceeds a threshold.

In practice, the method 1100 can be performed for a plurality of candidate primer sequences (e.g., it is repeated for other candidate primer sequences).

Example 16 - Example System Implementing Proximity Groupings

FIG. 12 is a block diagram of an example system 1200 implementing string or sequence proximity groupings and can be used in any of the examples herein to identify an off-target match condition. The off-target correlator 1250 can be incorporated into an off-target detection tool (e.g., as correlator 127 in tool 150 of FIG. 1). Sequence proximity groupings can be used in systems not having a cache.

The correlator 1250 accepts verified matches 1210 and intended targets 1220. In practice, the system can process verified matches 1210 for a large number of candidate primer sequences determined via any of the technologies described herein. The intended targets 1220 indicate the targets intended for the candidate primer sequences, which can be organized in pairs as described herein.

The correlator 1250 can create sequence proximity groupings 1260 that assist in determining whether a verified match for a candidate primer sequence is an off-target match. As described herein, such a determination can be made with reference to two transcript sequences for which processing has been performed; two sequences can be represented via a single sequence as described herein.

Based on the sequence proximity groupings 1260, the correlator 1250 can output an off-target determination 1280. Such a determination can indicate that a particular candidate primer sequence results in an off-target match. Other information such as where on the transcript sequence the off-target match occurs, whether it is an inter-locus off-target match, or the like can be included.

Example 17 - Example Method of Identifying Off-Target Match Condition via Proximity Groupings

FIG. 13 is a flowchart of an example method 1300 of identifying off-target matches via sequence proximity groupings and can be implemented, for example, in a system such as that shown in FIG. 12 (e.g., by an off-target correlator). Sequence proximity groupings can be used in methods using or not using a cache.

At 1330, a plurality of verified matches for a plurality of candidate primer sequences are received. As described herein, a verified match can include an indication of where on the transcript sequence the match occurs.

At 1340, the matches are placed or clustered into sequence proximity groupings according to where on the genome sequence the matches occur. The groupings can be based on an off-target condition window length.

At 1350, the sequence proximity groupings can be checked to identify an off-target match condition as described herein.

Example 18 - Example Sequence Proximity Groupings

In any of the examples herein, a transcript sequence can be divided into ranges of locations. The size of the ranges can be based on an off-target condition window length. Thus, a first group covers locations 1 through window_length, a second group covers locations window_length+1 through window _length*2, etc. The range for a group g is thus 1+(window_length * (g-1)) through (window_length * g).

The group contains a list of the verified matches that occur at a location within the range of the group. Checking for an off-target match pair can be simplified because checking need only be done between match pairs occurring in proximate locations (e.g., neighboring groups) of a transcript sequence. In this way, matches within an off-target condition window length's distance of each other can be identified and processed for detecting an off-target condition.

Example 19 - Example Implementation: Specificity Calculations for Primer Pairs

As described herein, a *k*-mer index can be applied, and intermediate results can be cached in the rule satisfaction calculation cache to reduce runtime without losing accuracy.

The task of specificity checking can proceed via two phases: searching primer hits (matches) and checking whether such matches result in an off-target match condition for two of the primers. Given a primer p with length *l* and a genome region *r*, *r* is a hit of the primer when it satisfies the following three conditions (matching rules): 1. There are at least *k* consecutive matches 2. there cannot be more then e * *l* mismatches in total and 3. There cannot be more than m mismatches on the 3' end of the primer. The conditions can be implemented as the matching rules as described herein. (As would be understood in this example, a T in a DNA amplicon from RNA or transcript transcribable from a reference genome according to a gene model would correspond to a U in the RNA molecule.)

```
GCAGCTGGTTGTGATCACGT
||||||||||xx|||||x|x
GCAGCTGGTTTGGATCAGGG
```

For example, transcript region r can be a hit when: 1. there are at least 6-10 (such as at least 6-8) consecutive matches, for example, at least 6, 7, 8, 9, or 10 consecutive matches, between the primer nucleotide sequence and the nucleotide sequence of transcript region r, 2. no more than 20% (such as no more than 15% or no more than 10%) of the primer nucleotides are mismatched between the primer nucleotide sequence and the nucleotide sequence of transcript region *r*, and 3. No more than 5 mismatches (such as no more than 4, no more than 3, or no more than 2 mismatches, or no more than 1 mismatch) between the primer nucleotide sequence and the nucleotide sequence of transcript region *r* are present (e.g., consecutively) on 20% of the primer (by nucleotides) from the 3' end of the primer. The 3' end of the primer can be defined as 5 base pairs long in some embodiments. In other embodiments, the 3' end of the primer can be defined as 1-5 base pairs long. For example, the cutoff can be no more than 3 mismatches in the last 5 base pairs or no more than 2 mismatches in the last three base pairs, dependent on the polymerase than the length of the primer. Typically, a 3' end mismatch could prevent amplification (the polymerase may not be able to extend from a mismatch). However, high-fidelity polymerases typically can chew back mismatching bases and resynthesize, thus correcting errors, but also increasing the chance an off-target is amplified.

Thus, the technologies allow specification of the total number of mismatches allowed as a percentage of the primer length between primer and targets. A custom region at the 3' can be defined, and the number of mismatches allowed in the region between the primer and targets can be specified. Specificities for multiple pre-existing primers can be determined. The technologies can scale to hundreds of thousands of primers.

Matches on the transcript strands can be considered candidate matches until the three Rules are verified as satisfied.

Example 20 - Example Implementation: Off-Target Determination

FIG. 15 is a block diagram of an example system 1500 employing sequence proximity groupings for off-target determination and can be used for the arrangements shown in FIGs. 12 or 13. In the example, the target sequence strands 1580 for the transcript sequence are represented by a transcript sequence set divided into ranges according to an off-target condition window length 1525A. The negative strand is represented by the transcript sequence 1580 in that the reverse complement of a primer is also included as a candidate primer sequence. Thus, off-target locations that would cause undesirable amplification or interference with amplification of target locations during the PCR process can be identified. In this way, sequence proximity groupings as described herein are implemented. In an alternative embodiment, two different sequences (reversed and complementary to each other) can be used to represent the different strands.

Verified matches against the strands 1580 are placed in lists 1520A-N according to where on the strand the verified match occurs. For example, the method of FIG. 2 can be performed for the primer sequences and the reverse complements of the primer sequences, resulting in verified matches for both strands. Off-target matches can then be identified using the lists.

Checking for off-target match conditions can be accomplished by checking 1530 matches within a same group and in neighboring groups. Because checking can proceed seriatim for the groups, in practice, a group can simply be checked against the next group (e.g., when processing the list 1520B, it is not necessary to check against list 1520A because processing for 1520A has already done so). For example, matches in the list 1520A can be checked against matches in the list 1520B to see if an off-target match condition exists (e.g., there are two primer hits within an off-target condition window length of each other that are not a desired target), and then matches in 1520B can be checked against 1520C and so forth. If so, the primer in the off-target match condition can be noted as involved in an off-target match condition. The primer pair can also be so noted.

The lists 1520A-N thus can function as an index of the matches to greatly speed up off-target detection processing.

Specificity can thus be calculated based on the number of off-target match conditions detected per primer or primer pair. Specificity can take the form of a counted number of off-target matches. Some applications may demand that a single off-target match is considered unacceptable. However, more complex statistical techniques can be applied depending on the application because it may not always be possible to find candidate primers that satisfy such stringent conditions.

Off-target prediction can be accomplished, where a candidate string takes the form of a candidate primer sequence. Such candidate primer sequences can be pre-filtered from further consideration when the prediction meets a threshold as described herein. For such pre-filtered sequences, the cache and off-target consideration calculations need not be performed. Such calculations can instead be skipped.

Example 21 - Example Further Description

FIG. 16 is a block diagram showing caching for common regions. In the example, seed sequences were found for primer clusters. The seed sequences were extended to common regions. The multi-level cache stores calculations for common regions that have *k* consecutive matches. Therefore, such common regions can be considered to satisfy rule #1 without having to re-calculate for other primers.

The multi-level cache stores calculations for common regions that have at most $e * l$ mismatches in total. Therefore, such common regions can be considered to fail rule #2 without having to re-calculate for other primers of length $l$. Another level of the cache stores calculations for common regions that have at most $e * (l + 1)$ mismatches in total. Therefore, such common regions can be considered to fail rule #2 without having to re-calculate for other primers of length $l$+1.

FIG. 17 is a block diagram showing skipped candidates via a cache. In the example, the space to search includes those primer sequences having a common region that is determined to satisfy rules #1 and #2. Those that failed rule #2 can be safely skipped. A new k-mer list can be checked for the region of the primer sequence outside of the common region.

FIG. 18 is a block diagram showing an arrangement 1800 for extending a common region for clustered primer sequences 1840. The line 1820 on the lower portion of the figure reflects the number of primers that have identical nucleotides at a particular location of a primer (e.g., when the primers are aligned by overlapping regions). In the example, an initially discovered common region 1825 (e.g., sometimes called a "seed sequence") is being considered for extension. The number of primer sequences 1820 sharing the same value at a location can be considered as described herein when determining whether calculations will increase or decrease. In some cases, extending the common region 1825 will result in logically separate common regions, some of which are shared by different of the primers 1840.

Example 22 - Example Implementation Results: Cache

Implementation of a cache allowed searching of some sequences with the cache. Some candidates could be verified or skipped via the cache, resulting in a 10-fold speedup in determination time.

A straightforward method did not use a cache, filtering, or sequence proximity groupings. Instead, the approach simply decomposed the primer into k-mers, searched a k-mer index for position lists, took the union of all the lists, and then verified the candidates to get final results. This approach could have been optimized with bit operation. Such an approach took 5.5 seconds per primer sequence on average, which resulted in 175 hours running time for 115,116 primer sequences (with 687 targets).

FIG. 19 is a block diagram showing results with a rule satisfaction cache. In the example (using a human

reference genome sequence, as an example, but transcript sequences transcribable from a human reference genome sequence could equally be used), 96.9 % of sequences could be searched with the cache, of which 32.5% were verified candidates, and 67.5% were skipped candidates. The resulting time to complete the determination was 0.38 seconds per primer, resulting in a 10-fold speed up over 5.5 seconds per primer for the straightforward method (e.g., without cache).

Example 23 - Example Implementation Results: Off-target Prediction

FIG. 20 is a block diagram showing correlation between hits on positive and negative strands of a reference human genome sequence. As shown, a primer's number of hits on the positive strand and the number of hits on the negative strand can be usually highly correlated, for example on the human genome. Therefore, a prediction for one strand can be used for both strands without negative consequences. Thus, the predictor as shown herein can generate a single prediction for a single strand and be used to filter candidate primer sequences without over or under filtering. Comparable analysis would apply if using transcripts transcribable from a reference human genome according to a gene model.

FIG. 21 is a block diagram showing correlation between number of candidates and number of hits for different sequence lengths. As shown, correlation is present across different sequence lengths. The observed phenomenon of correlation between sequence length of the primer and number of actual hits on the reference human genome sequence (e.g., for a variety of sequence lengths) can be used as a basis for constructing a predictor based on sequence length as described herein. Comparable analysis would apply if using transcripts transcribable from a reference human genome according to a gene model in place of the reference human genome.

FIG. 22 shows historical data of number of hits versus a prediction (e.g., predicted number of hits) using Calculation A described above. In the example, the human genome was used, and training resulted in the parameters shown. The parameters used were a=1.97, b=1.23, c=1.96, d=-4.43. Using such parameters, the number of matches (hits) for a primer can be predicted before searching for matches. The historical data establishes that the predictor is accurate due to the strong correlation between actual number of matches and predicted number of matches evident in the figure. The parameters can be derived based on historical data and may vary depending on which version of the genome is used. Comparable analysis would apply if using transcripts transcribable from a reference human genome according to a gene model in place of the reference human genome.

Example 24 - Further Combinations

Further, the technologies can be combined so that caching, filtering by match prediction, and sequence proximity groupings operate together. In such an example, a computer-implemented method of identifying off-target matches on a transcript sequence includes receiving a candidate primer sequence; for the candidate primer sequence, identifying a plurality of candidate matching locations on the transcript sequence; out of the candidate matching locations, identifying verified matching locations on the transcript sequence, wherein identifying verified matching locations comprises determining which of the candidate matching locations on the transcript sequence satisfy one or more matching verification rules and reusing a rule satisfaction calculation already calculated for a different candidate primer sequence sharing a common region with the candidate primer sequence; and determining whether the verified matching locations form an off-target match condition on the transcript sequence when considered in conjunction with at least one other match for at least one other candidate primer sequence; wherein the method further comprises filtering at least one additional candidate primer sequence, wherein the filtering comprises generating a prediction of a number of matches on the transcript sequence for the additional candidate primer sequence and, responsive to determining that the number of matches exceeds a threshold, discarding the additional candidate primer sequence; wherein the method further comprises placing the verified matches into sequence proximity groupings; and checking the proximity groupings to identify the off-target match condition.

Example 25 - Example Computing Systems

FIG. 25 illustrates a generalized example of a suitable computing system 2500 in which several of the described innovations may be implemented. The computing system 2500 is not intended to suggest any limitation as to scope of use or functionality, as the innovations may be implemented in diverse computing systems, including special-purpose computing systems. In practice, a computing system can comprise multiple networked instances of the illustrated computing system.

With reference to FIG. 25, the computing system 2500 includes one or more processing units 2510, 2515 and memory 2520, 2525. In FIG. 25, this basic configuration 2530 is included within a dashed line. The processing units 2510, 2515 execute computer-executable instructions. A processing unit can be a central processing unit (CPU), processor in an application-specific integrated circuit (ASIC), or any other type of processor. In a multiprocessing system,

multiple processing units execute computer-executable instructions to increase processing power. For example, FIG. 25 shows a central processing unit 2510 as well as a graphics processing unit or co-processing unit 2515. The tangible memory 2520, 2525 may be volatile memory (e.g., registers, cache, RAM), non-volatile memory (e.g., ROM, EEPROM, flash memory, etc.), or some combination of the two, accessible by the processing unit(s). The memory 2520, 2525 stores software 2580 implementing one or more innovations described herein, in the form of computer-executable instructions suitable for execution by the processing unit(s).

A computing system may have additional features. For example, the computing system 2500 includes storage 2540, one or more input devices 2550, one or more output devices 2560, and one or more communication connections 2570. An interconnection mechanism (not shown) such as a bus, controller, or network interconnects the components of the computing system 2500. Typically, operating system software (not shown) provides an operating environment for other software executing in the computing system 2500, and coordinates activities of the components of the computing system 2500.

The tangible storage 2540 may be removable or non-removable, and includes magnetic disks, magnetic tapes or cassettes, CD-ROMs, DVDs, or any other medium which can be used to store information in a non-transitory way and which can be accessed within the computing system 2500. The storage 2540 stores instructions for the software 2580 implementing one or more innovations described herein.

The input device(s) 2550 may be a touch input device such as a keyboard, mouse, pen, or trackball, a voice input device, a scanning device, or another device that provides input to the computing system 2500. For video encoding, the input device(s) 2550 may be a camera, video card, TV tuner card, or similar device that accepts video input in analog or digital form, or a CD-ROM or CD-RW that reads video samples into the computing system 2500. The output device(s) 2560 may be a display, printer, speaker, CD-writer, or another device that provides output from the computing system 2500.

The communication connection(s) 2570 enable communication over a communication medium to another computing entity. The communication medium conveys information such as computer-executable instructions, audio or video input or output, or other data in a modulated data signal. A modulated data signal is a signal that has one or more of its characteristics set or changed in such a manner as to encode information in the signal. By way of example, and not limitation, communication media can use an electrical, optical, RF, or other carrier.

The innovations can be described in the general context of computer-executable instructions, such as those included in program modules, being executed in a computing system on a target real or virtual processor. Generally, program modules include routines, programs, libraries, objects, classes, components, data structures, etc. that perform particular tasks or implement particular abstract data types. The functionality of the program modules may be combined or split between program modules as desired in various embodiments. Computer-executable instructions for program modules may be executed within a local or distributed computing system.

For the sake of presentation, the detailed description uses terms like "determine" and "use" to describe computer operations in a computing system. These terms are high-level abstractions for operations performed by a computer, and should not be confused with acts performed by a human being. The actual computer operations corresponding to these terms vary depending on implementation.

A computer system structured for performing RNA alignment methods as further disclosed herein is also provided. A computer system may include a processor or processors, such as a microprocessor or microprocessors, capable of executing code for performance of the methods as described. The computer system may also have a storage device or devices such as hard drives for storage of information such as a reference genome sequence, transcript sequences transcribable from the reference genome, sequences of primers in a set of primers, targets transcribable from the transcript sequences of the reference genome with the prime sequences of the set of primers, a modified reference genome including the amplified target sequences, and sequence read files corresponding to the reads obtained from a test sample's or samples' RNA. The microprocessor or microprocessors are in communication with the storage unit or units, from which the microprocessor(s) access information stored therein, and in which sequence and other data generated by the microprocessor or microprocessors when executing the method may be stored. The computer system may have a cache for temporary storage of information generated and accessed during RNA alignment, and a RAM for execution of code used in performing aspects of the methods.

A computer system may be a part of other hardware, such as a computer system included with or as part of a sequencing apparatus, or could be separate from such other apparatus. A computer system could also be self-contained or it could be networked on a network system, with a processor and a storage unit in different locations but in communication with each other across a network. A network may be wired or may be wireless or may incorporate both forms of connectivity. Some portions of a computer system may be included with or a part of a sequencing or other apparatus while other portions of the computer system may be separate, while all aspects of the computer system communicate either in wired communication or wirelessly. A computer system could also be a cloud-based system, where certain components of the system are in one location and other components are in another location, and the components communicate with one another through the internet.

Example 26 - Computer-Readable Media

Any of the computer-readable media herein can be non-transitory (e.g., volatile memory such as DRAM or SRAM, nonvolatile memory such as magnetic storage, optical storage, or the like) and/or tangible. Any of the storing actions described herein can be implemented by storing in one or more computer-readable media (e.g., computer-readable storage media or other tangible media). Any of the things (e.g., data created and used during implementation) described as stored can be stored in one or more computer-readable media (e.g., computer-readable storage media or other tangible media). Computer-readable media can be limited to implementations not consisting of a signal.

Any of the methods described herein can be implemented by computer-executable instructions in (e.g., stored on, encoded on, or the like) one or more computer-readable media (e.g., computer-readable storage media or other tangible media) or one or more computer-readable storage devices (e.g., memory, magnetic storage, optical storage, or the like). Such instructions can cause a computing device to perform the method. The technologies described herein can be implemented in a variety of programming languages.

Example 27 - Alignment of RNA to a Modified Reference Genome

FIG. 26 shows a flowchart 2600 for RNA alignment as disclosed herein. Transcript sequences and primer sequences may be received 2610 into a data storage unit or units of a computer system. The primers may have been selected or designed for amplification of selected targets or proposed for identification of unknown targets which they may be determined to be useful for amplification, or a combination of both. Transcript sequences include transcripts that could be transcribed from the reference genome according to a gene model. Depending on the structure and parameters of the gene model, the transcript sequences will contain primary transcripts that may be transcribed from the reference genome, based on sequence information contained in the reference genome that indicates what sequences correspond to transcribed regions may also include information pertaining to splicing events predicted to occur in transcripts and RNA fusion events known, predicted, or hypothesized to occur from among the transcripts, or may include all of the above. As would be understood, it is not necessary that primer sequences and a modified reference genome be received together, as either may be prepared and provided separately from the other.

Target sequences amplifiable from the modified reference genome are then generated 2620. A microprocessor determines target sequences on the transcript sequences that would be predicted to be amplified from an RNA test sample from a given set of primers if the transcript sequences were present in the RNA test sample. A modified reference genome is then generated 2630 from the target sequences amplifiable from the transcript sequences. The modified reference genome includes target sequences predicted to be generated from the transcript sequences of the reference genome. Some of the targets may be on-target. Some may be off-target sequences, depending on whether off-target sequences were predicted to be generated during generation of target sequences and, if so, the parameters adopted for generation of modified reference genome permitted inclusion of sequences determined to be off-target sequences therein.

A sequence read file or files are then received 2640 into a storage unit and aligned with the modified reference genome 2650 by a microprocessor using alignment software. The aligning software may generate an alignment profile 2660 that can include placement, a quality score, and sequence integrity, or other characteristics or metrics of the sequence reads.

Example 28 - Matching Primers to Transcript Sequence

FIG. 27 shows an example of a process for determining matches of primers from a primer set to transcript sequences for the generation of targets in creating a modified reference genome. Shown is a transcript sequence and highlighted within the transcript sequence are sequences to which various primers have matching sequences, some forward-oriented (fwdA1, fwdB1', fwdB1, fwdB2, and fwdA3) and other reverse-oriented (revA1, revB1, revA2, revB2, revA3). Beginning at the 3' end of the transcript sequence potential primer match sites may be identified. As a forward-oriented primer match site is identified the primer and its location is cached, then other primers may be checked for potential match sites downstream from that of the first. If a match site for another, reverse-oriented primer is identified, it may be referenced to prior cached primer locations to determine whether a target that meets parameters for inclusion of target sequences in a modified reference genome (e.g., minimum length) are satisfied. If so, the target may be included in the modified reference genome. A forward primer can be removed from cache once sites to which primers are being matched to the transcript sequence are far enough along down the transcript sequence that any target sequence that would be amplifiable between the cached primer and the currently matching primer would exceed maximum target sequence length parameters.

For example, with reference to the transcript sequence and primers in FIG. 27, primer matching would begin at the 3' (upper-left) end of the transcript sequence, to which primer sequence fwdA1 is a match and therefore would

be added to cache. Moving down the transcript sequence in the 3'-to'5' direction, revA1 would be added to cache. Although fwdA1 and revA1 are a pair of facing primers, a target sequence of fwdA1-to-revA1 would not be added to the modified reference genome in this case as its length is below the minimum target sequence length selected for this example (25 bases). Next fwdB1' would be added to the cache, and a sequence of revA1-to-fwdB1' would be added to the modified reference genome, as would, next, revA1-to-fwdB1. Next, revB1 would be added, checked against fwdA1 and revA1, and fwdA1-to-revB1 would be added to the modified reference genome. revA2 would then be added to cache. fwdB2 would then be checked against fwdA1, revA1, revA2, and fwdB2-to-revA1 and fwdB2-to-revA2 would be added to the modified reference genome. revB2 would then be added, and checked against fwdA1, revA1, and revA2, and fwdA1-to-revB2 would be added to the modified reference genome. Because this is the longest acceptable target sequence length in this example (200 bases), fwdA1 could be discarded from checks against subsequent primer matches downstream from revB2.

Example 29 - Assigning Loci to Primers

FIG. 28 shows an example of how a locus or loci are assigned to a primer or primers. If a primer sequence matches to only one transcript sequence locus, it is assigned that locus. If a primer sequence matches to two transcript sequence loci, its assigned locus depends on the primer with which it is paired in amplifying a target (i.e., the oppositely oriented primer with which it pairs in amplifying a transcript sequence). If there is only one transcript sequence locus to which both primers match, that locus is assigned the primers. In the event a primer would be assigned multiple loci according to the foregoing rules, it is assigned the locus having the first loci ID according to alphabetical order.

For example, in FIG. 28, loci are assigned to 7 primer pairs across 4 loci. For the pair of primers forward_1_2 and reverse_1, both are assigned locus 1 because that is the only locus to which primer reverse_1 matches. For primers forward_1_2 and reverse_2_3, they are both assigned locus 2 because that is the only locus to which both match. For primers forward_3 and reverse_2_3, they are both assigned locus 3 because that is the only locus to which they both match. For primers forward_4 and reverse_4 they are assigned locus 4 because that is the only locus to which either primer matches. For primer forward_3 and reverse_1, they are assigned loci 3 and 1, respectively, because each matches to only 1 locus but not to the same locus. For primers forward_4 and reverse_2_3, they are assigned to different loci because there is no single loci to which they both match, primer forward_4 is assigned locus 4 because that is the only locus to which it matches, and primer reverse_2_3 is assigned locus 2 because of the loci to which it matches locus 2 comes first in alphabetical order. And for primers forward _1_2 and reverse_4, they are assigned to different loci because there is no single loci to which they both match, primer forward_1_2 is assigned locus 1 because of the loci to which it matches locus 1 comes first in alphabetical order, and primer reverse_4 is assigned locus 4 because that is the only locus to which it forms a match.

Example 30 - Filtering Cross-Loci Targets

FIG. 29 shows a schematic of an example for filtering out expected cross-loci targets. Cross-loci targets would be expected where some primer pairs of the set of primers would be predicted to amplify targets that are in relative proximity to one another. In such cases, a subset of the primers responsible for amplifying the targets could also combine to amplify a larger target, from multiple loci, that would include the two original targets. Three intended targets are shown, flanked by their respective upstream locus-specific oligo (ULSO) and downstream locus-specific oligo (DLSO). Other combinations of ULSO and DLSO than the combinations used to amplify the intended targets are also possible from among the 6 primers used to amplify them, as shown below. For example, a cross-locus target could be amplified using the ULSO from the left-most intended target and the DLSO from the rightmost intended target, which cross-locus target would encompass the sequences of all of the targets. Likewise a cross-locus target could be amplified that encompasses to the two rightmost intended targets, or the two left-most intended targets. Such off-target cross locus targets may be filtered out of the modified reference genome. For example, if an off-target sequence has a ULSO and DLSO that match to separate intended targets and is larger than either of the targets, it can be filtered out of the modified reference genome as a cross-locus target.

Example 31 - Identification of Amplicons from Various Transcripts

FIG. 30 is a schematic of different amplifiable targets that could be generated from different RNA transcripts that share some sequences (e.g., some exons) but not others. Different pairs of primers would be predicted to amplify sequences from one, the other, or both transcripts. In generating a modified reference genome, references are maintained as to which targets may be amplified from which transcript sequences of the reference genome. For example, primers greenA and greenB would amplify identical sequences from the red and the blue transcripts, whereas primers orangeA and orangeB/yellowB would amplify sequences from the red and blue transcripts that differ from each other (due to the

presence of intervening exon 3 in the blue transcript but not the red transcript), and primers yellowA and orangeB/yellowB would amplify a sequence from the blue transcript but not the red transcript (because primer yellowA forms a match to a sequence of exon 3).

Example 32 - Translating Sequence Reads Aligned to Targets Derived From Splice and Fusion Junctions

In some examples, reads as aligned to a modified reference genome may be further aligned to a reference genome from which the modified reference genome was generated, such as by identification of transcribable transcript sequences based on a gene model as disclosed herein. In some instances, an RNA read whose sequence crosses an exon-exon boundary will align to a modified reference genome. For example, a read may be identified as corresponding to a given target from a modified reference genome. Such target may include exon-exon junctions, as reflected in contiguous portions of sequence within the read. It may be desirable to identify loci within the reference genome from which the modified reference genome was derived that correspond to the read. A modified reference genome may include corresponding information of where on a given chromosome from the reference genome its sequence--in particular, for example, its exons--were derived. It would be understood that such exonic sequences may be separated by untranscribed portions of the reference genome, or transcribed portions of the genome that correspond to intronic sequences removed during splicing. When a read is aligned to a modified reference genome containing such genomic locus identification, the read may be aligned not only to the modified reference genome but translated back to the corresponding location in the modified reference genome, to indicate what portions of the genome were transcribed in order to give rise to the portions of the read.

An example is illustrated in FIG. 31. FIG. 31 shows an pictorial representation of a process of translating an RNA read to chromosomal loci corresponding to sites from which portions of the RNA read were transcribed 3100. In this example, sn RNA read 3110 is aligned to a modified reference genome target 3120. In this target, t, several exons, $3120_A$, $3120_B$, 3120c, $3120_D$, and $3120_E$, are present. RNA read 3110 aligns to boundaries between these exons. Modified reference genome 3120 includes locus-identifiers indicating loci on the reference genome 3130 to which its exons correspond, i.e., from where on a given chromosome in the reference genome 3130 it was transcribed. RNA read 3110 aligning to target t in the modified reference genome can be translated back to the reference genome 3130, chromosome c, and specific loci within the chromosome, *l*, encoding the aligned exons identified. In some examples, an alignment profile may be created including placement information identifying chromosomal locations corresponding to sequences contained in RNA reads.

In some examples, an RNA read may correspond to a target lacking exon-exon boundaries, or to a portion of a target lacking such boundaries, such as where the target or read consists of a single exon or a sequence within a single exon. Such reads could also be translated back to the reference genome in a comparable manner as illustrated in FIG. 31. In other examples, an RNA read may align to a target corresponding to a fusion RNA, including sequences that were fused together from transcripts that originated as separate RNA molecules upon initial transcription. When a modified reference genome includes such potential fusion targets, and corresponding chromosomal loci-identifying information, portions of an RNA read corresponding to such fusion RNA targets may also be translated back to chromosomal locations in the reference genome, comparably to how an RNA read spanning exon-exon boundaries may be translated back to a reference genome as illustrated in FIG. 31. Such cases may include translating portions of the read back to different chromosomes. Where a sequence of a read aligns to a portion of a fusion RNA that does not include a fusion junction, it may also be translated back to the locus of its chromosomal origin as well.

Example 33 - Aligning Unaligned Fusion Junctions to a Reference Genome.

As disclosed herein, a sequence read might not be alignable or aligned to a modified reference genome, such as if the sequence read corresponds to a fusion junction and the fusion junction was not included in the gene model used for generation of the modified reference genome. In such cases, sequence reads classified as unaligned fusion junctions after non-alignment to a modified reference genome may be aligned to a reference genome. Provided such alignments satisfy minimum requirements to avoid characterization of a sequence read as a fusion junction false positive, the sequence read may be characterized as a fusion junction and it may be included in an alignment profile as such.

In an example, sequence reads were generated from each of four samples, two known to lack fusion junctions and two known to possess fusion junctions. Eight replicates of each sample were used, yielding 32 samples total. After aligning sequence reads of the samples to a modified reference genome as disclosed herein, unaligned fusion junctions were identified. These unaligned fusion junctions were then aligned to a reference genome. Some were subsequently confirmed as corresponding to fusion junctions present in the samples (i.e., fusion junctions not present in the gene model and thus not aligned or alignable to the modified reference genome, but aligned and accurately identified as fusion junctions to the reference genome). Fusion junctions that had been independently confirmed as being present in some samples, and which had been classified as unaligned fusion junctions following alignment to a modified reference

genome, were correctly identified as fusion junctions present in the sample after subsequent alignment to a modified reference genome.

Others were characterized as fusion junction false positives after aligning to the reference genome. For example, their fusion alignment length either did not exceed a minimum fusion alignment length threshold, or an insufficiently low number of corresponding sequence reads were present, or the ratio of a sequence read's alignment length to that of a local alignment length was not greater than 1. In an example, over 2,100 sequence reads (2,165) aligned to the reference genome as if they were fusion junctions but they were confirmed not to accurately represent fusion junctions present in the sample. However, upon screening them for classification of fusion junction false positives as disclosed herein, over 2,100 of them (2,107) were correctly classified as fusion junction false positives. Specifically, such sequence reads were classified as fusion junction false positives if they satisfied any one or more of the following three criteria: (1) sequence read fusion alignment length did not exceed 70 nucleotides, (2) there were not more than 100 sequence reads corresponding to the purported fusion junction, and/or (3) the fusion alignment length was not at least as long as the alignment length or a read to the location with a higher alignment score than any other read aligned there.

For the above example, FIG. 32 shows plots of fusion junction false positives in a manner that may permit identification and elimination of a number of false positives obtained. Of the 2,165 false positives identified in the above example, plotted in FIG. 32 are those with sequence read lengths of greater than 70, according to the following rules.

For a sequence read initially identified as a fusion junction, region (or, for a purported fusion junction, the non-contiguous regions) in a reference genome to which it aligns were identified. The length of reference genome to which the fusion junction aligned (combined length of alignment at each end of the sequence read fusion junction alignment) was determined. If the sequence read was alternatively alignable to a contiguous region of the reference genome, referred to as the sequence read's local alignment, the length of such local alignment was determined, referred to as the local alignment length. If more than one local alignment was potentially alignable, the local alignment with the longest local alignment length was selected for the local alignment length. A ratio was then calculated for each sequence read initially identified as a fusion junction. The numerator of such ratio was the alignment length of the purported fusion junction, and the denominator of such ration was the local alignment length. This ratio is plotted along the x-axis of the plot shown in FIG. 32. In this example, any purported fusion junction with a ratio of 1 or less (vertical line) was identified as a false positive.

Furthermore, the number of sequence reads corresponding to each purported fusion junction was also identified, plotted on the y-axis in FIG. 32. In this example, a purported fusion junction was identified as a false positive if the number of corresponding sequence reads indicating such fusion junction was not more than 100 (horizontal line).

Lines on the plot in FIG. 32 indicate fusion junction false positive criteria used in this example (in addition to alignment length of greater than 70): ratio of alignment length to local alignment length of greater than 1 (vertical line), and number of reads of greater than 100 (horizontal line). Many fusion junction false positives are plotted outside of these exclusion criteria (i.e., to the left of the vertical line and below the horizontal line) and thereby identified as false positives and not finally identified as indicating fusion junctions.

Alternatives

The technologies from any example can be combined with the technologies described in any one or more of the other examples. In view of the many possible embodiments to which the principles of the disclosed technology may be applied, it should be recognized that the illustrated embodiments are examples of the disclosed technology and should not be taken as a limitation on the scope of the disclosed technology. Rather, the scope of the disclosed technology includes what is covered by the following claims.

Although preferred embodiments have been depicted and described in detail herein, it will be apparent to those skilled in the relevant art that various modifications, additions, substitutions, and the like can be made within the scope of the present disclosure as defined in the claims that follow.

## Claims

**1.** A computer-implemented method of aligning RNA comprising:

receiving onto a data storage unit a plurality of primer sequences and a plurality of transcript sequences transcribable from a reference genome;
analyzing, using a microprocessor, the plurality of primer sequences to determine a plurality of target sequences amplifiable from the plurality of transcript sequences by the plurality of primer sequences;
generating, using a microprocessor, a modified reference genome based on the plurality of target sequences, wherein the modified reference genome comprises the plurality of target sequences and excludes sequences

not amplifiable from the plurality of transcript sequences by the plurality of primer sequences;
aligning, using a microprocessor, sequence reads generated from a test sample comprising RNA amplicon molecules to the modified reference genome, wherein the RNA amplicon molecules are generated by amplifying RNA sequences in the test sample using primers having sequences of the plurality of primer sequences; and
generating an alignment profile for the test sample based on the aligning.

2. The method of claim 1, further comprising assigning primer sequences individual loci corresponding to loci of respective transcript sequences, optionally further comprising removing one or more of the generated target sequences based on the one or more of the generated target sequences spanning more than one on-target sequence, or optionally wherein the plurality of primer sequences comprises a plurality of primer pairs, and a first primer pair comprises a first primer and a second primer for a first locus, and a second primer pair comprises the first primer and a second primer for a second locus.

3. The method of claim 1, wherein the transcript sequences are transcribable from the reference genome according to a gene model, wherein the gene model comprises identification of splice junctions, fusion junctions, or both, in the transcript sequences transcribable from the reference genome, optionally further comprising translating sequence reads aligned to targets derived from splice and fusion junctions or wherein the alignment profile comprises a fusion junction and the fusion junction was identified in the gene model.

4. The method of claim 1, wherein the plurality of target sequences comprises on-target sequences and off-target sequences; optionally further comprising reducing a number of off-target sequences by excluding one or more primer sequence from the plurality of primer sequences.

5. The method of claim 1, further comprising computationally comparing gene expression of two or more samples, wherein aligned reads generated from a first sample of RNA are compared to aligned reads generated from a second sample of RNA, wherein the alignment is performed using the plurality of target sequences.

6. The method of claim 1, wherein the alignment profile includes at least one of placement, a quality score, and sequence integrity for the sequence reads of the test sample.

7. The method of claim 1, further comprising:
translating the sequence reads from the test sample to a whole reference genome using the mapped target sequences and the modified reference genome.

8. The method of claim 1, wherein generating an alignment profile further comprises aligning a sequence read comprising an unaligned fusion junction to non-contiguous sequences of the reference genome.

9. A computer-implemented method of aligning RNA comprising:

receiving onto a data storage unit a plurality of primer sequences and a plurality of transcript sequences from a reference genome, the transcript sequences being transcribable from the reference genome according to a gene model comprising identification of splice junctions, fusion junctions, or both, in the reference genome;
assigning primer sequences individual loci corresponding to loci of respective transcript sequences;
analyzing, using a microprocessor, the plurality of primer sequences to determine a plurality of target sequences amplifiable from the plurality of transcript sequences by the plurality of primer sequences;
generating, using a microprocessor, a modified reference genome based on the plurality of target sequences, wherein the modified reference genome comprises the plurality of target sequences and excludes sequences not amplifiable from the plurality of transcript sequences by the plurality of primer sequences;
aligning, using a microprocessor, sequence reads generated from a test sample comprising RNA amplicon molecules to the modified reference genome, wherein the RNA amplicon molecules are generated by amplifying RNA sequences in the test sample using primers having sequences of the plurality of primer sequences;
generating an alignment profile wherein the alignment profile includes at least one of placement, a quality score, and sequence integrity for the sequence reads of the test sample; and
translating the sequence reads from the test sample to a whole reference genome using the mapped target sequences and the modified reference genome.

10. A computer system for aligning RNA comprising:

one or more microprocessors; and
one or more memories storing a plurality of primer sequences and a plurality of transcript sequences transcribable from a reference genome,
the one or more memories storing instructions that, when executed by the one or more microprocessors, cause the computer system to:

analyze the plurality of primer sequences to determine a plurality of target sequences amplifiable from the plurality of transcript sequences by the plurality of primer sequences;
generate a modified reference genome based on the plurality of target sequences, wherein the modified reference genome comprises the plurality of target sequences and excludes sequences not amplifiable from the plurality of transcript sequences by the plurality of primer sequences;
align sequence reads generated from a test sample comprising RNA amplicon molecules to the modified reference genome, wherein the RNA amplicon molecules are generated by amplifying RNA sequences in the test sample using primers having sequences of the plurality of primer sequences; and
generate an alignment profile for the test sample based on the aligning.

**11.** The computer system of claim 10, wherein the instructions cause the computer system to assign primer sequences individual loci corresponding to loci of respective transcript sequences, optionally wherein the instructions cause the computer system to remove one or more of the generated target sequences based on the one or more of the generated target sequences spanning more than one on-target sequence or wherein the plurality of primer sequences comprises a plurality of primer pairs, and a first primer pair comprises a first primer and a second primer for a first locus, and a second primer pair comprises the first primer and a second primer for a second locus.

**12.** The computer system of claim 10, wherein the transcript sequences are transcribable from the reference genome according to a gene model, wherein the gene model comprises identification of splice junctions, fusion junctions, or both, in the transcript sequences transcribable from the reference genome, optionally, wherein the alignment profile comprises a fusion junction and the fusion junction was identified in the gene model.

**13.** The computer system of claim 10, wherein the plurality of target sequences comprise on-target sequences and off-target sequences.

**14.** The computer system of claim 13, wherein the instructions cause the computer system to reduce a number of off-target sequences by excluding one or more primer sequence from the plurality of primer sequences; optionally wherein the instructions cause the computer system to compare gene expression of two or more samples, whereby aligned reads generated from a first sample of RNA are compared to aligned reads generated from a second sample of RNA.

**15.** The computer system of claim 10, wherein generating an alignment profile further comprises aligning a sequence read comprising an unaligned fusion junction to non-contiguous sequences of the reference genome

**Patentansprüche**

**1.** Computerimplementiertes Verfahren zum Alignieren von RNA, Folgendes umfassend:

Empfangen einer Vielzahl von Primersequenzen und einer Vielzahl von Transkriptionssequenzen, die von einem Referenzgenom transkribierbar sind, auf einer Datenspeichereinheit;
Analysieren der Vielzahl von Primersequenzen unter Verwendung eines Mikroprozessors zum Bestimmen einer Vielzahl von Zielsequenzen, die aus der Vielzahl von Transkriptionssequenzen durch die Vielzahl von Primersequenzen amplifizierbar sind;
Erzeugen, unter Verwendung eines Mikroprozessors, eines modifizierten Referenzgenoms auf der Grundlage der Vielzahl von Zielsequenzen, wobei das modifizierte Referenzgenom die Vielzahl von Zielsequenzen umfasst und Sequenzen ausschließt, die durch die Vielzahl von Primersequenzen nicht aus der Vielzahl von Transkriptionssequenzen amplifizierbar sind;
Alignieren, unter Verwendung eines Mikroprozessors, von Sequenzlesungen, die von einer Testprobe erzeugt werden, die RNA-Ampliconmoleküle umfassen, an dem modifizierten Referenzgenom, wobei die RNA-Ampliconmoleküle durch Amplifizieren von RNA-Sequenzen in der Testprobe unter Verwendung von Primern, die Sequenzen der Vielzahl von Primersequenzen aufweisen, erzeugt werden; und

Erzeugen eines Alignierungsprofils für die Testprobe auf der Grundlage des Alignierens.

**2.** Verfahren nach Anspruch 1, ferner umfassend das Zuordnen von Primersequenzen zu einzelnen Loci, die Loci von jeweiligen Transkriptionssequenzen entsprechen, optional ferner das Entfernen einer oder mehrerer der erzeugten Zielsequenzen auf der Grundlage, dass der einen oder der mehreren der erzeugten Zielsequenzen mehr als eine On-Target-Sequenz überspannen, umfassend, oder optional, wobei die Vielzahl von Primersequenzen eine Vielzahl von Primerpaaren umfasst, und ein erstes Primerpaar einen ersten Primer und einen zweiten Primer für einen ersten Locus umfasst, und ein zweites Primerpaar den ersten Primer und einen zweiten Primer für einen zweiten Locus umfasst.

**3.** Verfahren nach Anspruch 1, wobei die Transkriptionssequenzen aus dem Referenzgenom gemäß einem Genmodell transkribierbar sind, wobei das Genmodell eine Identifizierung von Spleißverbindungen, Fusionsverbindungen oder beidem in den aus dem Referenzgenom transkribierbaren Transkriptionssequenzen umfasst, optional ferner umfassend das Übersetzen von Sequenzlesungen, die an Zielen aligniert sind, die von Spleiß- und Fusionsverbindungen abgeleitet sind, oder wobei das Alignierungsprofil eine Fusionsverbindung umfasst und die Fusionsverbindung in dem Genmodell identifiziert wurde.

**4.** Verfahren nach Anspruch 1, wobei die Vielzahl von Zielsequenzen On-Target-Sequenzen und Off-Target-Sequenzen umfasst; optional ferner umfassend das Reduzieren einer Anzahl von Off-Target-Sequenzen durch Ausschließen einer oder mehrerer Primersequenzen aus der Vielzahl der Primersequenzen.

**5.** Verfahren nach Anspruch 1, ferner umfassend das rechnerische Vergleichen einer Genexpression von zwei oder mehr Proben, wobei aus einer ersten RNA-Probe erzeugte alignierte Lesungen mit aus einer zweiten RNA-Probe erzeugten alignierten Lesungen verglichen werden, wobei die Alignierung unter Verwendung der Vielzahl von Zielsequenzen durchgeführt wird.

**6.** Verfahren nach Anspruch 1, wobei das Alignierungsprofil mindestens eine von einer Platzierung, einer Qualitätsbewertung und einer Sequenzintegrität für die Sequenzlesungen der Testprobe einschließt.

**7.** Verfahren nach Anspruch 1, ferner Folgendes umfassend:
Übersetzen der Sequenzlesungen aus der Testprobe in ein gesamtes Referenzgenom unter Verwendung der abgebildeten Zielsequenzen und des modifizierten Referenzgenoms.

**8.** Verfahren nach Anspruch 1, wobei das Erzeugen eines Alignierungsprofils ferner das Alignieren einer Sequenzlesung, die eine nichtalignierte Fusionsverbindung umfasst, an nicht zusammenhängende Sequenzen des Referenzgenoms umfasst.

**9.** Computerimplementiertes Verfahren zum Alignieren von RNA, Folgendes umfassend:

Empfangen einer Vielzahl von Primersequenzen und einer Vielzahl von Transkriptionssequenzen aus einem Referenzgenom auf einer Datenspeichereinheit, wobei die Transkriptionssequenzen aus dem Referenzgenom gemäß einem Genmodell transkribierbar sind, das eine Identifizierung von Spleißverbindungen, Fusionsverbindungen oder beidem in dem Referenzgenom umfasst;
Zuordnen von Primer-Sequenzen zu einzelnen Loci, die Loci von jeweiligen Transkriptionssequenzen entsprechen;
Analysieren der Vielzahl von Primersequenzen unter Verwendung eines Mikroprozessors, um eine Vielzahl von Zielsequenzen zu bestimmen, die aus der Vielzahl von Transkriptionssequenzen durch die Vielzahl von Primersequenzen amplifizierbar sind;
Erzeugen, unter Verwendung eines Mikroprozessors, eines modifizierten Referenzgenoms auf der Grundlage der Vielzahl von Zielsequenzen, wobei das modifizierte Referenzgenom die Vielzahl von Zielsequenzen umfasst und Sequenzen ausschließt, die durch die Vielzahl von Primersequenzen nicht aus der Vielzahl von Transkriptionssequenzen amplifizierbar sind;
Alignieren, unter Verwendung eines Mikroprozessors, von Sequenzlesungen, die von einer RNA-Ampliconmoleküle umfassenden Testprobe erzeugt wurden, an dem modifizierten Referenzgenom, wobei die RNA-Ampliconmoleküle durch Amplifizieren von RNA-Sequenzen in der Testprobe unter Verwendung von Primern mit Sequenzen der Vielzahl von Primersequenzen erzeugt werden;
Erzeugen eines Alignierungsprofils, wobei das Alignierungsprofil mindestens eine von Platzierung, Qualitätsbewertung und Sequenzintegrität für die Sequenzlesungen der Testprobe einschließt; und

Übersetzen der Sequenzlesungen aus der Testprobe in ein gesamtes Referenzgenom unter Verwendung der abgebildeten Zielsequenzen und des modifizierten Referenzgenoms.

**10.** Computersystem zum Alignieren von RNA, Folgendes umfassend:

einen oder mehrere Mikroprozessoren; und
einen oder mehrere Speicher, die eine Vielzahl von Primersequenzen und eine Vielzahl von Transkriptionssequenzen speichern, die aus einem Referenzgenom transkribierbar sind,
wobei der eine oder die mehreren Speicher Anweisungen speichern, die, wenn sie von dem einen oder den mehreren Mikroprozessoren ausgeführt werden, das Computersystem zu Folgendem veranlassen:

Analysieren der Vielzahl von Primersequenzen, um eine Vielzahl von Zielsequenzen zu bestimmen, die aus der Vielzahl der Transkriptionssequenzen durch die Vielzahl der Primersequenzen amplifizierbar sind;
Erzeugen eines modifizierten Referenzgenoms auf der Grundlage der Vielzahl von Zielsequenzen, wobei das modifizierte Referenzgenom die Vielzahl von Zielsequenzen umfasst und Sequenzen ausschließt, die nicht aus der Vielzahl von Transkriptionssequenzen durch die Vielzahl von Primersequenzen amplifizierbar sind;
Alignieren von Sequenzlesungen, die von einer Testprobe erzeugt werden, die RNA-Ampliconmoleküle umfasst, an dem modifizierten Referenzgenom, wobei die RNA-Ampliconmoleküle durch Amplifizieren von RNA-Sequenzen in der Testprobe unter Verwendung von Primern mit Sequenzen der Vielzahl von Primersequenzen erzeugt werden; und
Erzeugen eines Alignierungsprofils für die Testprobe auf der Grundlage des Alignierens.

**11.** Computersystem nach Anspruch 10, wobei die Anweisungen das Computersystem veranlassen, Primersequenzen einzelnen Loci zuzuordnen, die Loci von jeweiligen Transkriptionssequenzen entsprechen, wobei optional die Anweisungen das Computersystem veranlassen, eine oder mehrere der erzeugten Zielsequenzen zu entfernen, auf der Grundlage, dass der einen oder der mehreren der erzeugten Zielsequenzen mehr als eine On-Target-Sequenz überspannen, oder wobei die Vielzahl von Primersequenzen eine Vielzahl von Primerpaaren umfasst, und ein erstes Primerpaar einen ersten Primer und einen zweiten Primer für einen ersten Locus umfasst, und ein zweites Primerpaar den ersten Primer und einen zweiten Primer für einen zweiten Locus umfasst.

**12.** Computersystem nach Anspruch 10, wobei die Transkriptionssequenzen aus dem Referenzgenom gemäß einem Genmodell transkribierbar sind, wobei das Genmodell eine Identifizierung von Spleißverbindungen, Fusionsverbindungen oder beidem in den aus dem Referenzgenom transkribierbaren Transkriptionssequenzen umfasst, wobei optional das Alignierungsprofil eine Fusionsverbindung umfasst und die Fusionsverbindung in dem Genmodell identifiziert wurde.

**13.** Computersystem nach Anspruch 10, wobei die Vielzahl von Zielsequenzen On-Target-Sequenzen und Off-Target-Sequenzen umfassen.

**14.** Computersystem nach Anspruch 13, wobei die Anweisungen das Computersystem veranlassen, eine Anzahl von Off-Target-Sequenzen zu reduzieren, indem es eine oder mehrere Primersequenzen aus der Vielzahl von Primersequenzen ausschließt, optional wobei die Anweisungen das Computersystem veranlassen, eine Genexpression von zwei oder mehr Proben zu vergleichen, wodurch alignierte Lesungen, die aus einer ersten RNA-Probe erzeugt werden, mit alignierten Lesungen, die aus einer zweiten RNA-Probe erzeugt werden, verglichen werden.

**15.** Computersystem nach Anspruch 10, wobei das Erzeugen eines Alignierungsprofils ferner das Alignieren einer Sequenzlesung, die eine nichtalignierte Fusionsverbindung umfasst, an nicht zusammenhängende Sequenzen des Referenzgenoms umfasst.

## Revendications

**1.** Procédé mis en oeuvre par ordinateur d'alignement d'ARN comprenant :

la réception, sur une unité de stockage de données, d'une pluralité de séquences d'amorces et d'une pluralité de séquences de transcription transcriptibles à partir d'un génome de référence ;
l'analyse, en utilisant un microprocesseur, de la pluralité de séquences d'amorces pour déterminer une pluralité

de séquences cibles amplifiables à partir de la pluralité de séquences de transcription par la pluralité de séquences d'amorces ;

la génération, en utilisant un microprocesseur, d'un génome de référence modifié sur la base de la pluralité de séquences cibles, dans lequel le génome de référence modifié comprend la pluralité de séquences cibles et exclut des séquences non amplifiables de la pluralité de séquences de transcription par la pluralité de séquences d'amorces ;

l'alignement, en utilisant un microprocesseur, de lectures de séquences générées à partir d'un échantillon de test comprenant des molécules d'amplicon d'ARN par rapport au génome de référence modifié, dans lequel les molécules d'amplicon d'ARN sont générées en amplifiant des séquences d'ARN dans l'échantillon de test en utilisant des amorces présentant des séquences de la pluralité de séquences d'amorces ; et

la génération d'un profil d'alignement pour l'échantillon de test sur la base de l'alignement.

**2.** Procédé selon la revendication 1, comprenant en outre l'attribution à des séquences d'amorces de loci individuels correspondant à des loci de séquences de transcription respectives, optionnellement comprenant en outre la suppression d'une ou de plusieurs des séquences cibles générées sur la base du fait que les une ou plusieurs des séquences cibles générées recouvrent plus d'une séquence sur cible, ou optionnellement, dans lequel la pluralité de séquences d'amorces comprend une pluralité de paires d'amorces, et une première paire d'amorces comprend une première amorce et une seconde amorce pour un premier locus, et une seconde paire d'amorces comprend la première amorce et une seconde amorce pour un second locus.

**3.** Procédé selon la revendication 1, dans lequel les séquences de transcription sont transcriptibles à partir du génome de référence conformément à un modèle génique, dans lequel le modèle génique comprend une identification de jonctions d'épissage, de jonctions de fusion ou des deux dans les séquences de transcription transcriptibles à partir du génome de référence, optionnellement comprenant en outre la transposition de lectures de séquences alignées par rapport à des cibles dérivées à partir de jonctions d'épissage et de fusion ou dans lequel le profil d'alignement comprend une jonction de fusion et la jonction de fusion a été identifiée dans le modèle génique.

**4.** Procédé selon la revendication 1, dans lequel la pluralité de séquences cibles comprend des séquences sur cible et des séquences hors cible ; optionnellement comprenant en outre la réduction d'un nombre de séquences hors cible en excluant une ou plusieurs séquences d'amorces de la pluralité de séquences d'amorces.

**5.** Procédé selon la revendication 1, comprenant en outre la comparaison informatique d'une expression génique de deux échantillons ou plus, dans lequel des lectures alignées générées à partir d'un premier échantillon d'ARN sont comparées à des lectures alignées générées à partir d'un second échantillon d'ARN, dans lequel l'alignement est réalisé en utilisant la pluralité de séquences cibles.

**6.** Procédé selon la revendication 1, dans lequel le profil d'alignement inclut au moins un élément parmi un positionnement, un score de qualité et une intégrité de séquences pour les lectures de séquences de l'échantillon de test.

**7.** Procédé selon la revendication 1, comprenant en outre :
la transposition des lectures de séquences à partir de l'échantillon de test en un génome de référence entier en utilisant les séquences cibles mappées et le génome de référence modifié.

**8.** Procédé selon la revendication 1, dans lequel la génération d'un profil d'alignement comprend en outre l'alignement d'une lecture de séquences comprenant une jonction de fusion non alignée par rapport à des séquences non contiguës du génome de référence.

**9.** Procédé mis en oeuvre par ordinateur d'alignement d'ARN comprenant :

la réception, sur une unité de stockage de données, d'une pluralité de séquences d'amorces et d'une pluralité de séquences de transcription à partir d'un génome de référence, les séquences de transcription étant transcriptibles à partir du génome de référence conformément à un modèle génique comprenant une identification de jonctions d'épissage, de jonctions de fusion ou des deux dans le génome de référence ;

l'attribution à des séquences d'amorces de loci individuels correspondant à des loci de séquences de transcription respectives ;

l'analyse, en utilisant un microprocesseur, de la pluralité de séquences d'amorces pour déterminer une pluralité de séquences cibles amplifiables à partir de la pluralité de séquences de transcription par la pluralité de séquences d'amorces ;

la génération, en utilisant un microprocesseur, d'un génome de référence modifié sur la base de la pluralité de séquences cibles, dans lequel le génome de référence modifié comprend la pluralité de séquences cibles et exclut des séquences non amplifiables de la pluralité de séquences de transcription par la pluralité de séquences d'amorces ;
l'alignement, en utilisant un microprocesseur, de lectures de séquences générées à partir d'un échantillon de test comprenant des molécules d'amplicon d'ARN par rapport au génome de référence modifié, dans lequel les molécules d'amplicon d'ARN sont générées en amplifiant des séquences d'ARN dans l'échantillon de test en utilisant des amorces présentant des séquences de la pluralité de séquences d'amorces ;
la génération d'un profil d'alignement, dans lequel le profil d'alignement inclut au moins un élément parmi un positionnement, un score de qualité et une intégrité de séquences pour les lectures de séquences de l'échantillon de test ; et
la transposition des lectures de séquences à partir de l'échantillon de test en un génome de référence entier en utilisant les séquences cibles mappées et le génome de référence modifié.

**10.** Système informatique pour aligner l'ARN comprenant :

un ou plusieurs microprocesseurs ; et
une ou plusieurs mémoires stockant une pluralité de séquences d'amorces et une pluralité de séquences de transcription transcriptibles à partir d'un génome de référence,
les une ou plusieurs mémoires stockant des instructions qui, lorsqu'elles sont exécutées par les un ou plusieurs microprocesseurs, amènent le système informatique à :

analyser la pluralité de séquences d'amorces pour déterminer une pluralité de séquences cibles amplifiables à partir de la pluralité de séquences de transcription au moyen de la pluralité de séquences d'amorces ;
générer un génome de référence modifié sur la base de la pluralité de séquences cibles, dans lequel le génome de référence modifié comprend la pluralité de séquences cibles et exclut des séquences non amplifiables de la pluralité de séquences de transcription au moyen de la pluralité de séquences d'amorces ;
aligner des lectures de séquences générées à partir d'un échantillon de test comprenant des molécules d'amplicon d'ARN par rapport au génome de référence modifié, dans lequel les molécules d'amplicon d'ARN sont générées en amplifiant des séquences d'ARN dans l'échantillon de test en utilisant des amorces présentant des séquences de la pluralité de séquences d'amorces ; et
générer un profil d'alignement pour l'échantillon de test sur la base de l'alignement.

**11.** Système informatique selon la revendication 10, dans lequel les instructions amènent le système informatique à attribuer à des séquences d'amorces des loci individuels correspondant à des loci de séquences de transcription respectives, optionnellement dans lequel les instructions amènent le système informatique à supprimer une ou plusieurs des séquences cibles générées sur la base du fait que les une ou plusieurs des séquences cibles générées recouvrent plus d'une séquence sur cible, ou dans lequel la pluralité de séquences d'amorces comprend une pluralité de paires d'amorces, et une première paire d'amorces comprend une première amorce et une seconde amorce pour un premier locus, et une seconde paire d'amorces comprend la première amorce et une seconde amorce pour un second locus.

**12.** Système informatique selon la revendication 10, dans lequel les séquences de transcription sont transcriptibles à partir du génome de référence conformément à un modèle génique, dans lequel le modèle génique comprend une identification de jonctions d'épissage, de jonctions de fusion ou des deux dans les séquences de transcription transcriptibles à partir du génome de référence, optionnellement dans lequel le profil d'alignement comprend une jonction de fusion et la jonction de fusion a été identifiée dans le modèle génique.

**13.** Système informatique selon la revendication 10, dans lequel la pluralité de séquences cibles comprend des séquences sur cible et des séquences hors cible.

**14.** Système informatique selon la revendication 13, dans lequel les instructions amènent le système informatique à réduire un nombre de séquences hors cible en excluant une ou plusieurs séquences d'amorces de la pluralité de séquences d'amorces ; optionnellement, dans lequel les instructions amènent le système informatique à comparer une expression génique de deux échantillons ou plus, moyennant quoi des lectures alignées générées à partir d'un premier échantillon d'ARN sont comparées à des lectures alignées générées à partir d'un second échantillon d'ARN.

**15.** Système informatique selon la revendication 10, dans lequel la génération d'un profil d'alignement comprend en

outre l'alignement d'une lecture de séquences comprenant une jonction de fusion non alignée par rapport à des séquences non contiguës du génome de référence.

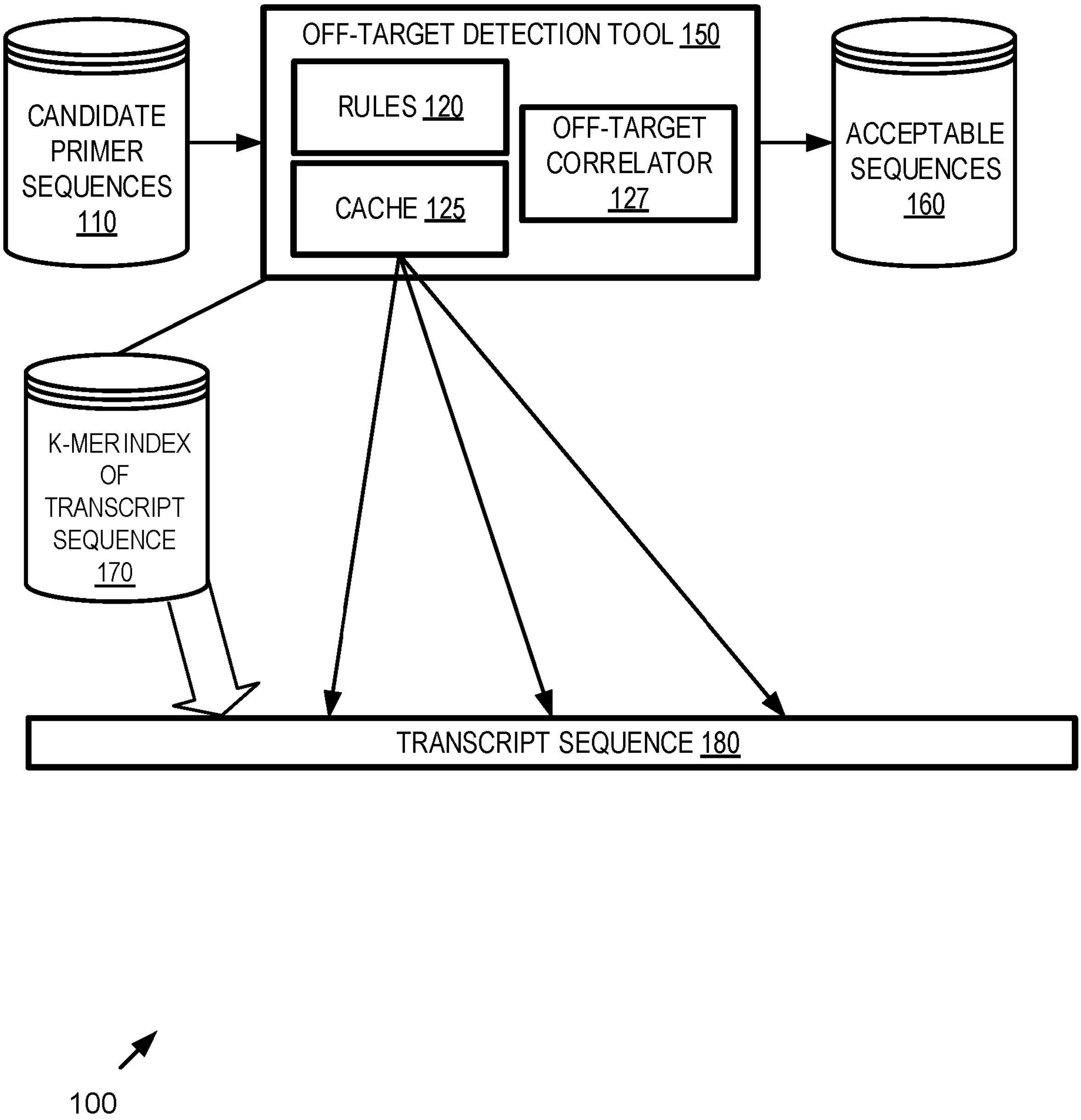
OFF-TARGET DETECTION TOOL 150
CANDIDATE PRIMER SEQUENCES 110
RULES 120
CACHE 125
OFF-TARGET CORRELATOR 127
ACCEPTABLE SEQUENCES 160
K-MER INDEX OF TRANSCRIPT SEQUENCE 170
TRANSCRIPT SEQUENCE 180
100

FIG. 1

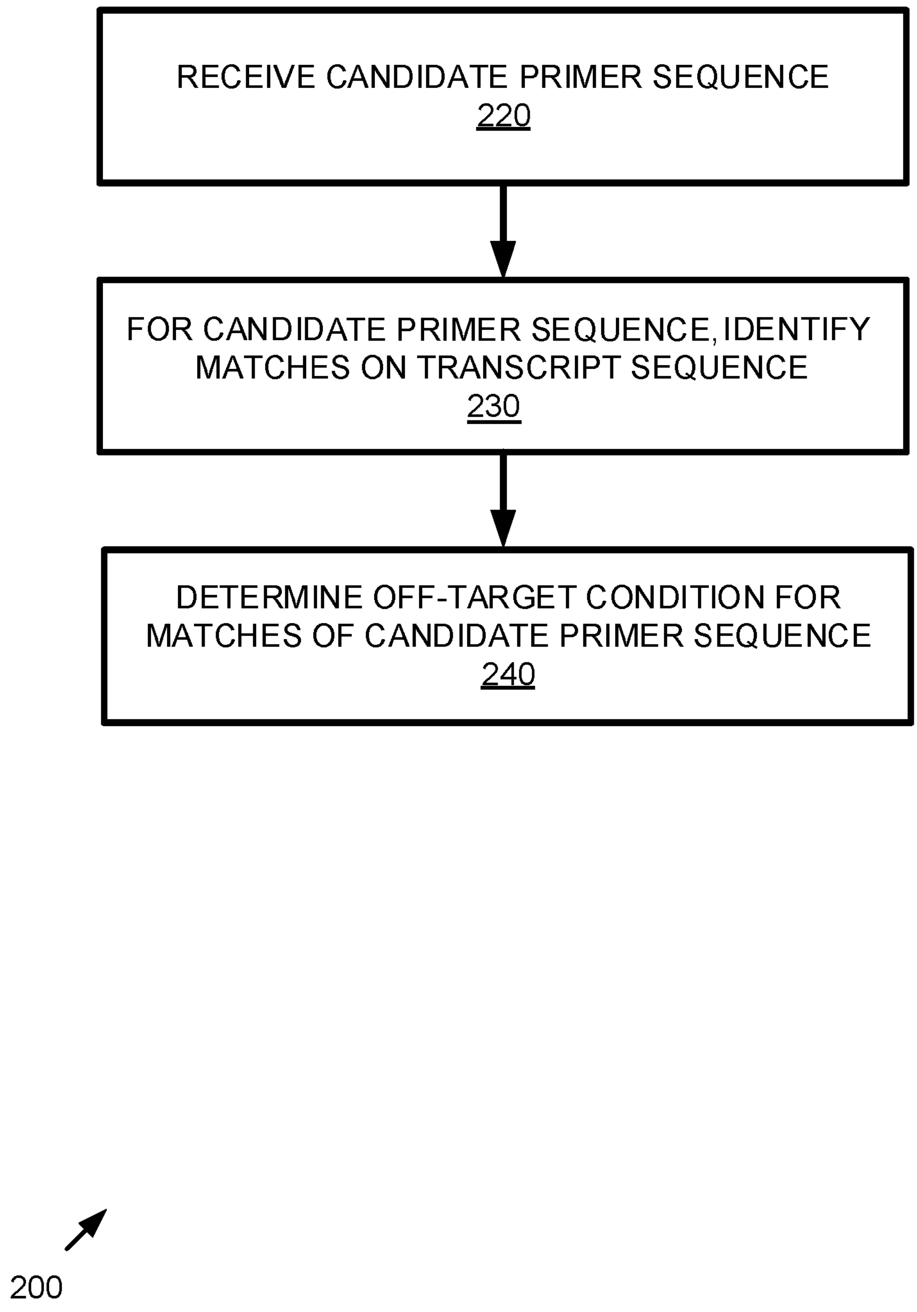
RECEIVE CANDIDATE PRIMER SEQUENCE
220
FOR CANDIDATE PRIMER SEQUENCE, IDENTIFY MATCHES ON TRANSCRIPT SEQUENCE
230
DETERMINE OFF-TARGET CONDITION FOR MATCHES OF CANDIDATE PRIMER SEQUENCE
240
200

FIG. 2

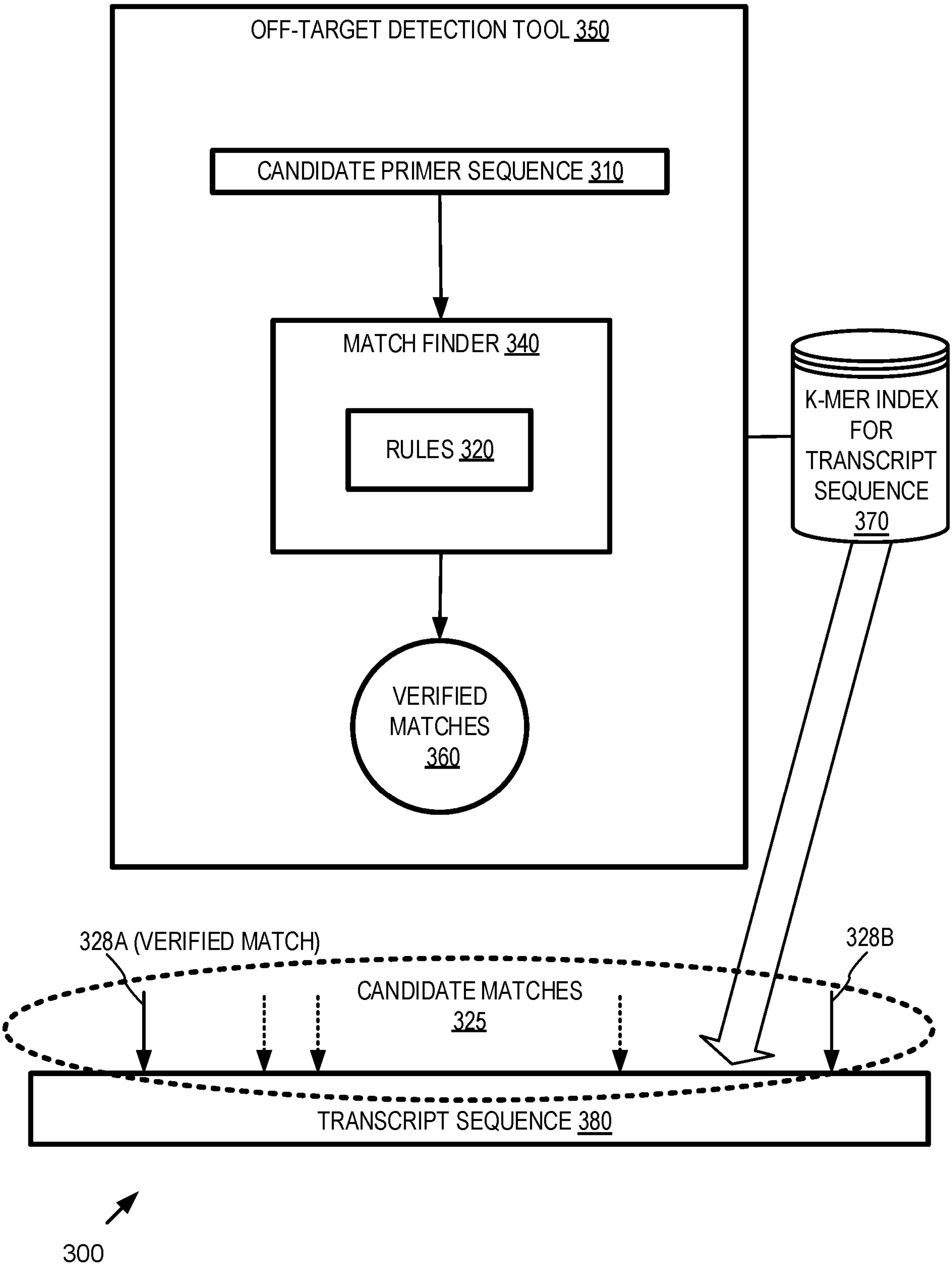
OFF-TARGET DETECTION TOOL 350
CANDIDATE PRIMER SEQUENCE 310
MATCH FINDER 340
RULES 320
VERIFIED MATCHES 360
K-MER INDEX FOR TRANSCRIPT SEQUENCE 370
328A (VERIFIED MATCH)
328B
CANDIDATE MATCHES 325
TRANSCRIPT SEQUENCE 380
300

FIG. 3

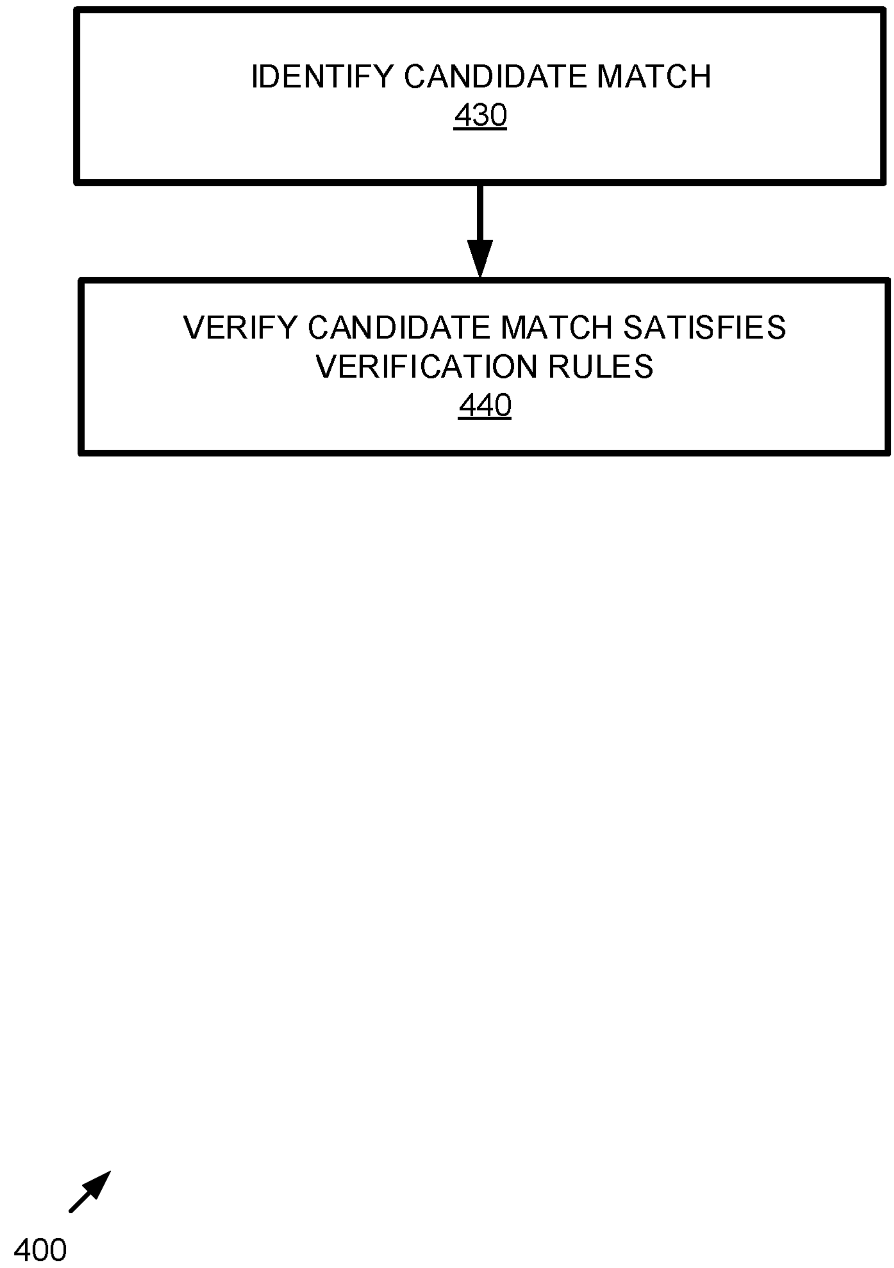
IDENTIFY CANDIDATE MATCH
430
VERIFY CANDIDATE MATCH SATISFIES VERIFICATION RULES
440
400

FIG. 4

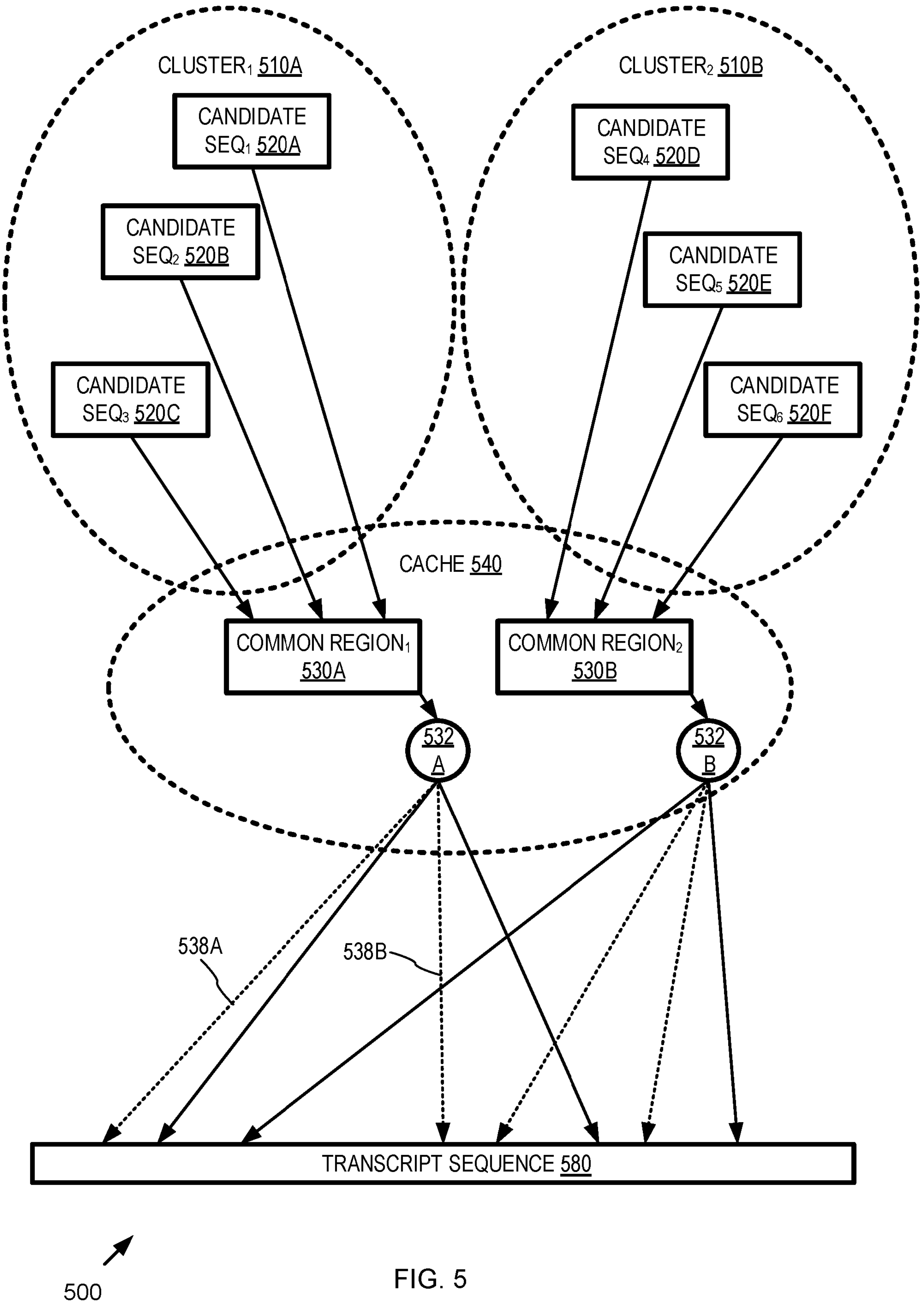
CLUSTER1 510A
CLUSTER2 510B
CANDIDATE SEQ1 520A
CANDIDATE SEQ2 520B
CANDIDATE SEQ3 520C
CANDIDATE SEQ4 520D
CANDIDATE SEQ5 520E
CANDIDATE SEQ6 520F
CACHE 540
COMMON REGION1 530A
COMMON REGION2 530B
532 A
532 B
538A
538B
TRANSCRIPT SEQUENCE 580
500

FIG. 5

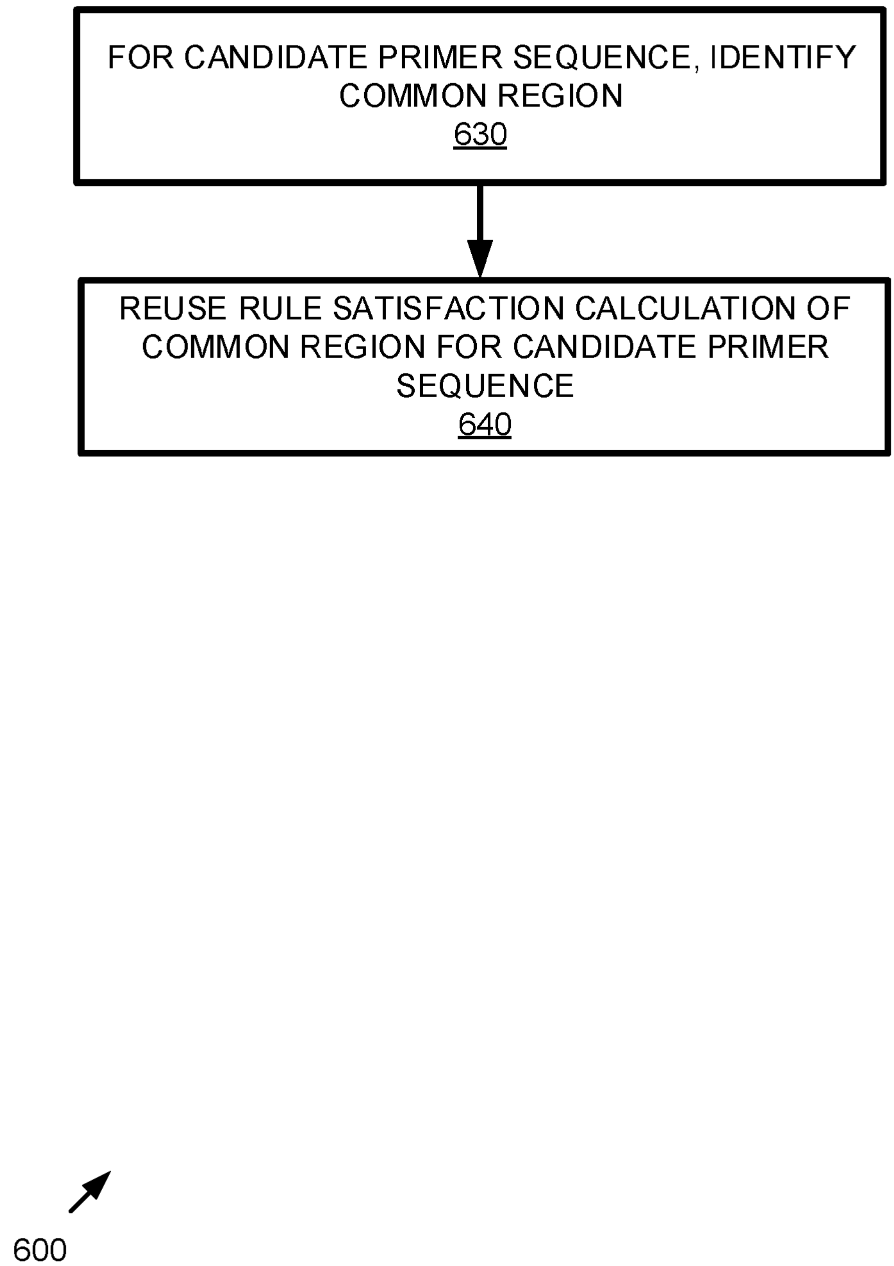
FOR CANDIDATE PRIMER SEQUENCE, IDENTIFY COMMON REGION
630
REUSE RULE SATISFACTION CALCULATION OF COMMON REGION FOR CANDIDATE PRIMER SEQUENCE
640
600

FIG. 6

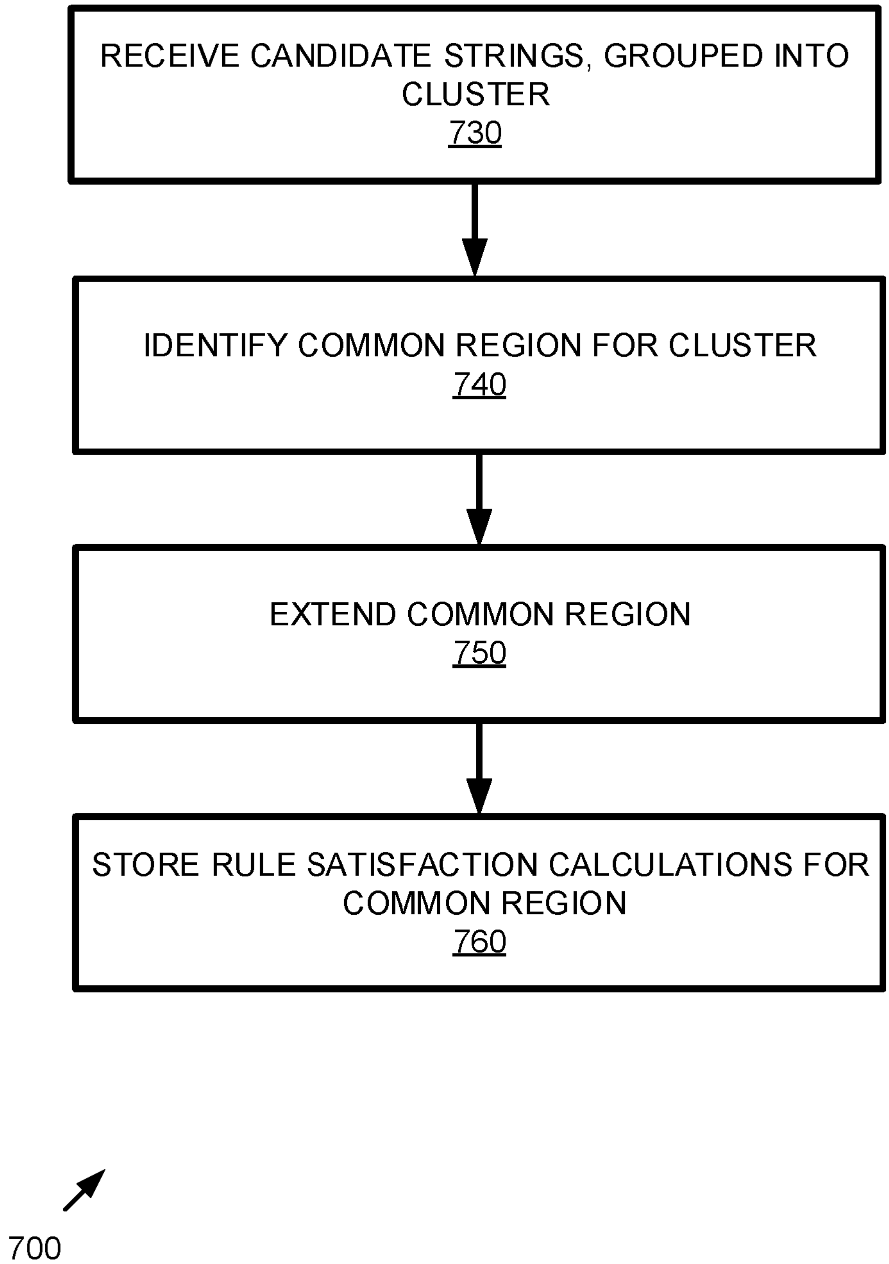
RECEIVE CANDIDATE STRINGS, GROUPED INTO CLUSTER
730
IDENTIFY COMMON REGION FOR CLUSTER
740
EXTEND COMMON REGION
750
STORE RULE SATISFACTION CALCULATIONS FOR COMMON REGION
760
700

FIG. 7

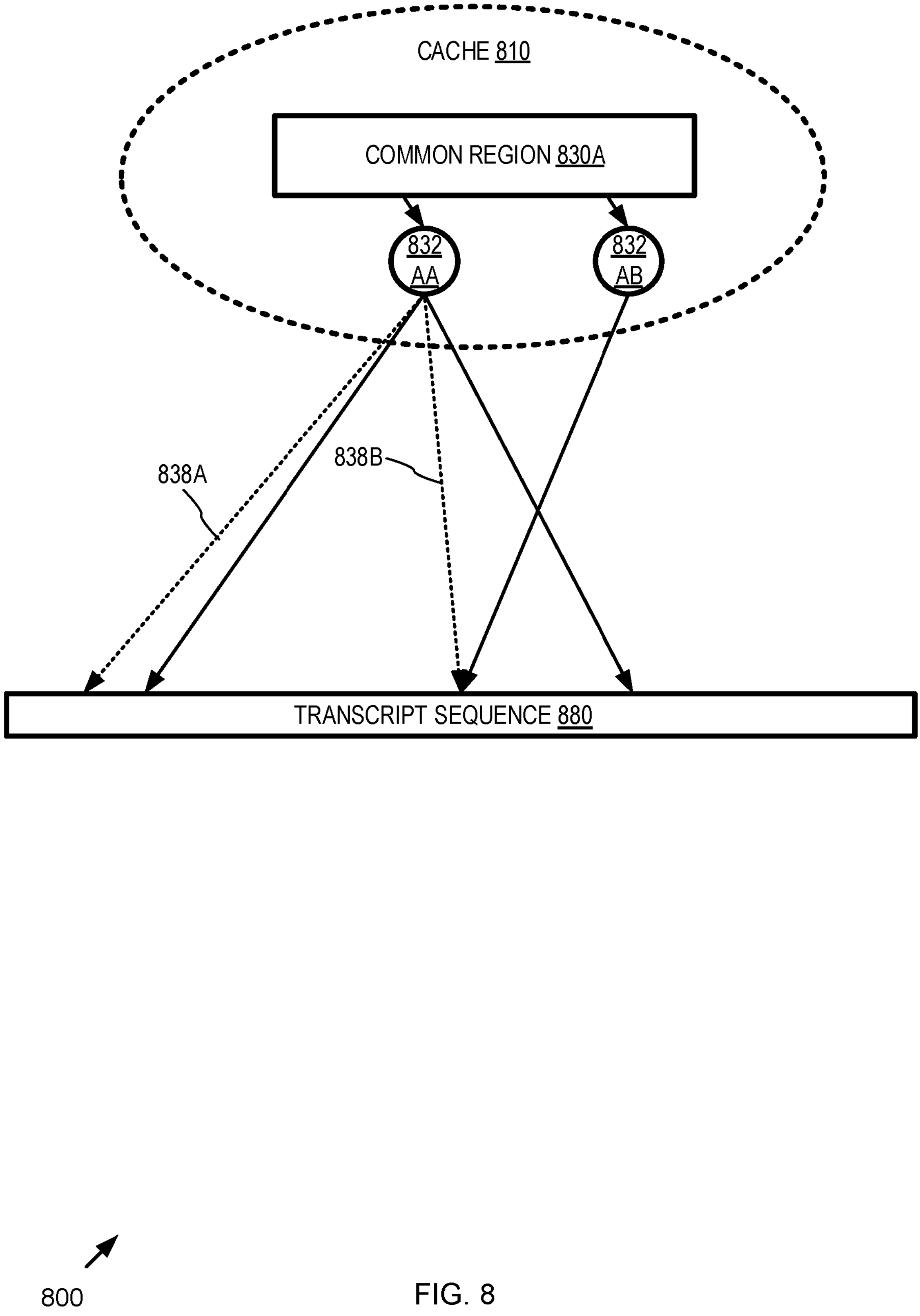
CACHE 810
COMMON REGION 830A
832 AA
832 AB
838A
838B
TRANSCRIPT SEQUENCE 880
800

FIG. 8

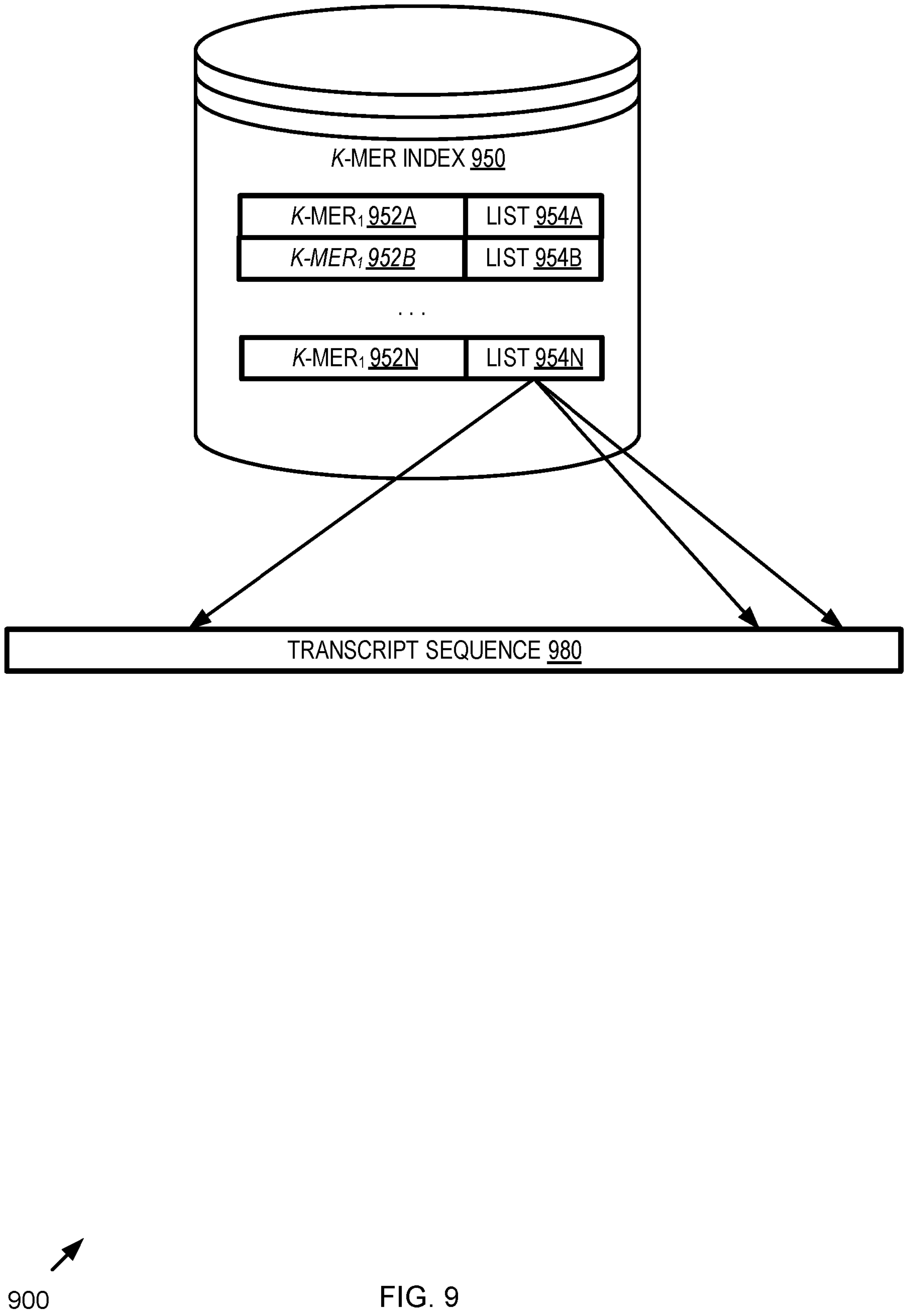
K-MER INDEX 950
K-MER1 952A
LIST 954A
K-MER1 952B
LIST 954B
. . .
K-MER1 952N
LIST 954N
TRANSCRIPT SEQUENCE 980
900

FIG. 9

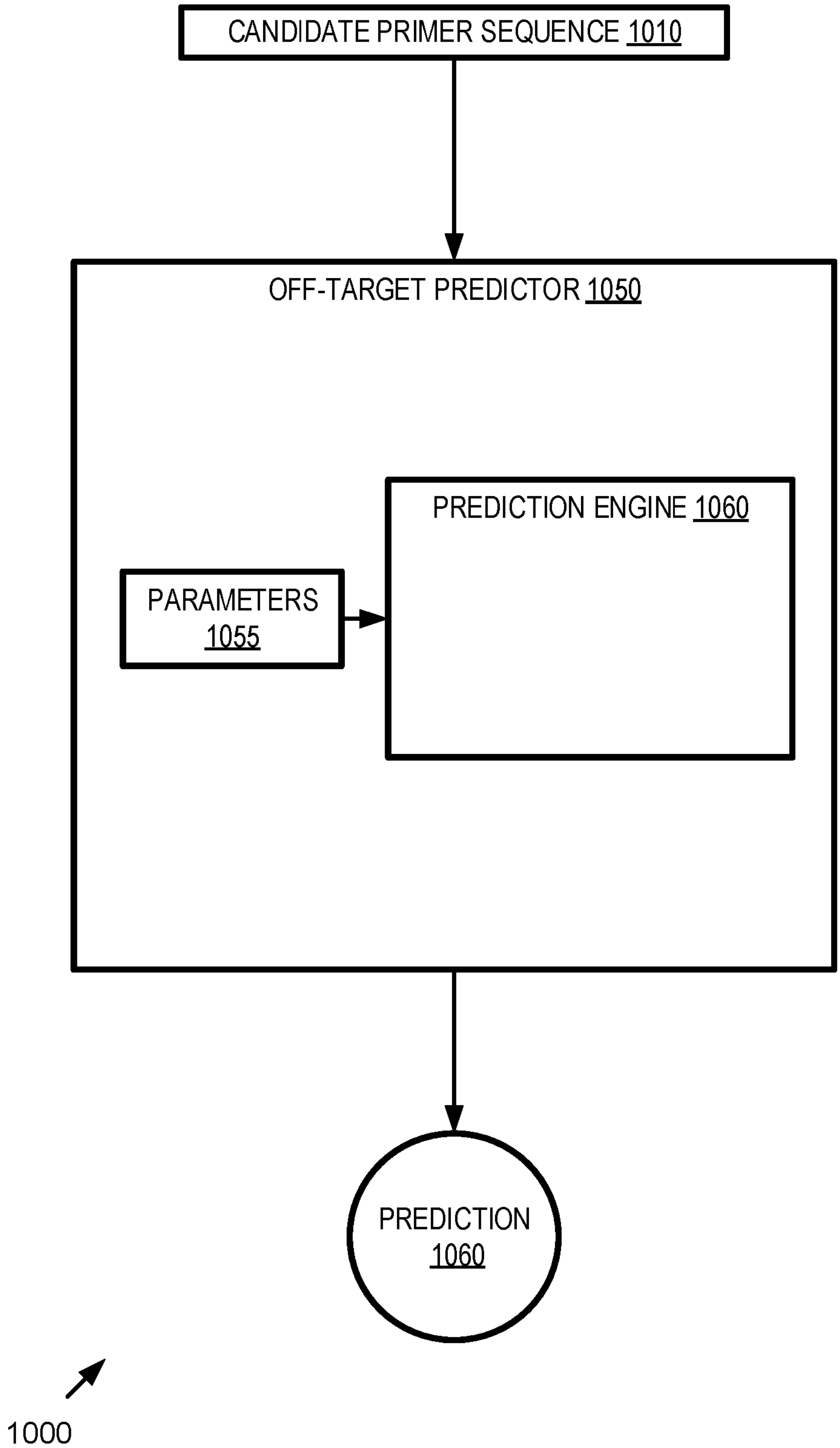
CANDIDATE PRIMER SEQUENCE 1010
OFF-TARGET PREDICTOR 1050
PREDICTION ENGINE 1060
PARAMETERS 1055
PREDICTION 1060
1000

FIG. 10

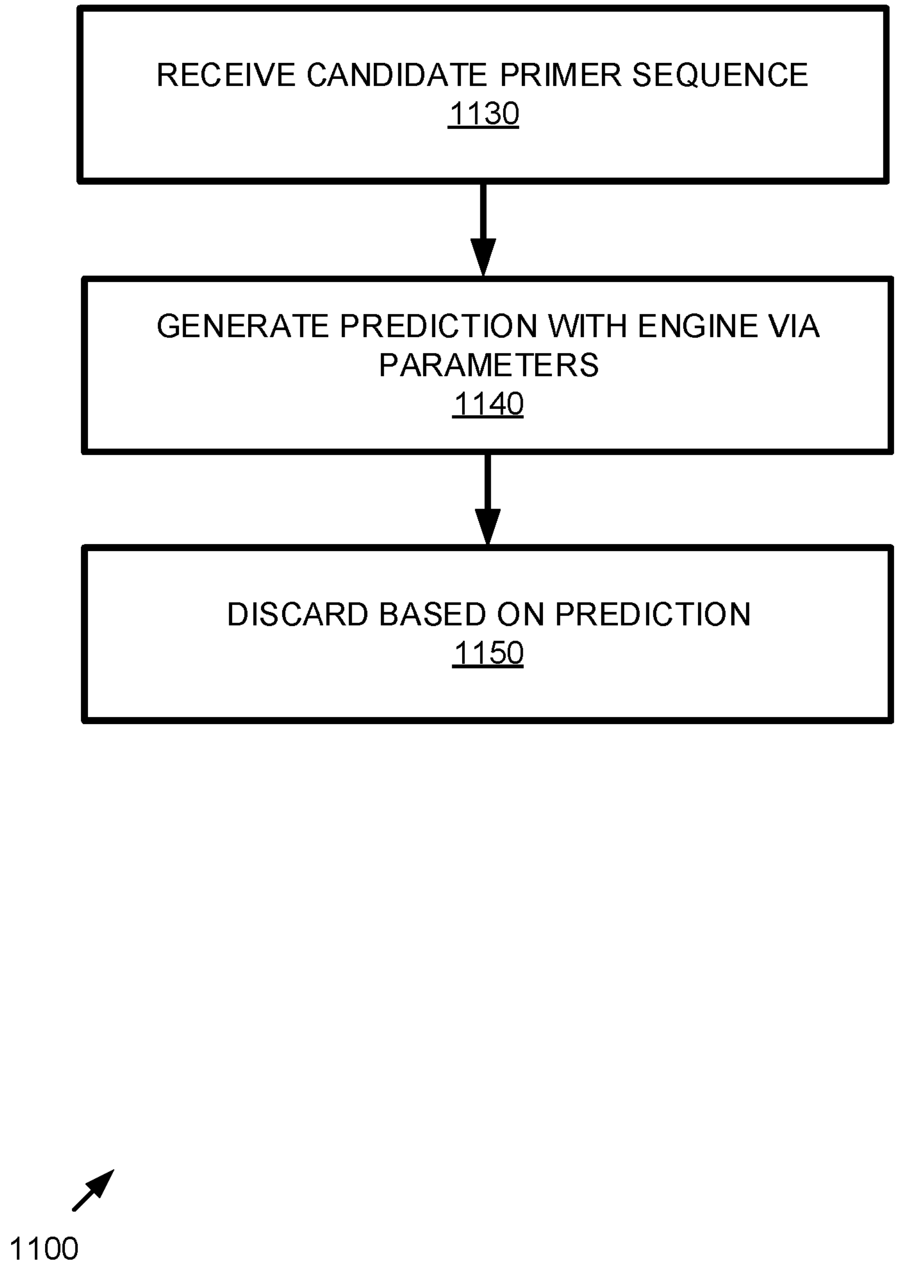
RECEIVE CANDIDATE PRIMER SEQUENCE
1130
GENERATE PREDICTION WITH ENGINE VIA PARAMETERS
1140
DISCARD BASED ON PREDICTION
1150
1100

FIG. 11

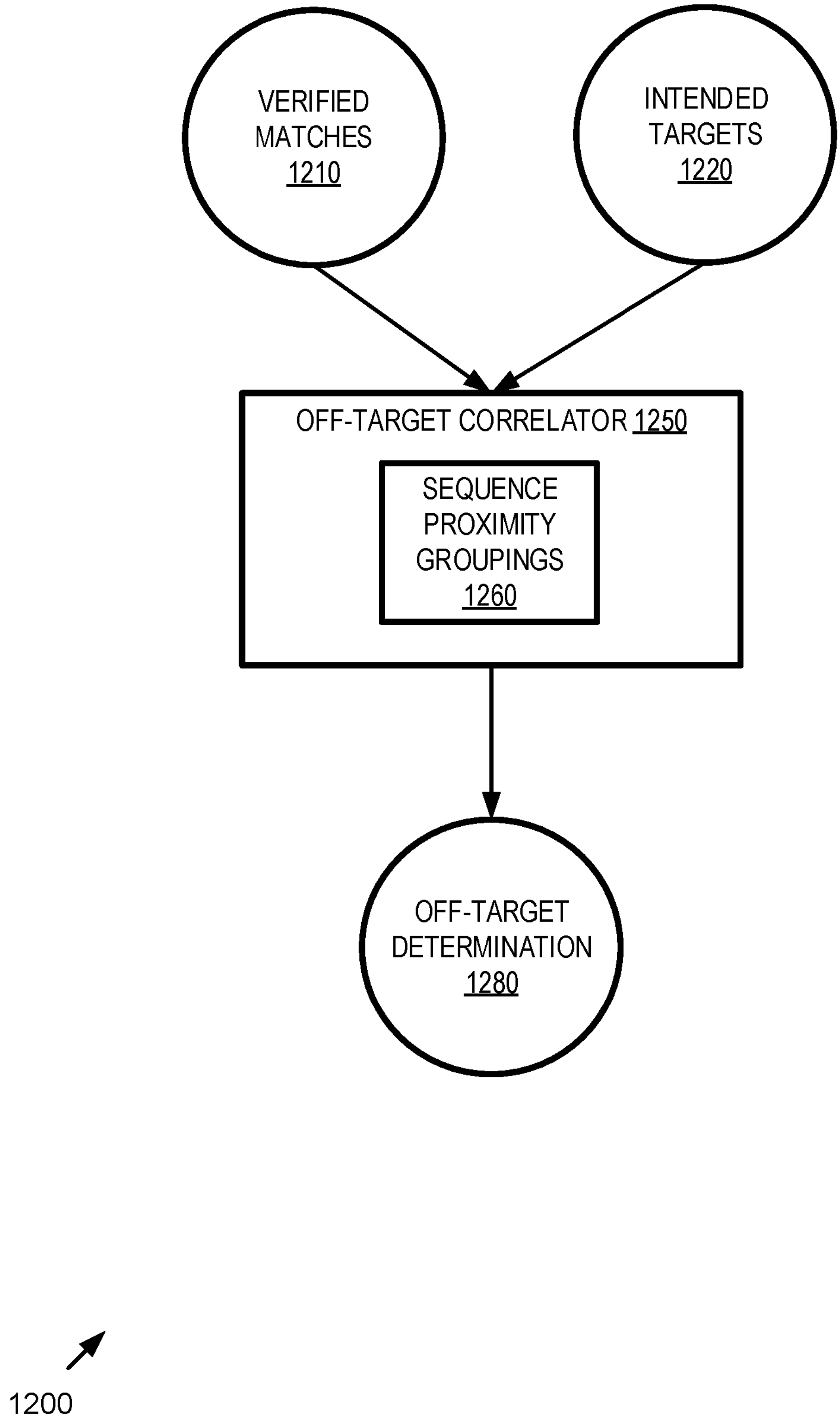
VERIFIED
MATCHES
1210
INTENDED
TARGETS
1220
OFF-TARGET CORRELATOR 1250
SEQUENCE
PROXIMITY
GROUPINGS
1260
OFF-TARGET
DETERMINATION
1280
1200

FIG. 12

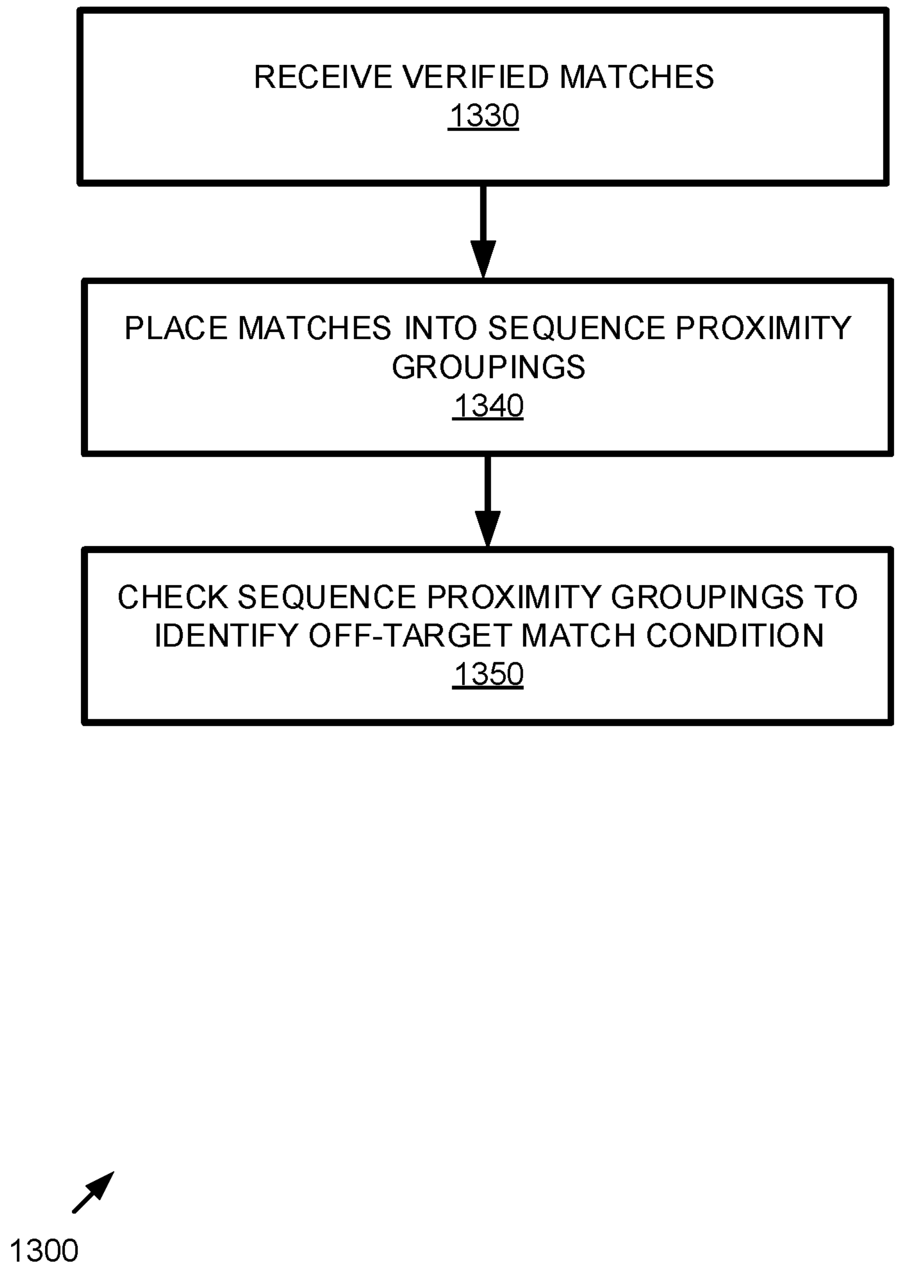
RECEIVE VERIFIED MATCHES
1330
PLACE MATCHES INTO SEQUENCE PROXIMITY GROUPINGS
1340
CHECK SEQUENCE PROXIMITY GROUPINGS TO IDENTIFY OFF-TARGET MATCH CONDITION
1350
1300

FIG. 13

- Amplified unintended regions are called off-targets

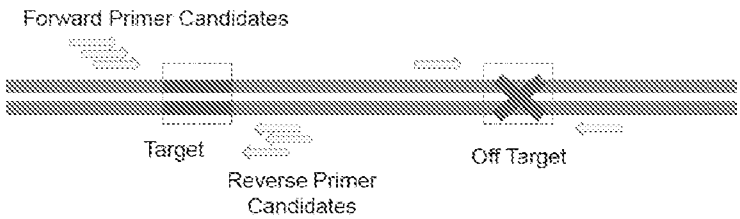


- With multiplex PCR primer design, primer sets for several targets have to be designed simultaneously

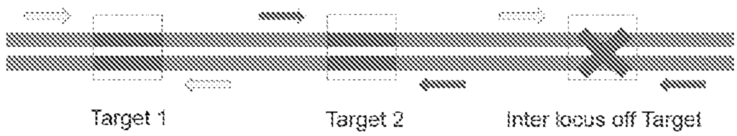


FIG. 14

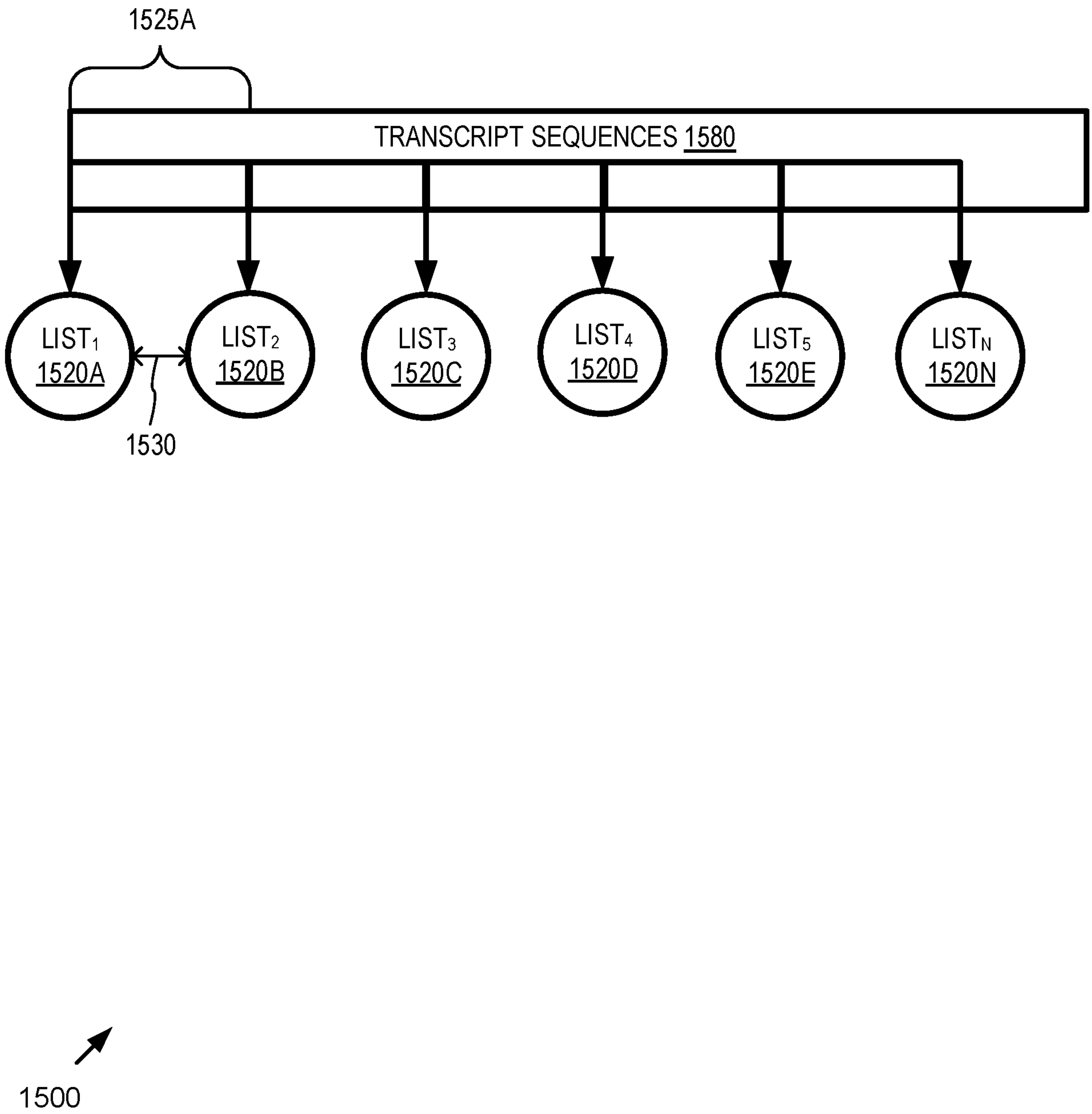
1525A
TRANSCRIPT SEQUENCES 1580
LIST1
1520A
LIST2
1520B
LIST3
1520C
LIST4
1520D
LIST5
1520E
LISTN
1520N
1530
1500

FIG. 15

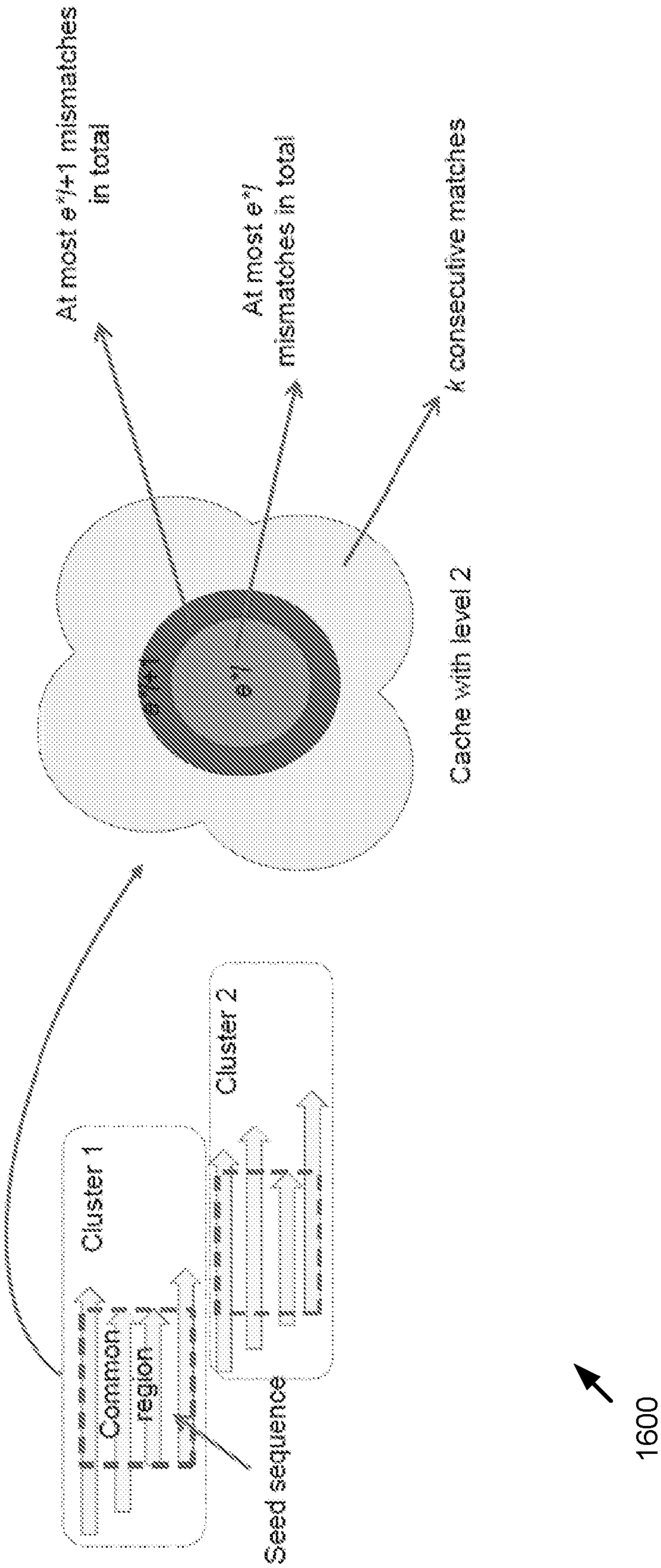
At most e*l+1 mismatches in total
At most e*l mismatches in total
k consecutive matches
e*l+1
e*l
Cache with level 2
Cluster 1
Cluster 2
Common region
Seed sequence
1600

FIG. 16

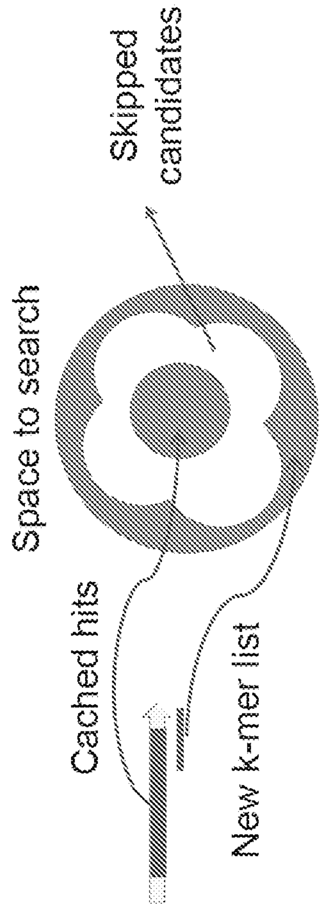
Space to search
Skipped candidates
Cached hits
New k-mer list

FIG. 17

1700

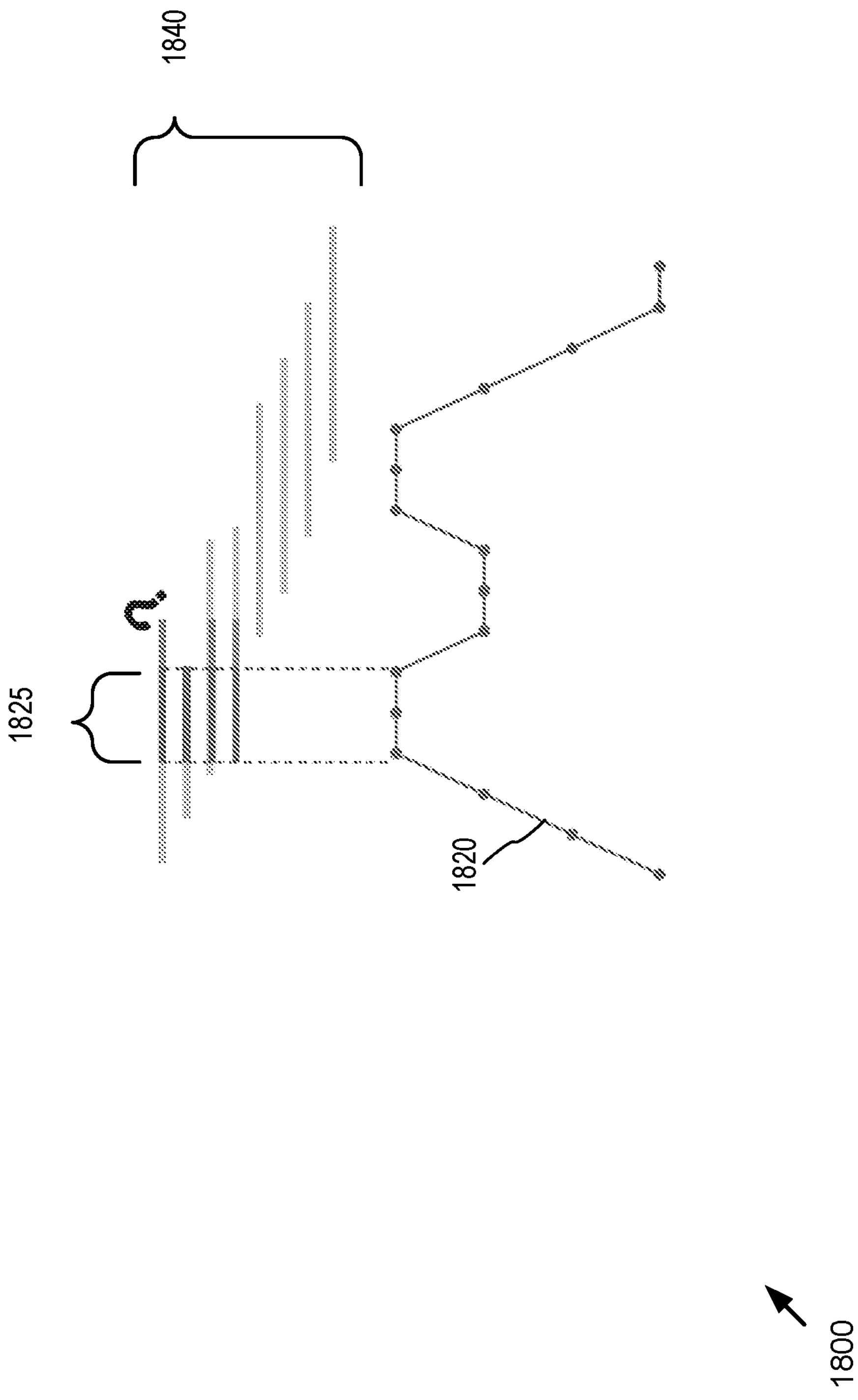


FIG. 18

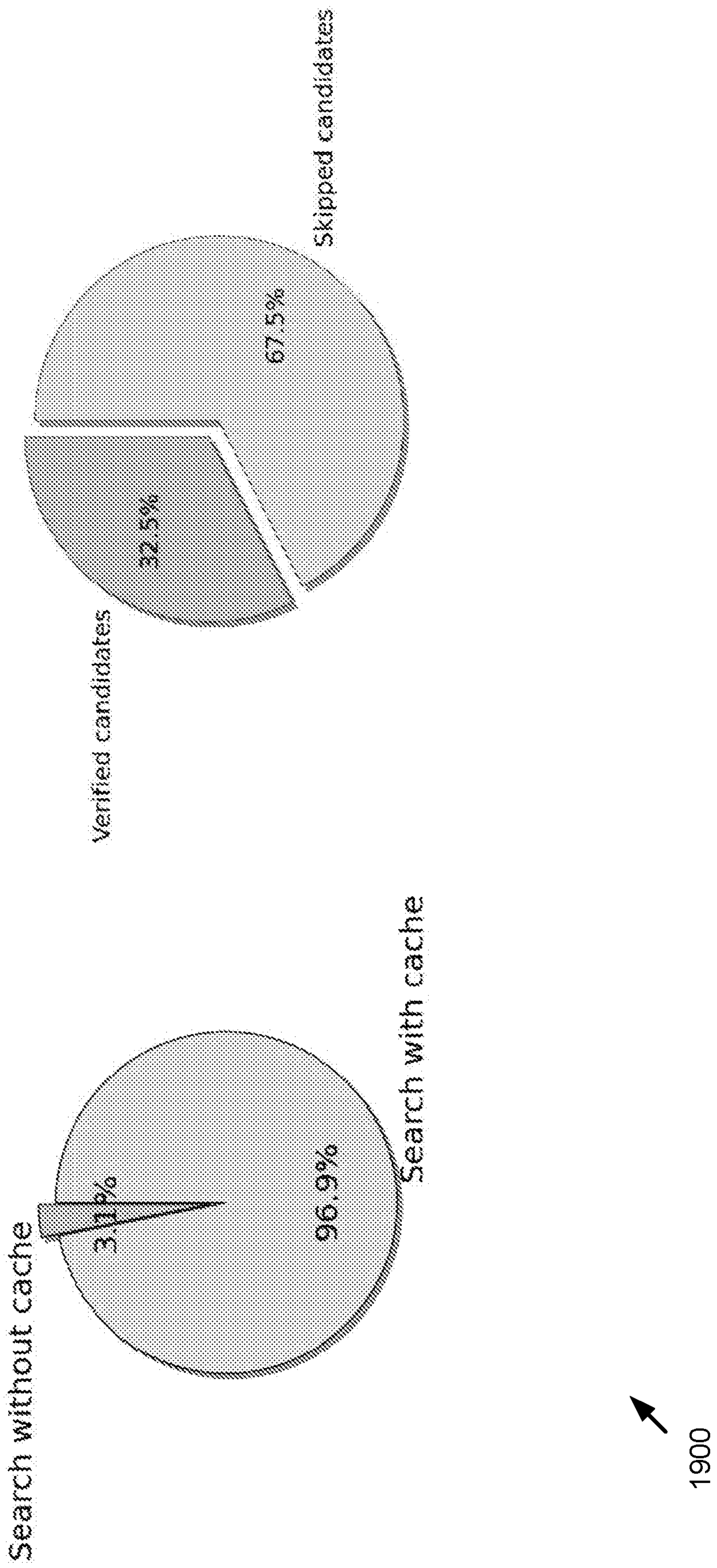
Search without cache
3.1%
96.9%
Search with cache
Verified candidates
32.5%
67.5%
Skipped candidates
1900

FIG. 19

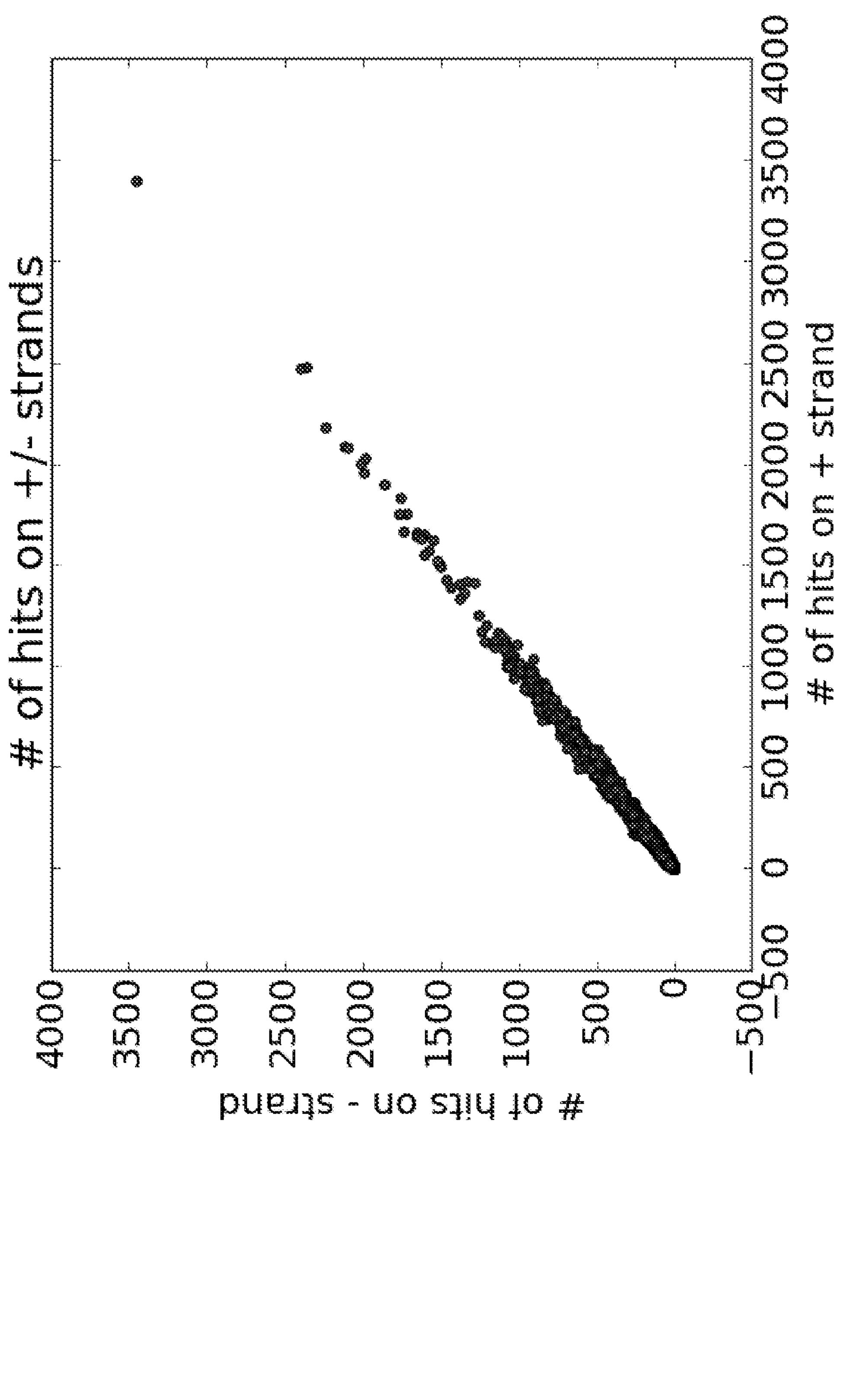

# of hits on +/- strands
# of hits on - strand
# of hits on + strand
4000
3500
3000
2500
2000
1500
1000
500
0
−500
−500
0
500
1000
1500
2000
2500
3000
3500
4000


FIG. 20

2000

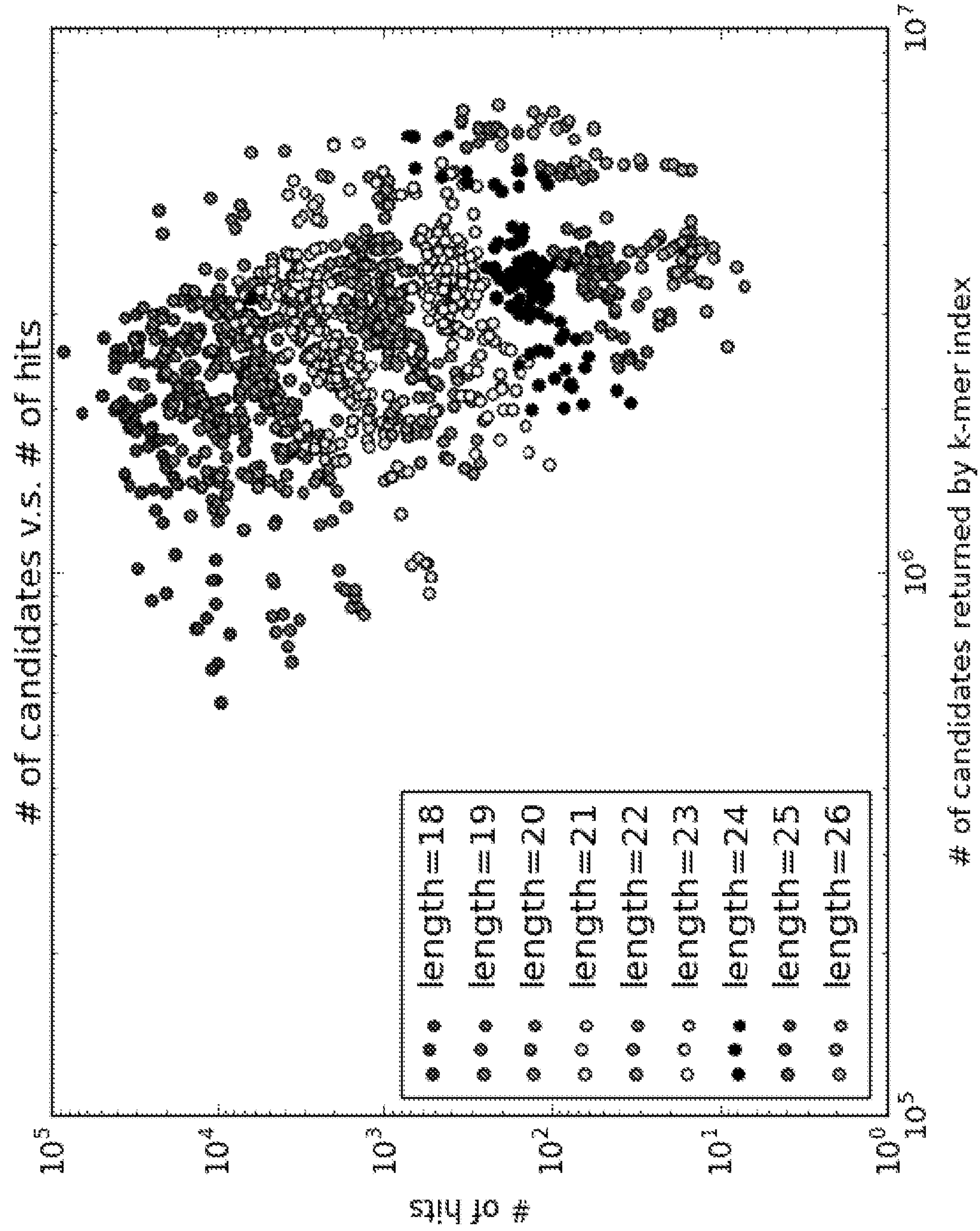

# of candidates v.s. # of hits
# of hits
# of candidates returned by k-mer index
$10^0$
$10^1$
$10^2$
$10^3$
$10^4$
$10^5$
$10^5$
$10^6$
$10^7$
length=18
length=19
length=20
length=21
length=22
length=23
length=24
length=25
length=26


FIG. 21

2100

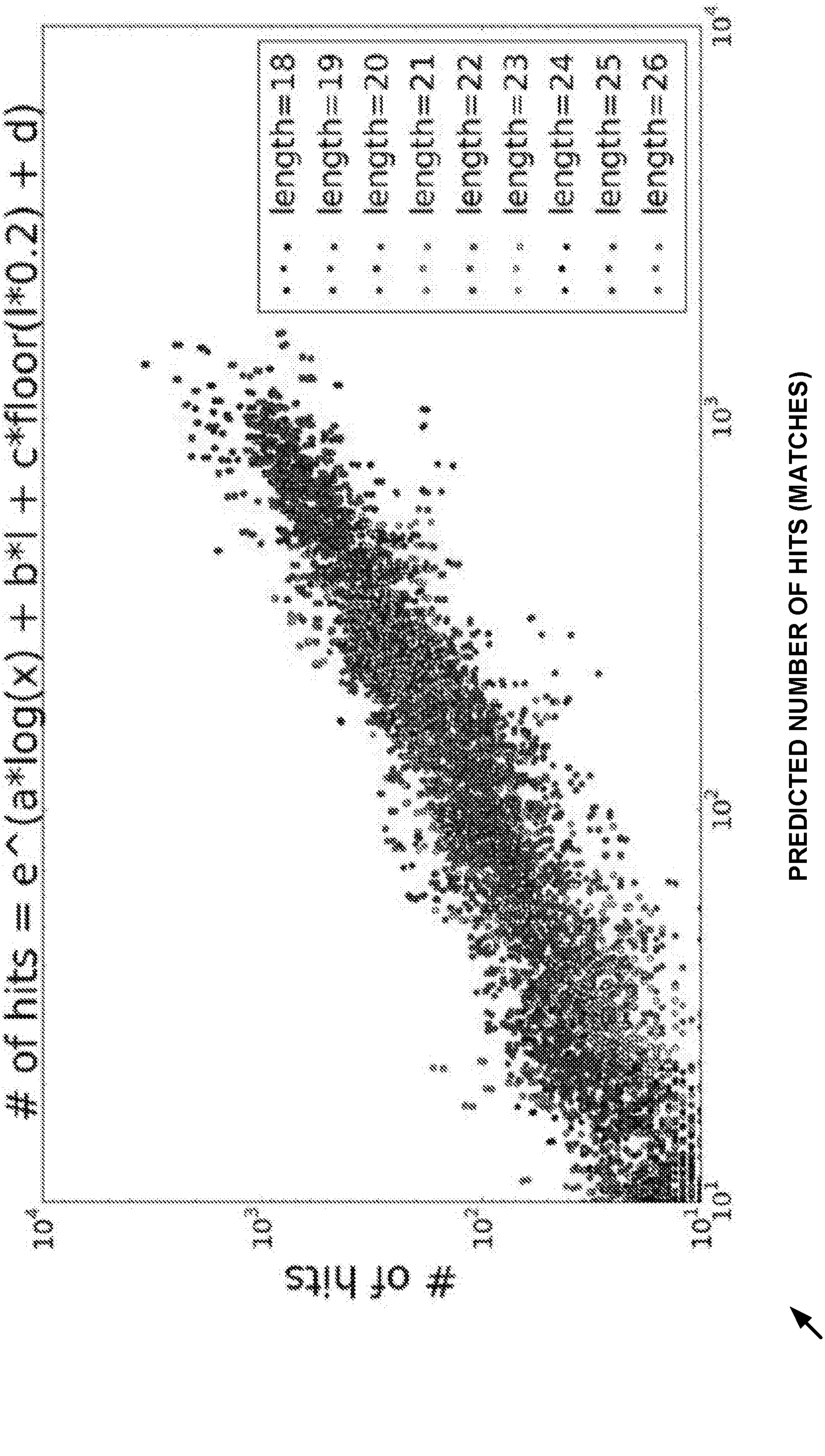

# of hits = e^(a*log(x) + b*l + c*floor(l*0.2) + d)
# of hits
10^4
10^3
10^2
10^1
length=18
length=19
length=20
length=21
length=22
length=23
length=24
length=25
length=26
PREDICTED NUMBER OF HITS (MATCHES)
2200


FIG. 22

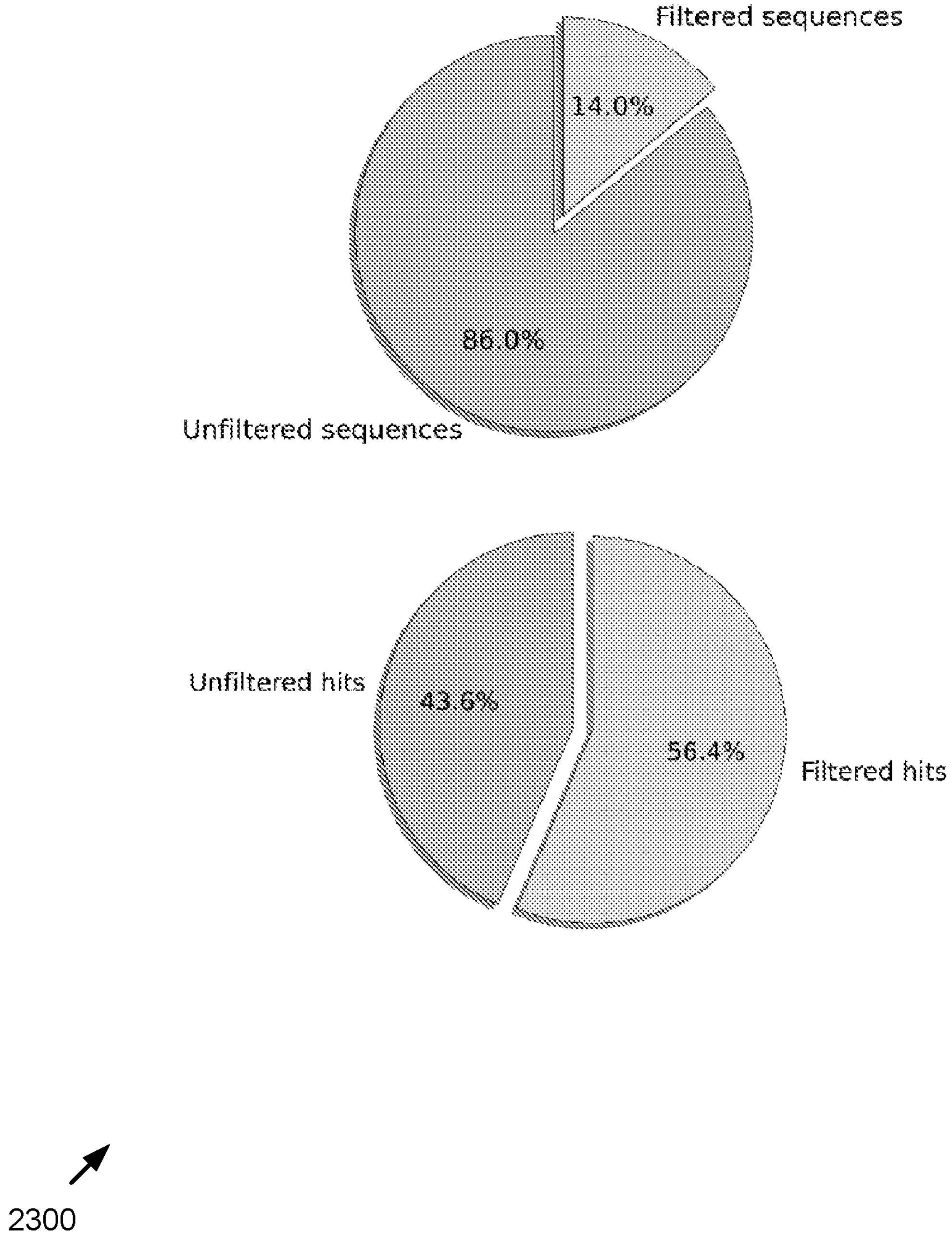
Filtered sequences
14.0%
86.0%
Unfiltered sequences
Unfiltered hits
43.6%
56.4%
Filtered hits
2300

FIG. 23

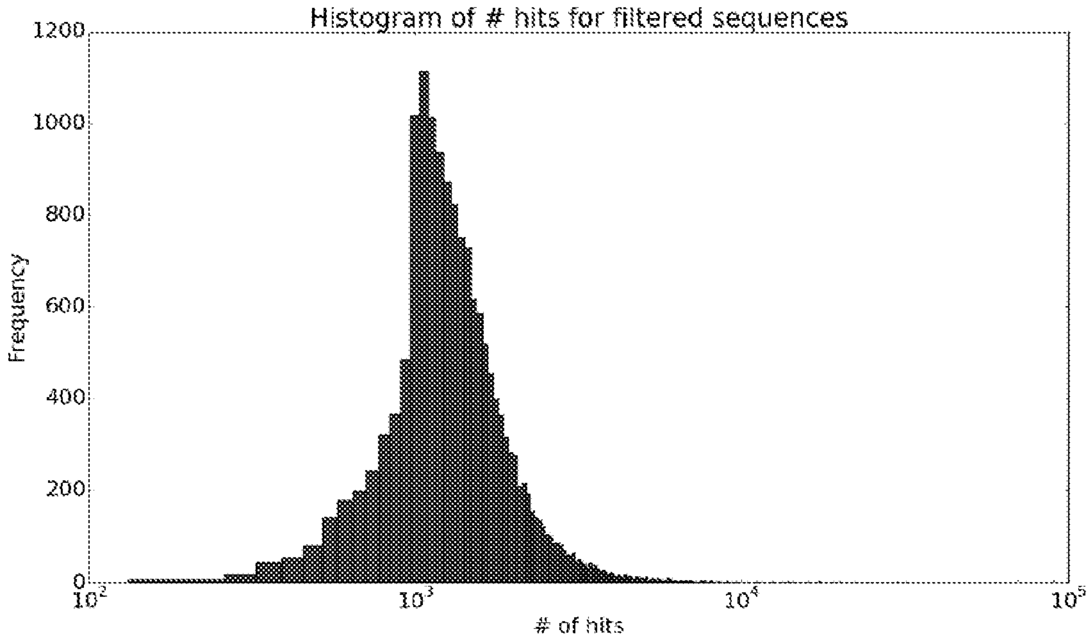
Histogram of # hits for filtered sequences
1200
1000
800
600
400
200
0
Frequency
$10^2$
$10^3$
$10^4$
$10^5$
# of hits

2400

FIG. 24

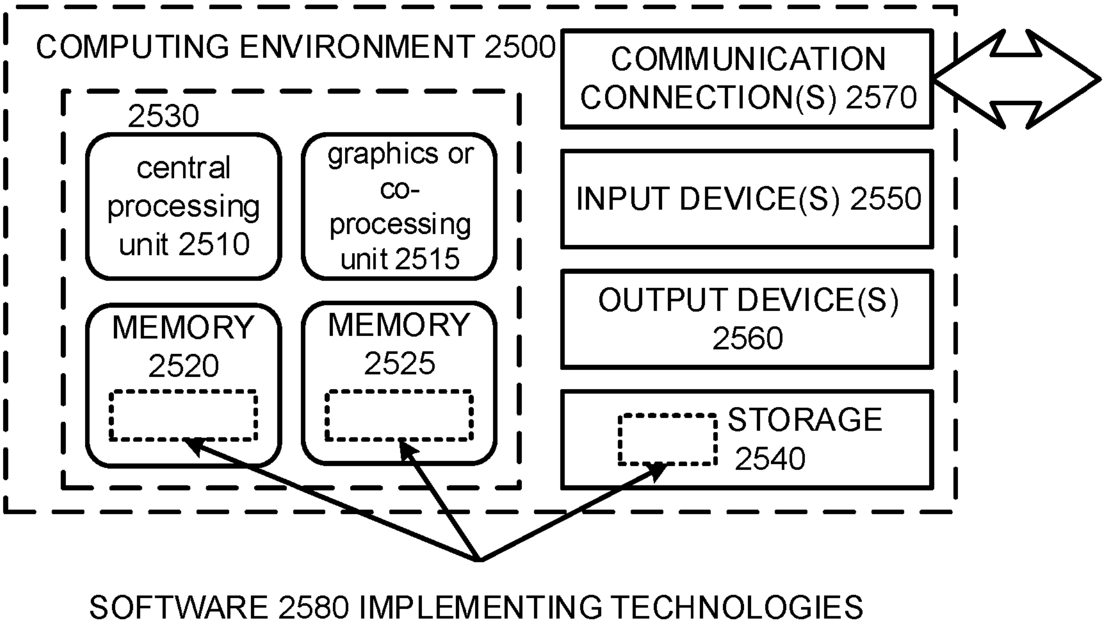
COMPUTING ENVIRONMENT 2500
2530
central processing unit 2510
graphics or co-processing unit 2515
MEMORY 2520
MEMORY 2525
COMMUNICATION CONNECTION(S) 2570
INPUT DEVICE(S) 2550
OUTPUT DEVICE(S) 2560
STORAGE 2540
SOFTWARE 2580 IMPLEMENTING TECHNOLOGIES

FIG. 25

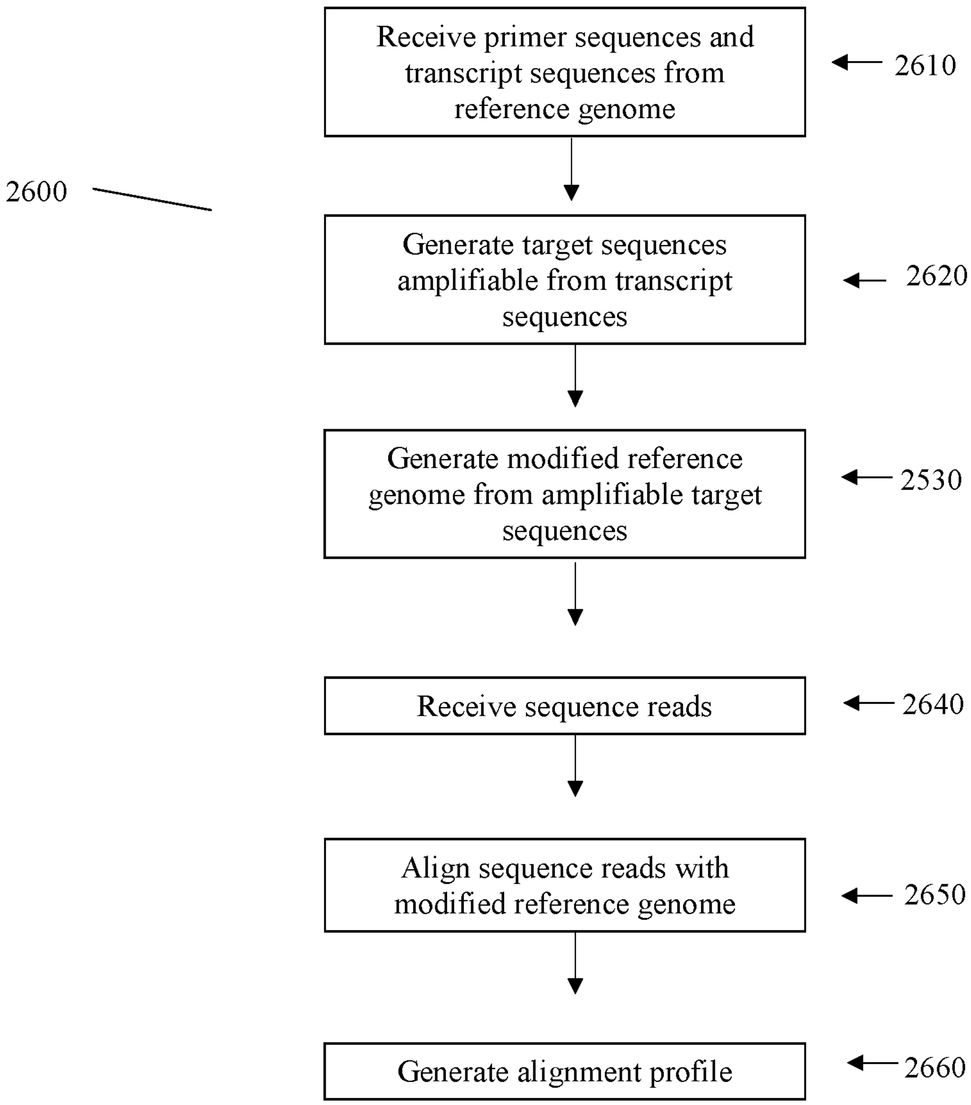
2600
Receive primer sequences and transcript sequences from reference genome
2610
Generate target sequences amplifiable from transcript sequences
2620
Generate modified reference genome from amplifiable target sequences
2530
Receive sequence reads
2640
Align sequence reads with modified reference genome
2650
Generate alignment profile
2660

FIG. 26

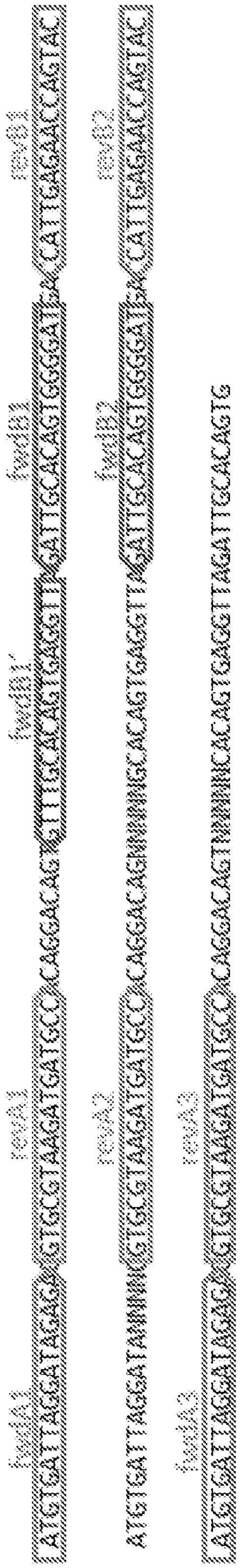
fwdA1
revA1
fwdB1'
fwdB1
revB1
revA2
fwdB2
revB2
fwdA3
revA3

FIG. 27

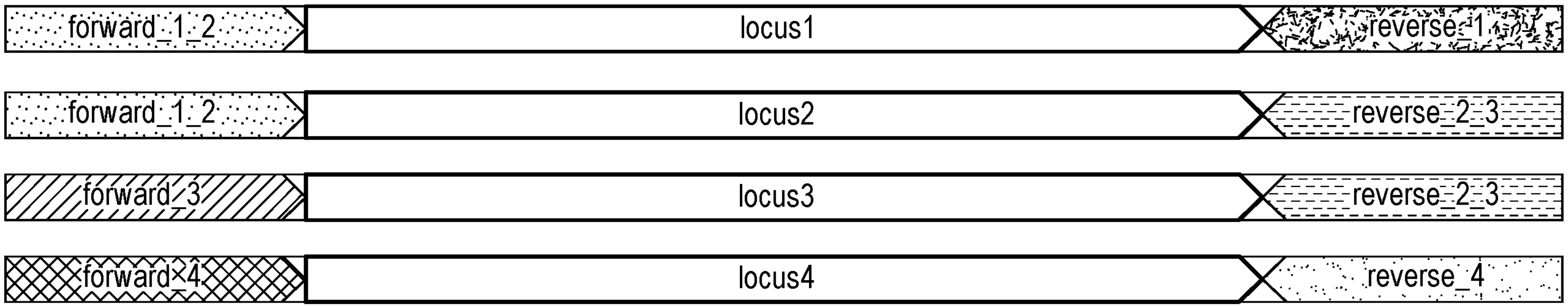


| forward | reverse | assigned forward locus | assigned reverse locus |
|---|---|---|---|
| forward_1_2 | reverse_1 | locus1 | locus1 |
| forward_1_2 | reverse_2_3 | locus2 | locus2 |
| forward_3 | reverse_2_3 | locus3 | locus3 |
| forward_4 | reverse_4 | locus4 | locus4 |
| forward_3 | reverse_1 | locus3 | locus1 |
| forward_4 | reverse_2_3 | locus4 | locus2 * |
| forward_1_2 | reverse_4 | locus1 * | locus4 |

* conflict: pick first in alphabetical order

FIG. 28

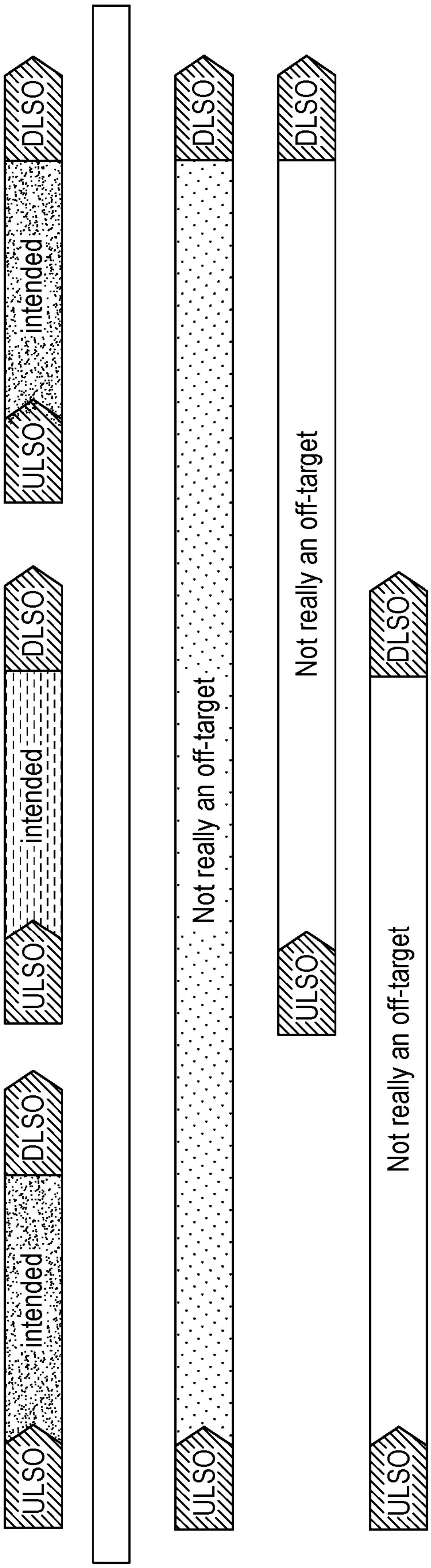
ULSO
intended
DLSO
ULSO
intended
DLSO
ULSO
intended
DLSO
ULSO
Not really an off-target
DLSO
ULSO
Not really an off-target
DLSO
ULSO
Not really an off-target
DLSO

FIG. 29

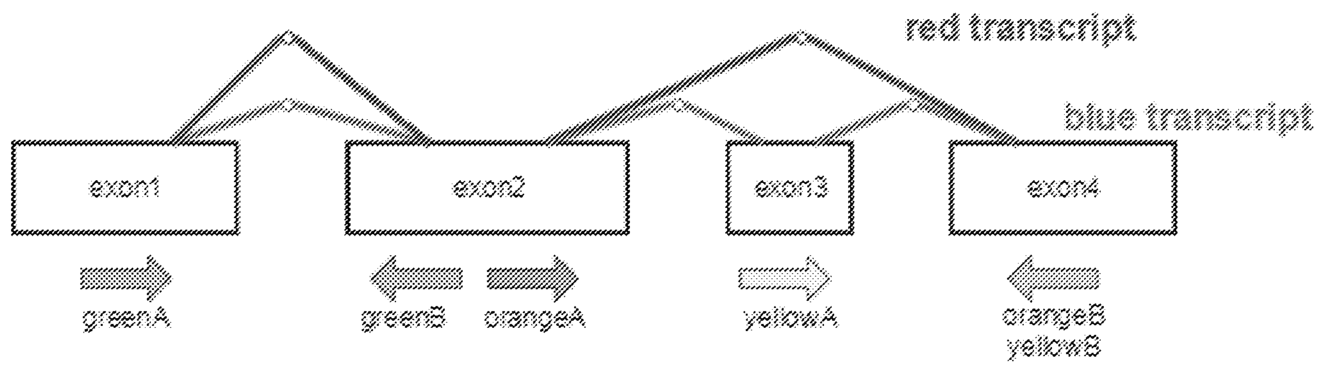


In the above diagram, we would have the following intended products:

| ProbeA | ProbeB | Transcripts |
| --- | --- | --- |
| green | green | red, blue |
| orange | orange/yellow | red |
| orange | orange/yellow | blue |
| yellow | orange/yellow | blue |

FIG. 30

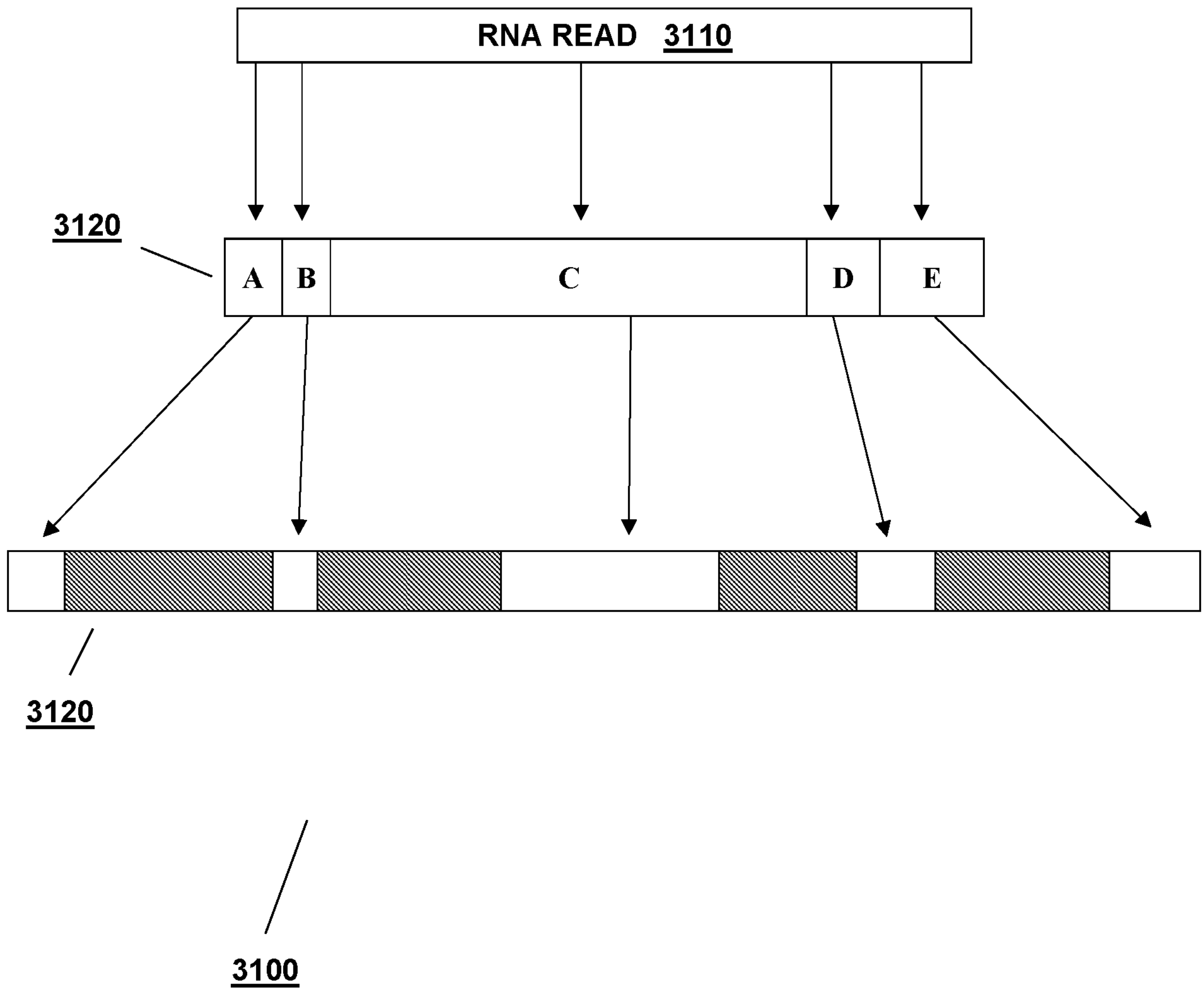


FIG. 31

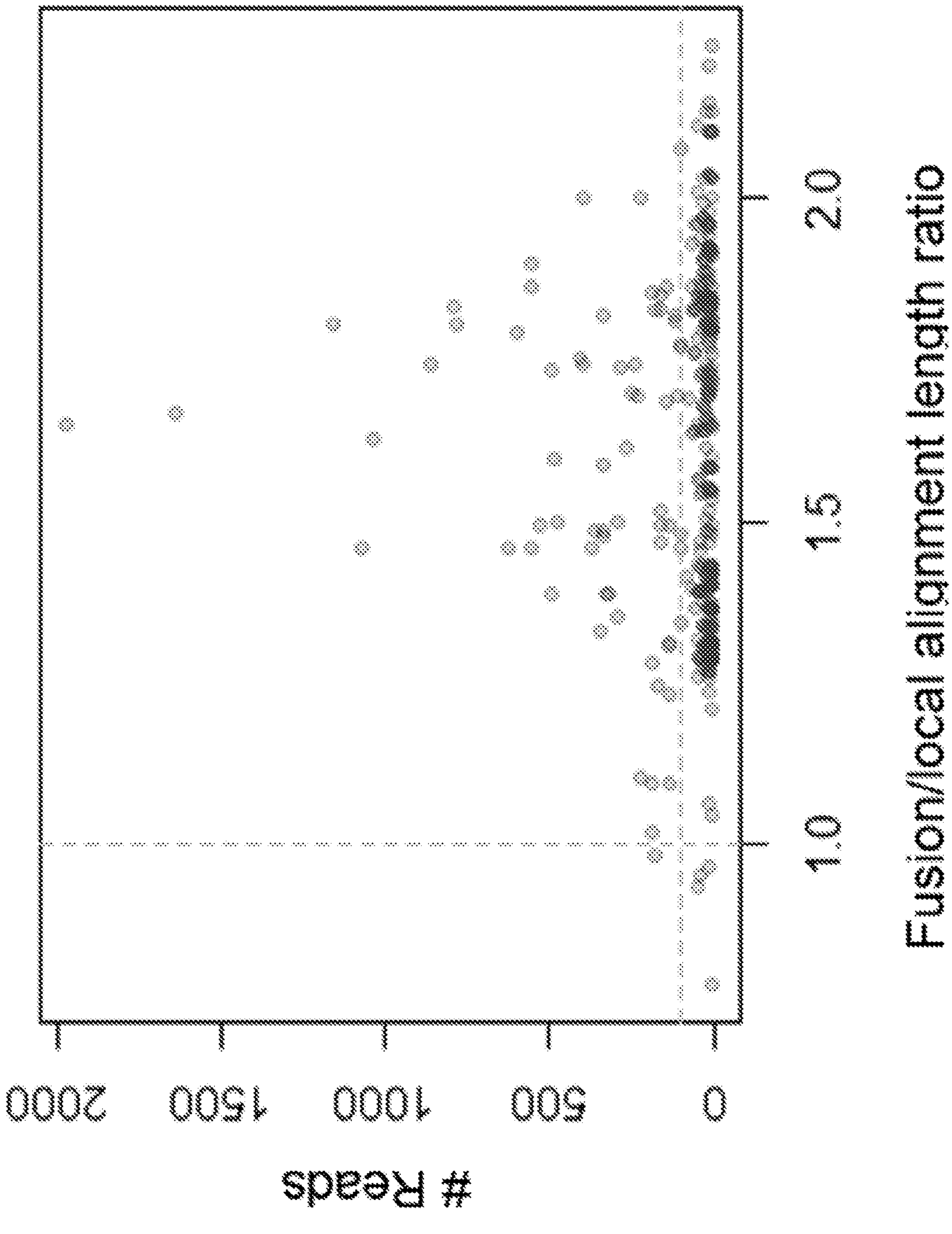

# Reads
0
500
1000
1500
2000
1.0
1.5
2.0
Fusion/local alignment length ratio


FIG. 32

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20160194694 A1
- US 2018075186 A1
- US 705079


### Non-patent literature cited in the description


- **CONESA A et al.** A survey of best practices for RNA-seq data analysis. *Genome Biol,* 2016, vol. 17, 13
- **GARBER M et al.** Computational methods for transcriptome annotation and quantification using RNA-seq. *Nat Methods,* June 2011, vol. 8 (6), 469-77
- **DOBIN A et al.** STAR: ultrafast universal RNA-seq aligner. *Bioinformatics,* 01 January 2013, vol. 29 (1), 15-21